# FASCICULE DE BREVET EUROPEEN

(11) **EP 1 353 926 B1**
(45) Date de publication et mention de la délivrance du brevet: **08.03.2006**
(21) Numéro de dépôt: 02710969.3
(22) Date de dépôt: 11.01.2002
(51) Int. Cl.: C07D 493/18, A61K 31/343, A61P 33/00, A61P 31/12, A61P 35/00

(54) **DERIVES DE MIKANOLIDE, LEUR PREPARATION ET LEURS APPLICATIONS THERAPEUTIQUES**
MIKANOLID-DERIVATE, IHRE HERSTELLUNG UND IHR GEBRAUCH FÜR THERAPEUTISCHE ANWENDUNGEN
MIKANOLIDE DERIVATIVES, THEIR PREPARATION AND THERAPEUTIC USES

(30) Priorité: 12.01.2001 FR 0100397; 14.11.2001 FR 0114688
(43) Date de publication de la demande: 22.10.2003
(73) Titulaire: Société de Conseils de Recherches et d'Applications Scientifiques ( S.C.R.A.S.), 75016 Paris (FR)
(72) Inventeur: LAVERGNE, Olivier, F-91120 Palaiseau (FR); LANCO, Christophe, F-91410 Dourdan (FR); PREVOST, Grégoire, F-92160 Antony (FR); TENG, Beng, Poon, F-91190 Gif-sur-Yvette (FR)
(74) Mandataire: Bourgouin, André
(86) Numéro de dépôt international: PCT/FR2002/000092
(87) Numéro de publication internationale: WO 2002/055523

(56) Documents cités:
- FR-A- 2 801 792
- FACEY P C ET AL: "INVESTIGATION OF PLANTS USED IN JAMAICAN FOLK MEDICINE FOR ANTI-BACTERIAL ACTIVITY" JOURNAL OF PHARMACY AND PHARMACOLOGY, LONDON, GB, vol. 51, 1999, pages 1455-1460, XP000929990 ISSN: 0022-3573
- KUPCHAN ET AL.: "The isolation and structural elucidation of two novel sesquiterpenoid tumor inhibitors from Elephantopus elatus" J. AM. CHEM. SOC., vol. 88, no. 15, 1966, pages 3674-6, XP002179333

## Description

L'invention a pour objet de nouveaux dérivés du mikanolide, leurs procédés de préparation ainsi que leurs applications thérapeutiques, notamment en tant qu'agents anti-cancéreux, anti-bactériens et anti-viraux.

Les plantes tropicales du genre *Mikania* telles que *M. cordata, M. scandens* ou *M. micrantha* font partie de certaines pharmacopées traditionnelles d'Inde, d'Amérique du Sud et d'Amérique Centrale. Sous les noms de *guaco, bejuco de finca, cepu, liane françois, matafinca,* ou encore *vedolin,* les extraits de *Mikania* sont employés comme antibiotiques et anti-inflammatoires.

Des substances actives provenant d'extraits de *Mikania* ont été isolées et caractérisées: il s'agit du mikanolide et du dihydromikanolide (voir leurs structures en figure ci-dessous), et dans une moindre mesure de scandénolides. Ce sont des sesquiterpènes de la famille des germacranes, c'est-à-dire ayant le 4-isopropyl-1,7-diméthylcyclodécane pour squelette hydrocarboné (Herz et coll., *Tetrahedron Lett.* (1967) 3111-3115; Kiang et coll., *Phytochemistry* (1968) 7: 1035-1037; Cuenca et coll., *J. Nat. Prod.* (1988), **51,** 625-626).

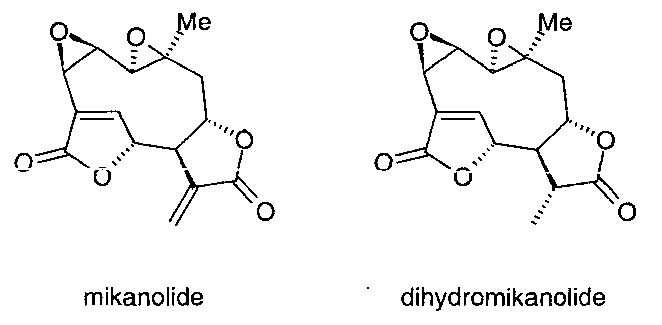

Les études des extraits de *Mikania* font état d'activité antimicrobienne contre *Staphylococus aureus* et *Candida albicans* (Facey et coll., *J. Pharm. Pharmacol.* (1999) **51**, 1455-1460; Mathur et coll., *Rev. Latinoam. Quim*. (1975), **6**, 201-205). Facey et son équipe ont notamment montré que les extraits organiques de *M. micrantha,* utilisés en médecine traditionnelle sous forme de décoctions destinées à traiter les infections cutanées et les ulcères, possédaient une activité anti-bactérienne et que le mikanolide et le dihydromikanolide étaient parmi les principes actifs desdits extraits organiques (Facey et coll., *J. Pharm. Pharmacol.* (1999), **51**(12), 1455-60). Des activités de mulluscicide, piscicide, et bactéricide ont aussi été décrites pour le dihydromikanolide (Vasquez et coll., AG Meeting, Amsterdam, Pays-Bas, 26-30 juillet 1999, poster n° 316).

A des fins d'élucidation de structures, certains dérivés simples du mikanolide et du dihydromikanolide, et notamment les composés de formules générales **(A1)** et **(A2)** représentées ci-dessous 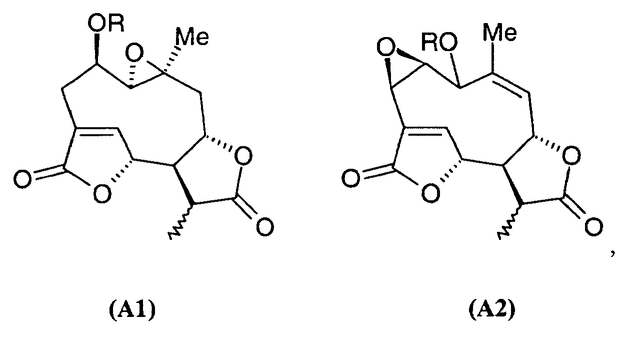
dans lesquelles R représente un atome d'hydrogène ou le groupe acétyle, ont été synthétisés et décrits dans Herz et coll., *J. Org. Chem.* (1970), **35,** 1453-1464. Cependant ce document ne décrit aucune activité pharmacologique pour ces composés.

Auparavant, S.M. Kupchan et son équipe (S.M. Kupchan et coll., *J. Am. Chem. Soc*. (1966), **88**(15), 3674-3676) avaient isolé deux sesquiterpène dilactones de type germacrane à partir de la plante *Elephantopus elatus* Bertol. (Compositae), à savoir l'éléphantine (**A3**) et l'éléphantopine (**A4**) représentées ci-dessous

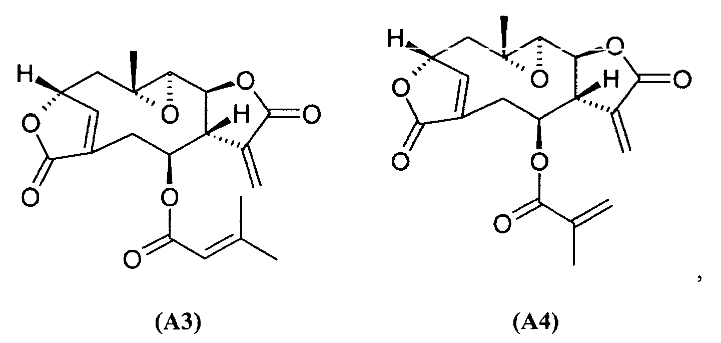

Ces deux composés étaient décrits comme inhibiteurs tumoraux.

La demanderesse a récemment décrit dans la demande de brevet français FR 2 801 792 que le mikanolide et le dihydromikanolide sont dotés d'une activité antiproliférative, laquelle serait liée à l'inhibition de l'ADN-polymérase (demande de brevet PCT WO 01/39720). Toutefois, les mikanolides ne possèdent pas toutes les propriétés physico-chimiques désirables pour une application médicamenteuse car ils ne sont ni très hydrosolubles, ni très stables, ce qui rend leur usage pharmaceutique relativement difficile, en particulier sous forme injectable.

A présent, la demanderesse a mis au point de nouveaux dérivés semi-synthétiques de mikanolide, lesquels peuvent être utilisés pour le traitement des maladies dues à des proliférations cellulaires anormales, notamment pour le traitement du cancer, des maladies virales et des infections bactériennes et parasitaires. Ces composés présentent l'avantage d'une meilleure solubilité et d'une meilleure stabilité que celle du mikanolide et du dihydromikanolide tout en conservant une activité de l'ordre de celle de ces derniers ou même supérieure.

L'invention a pour objet les composés de formule générale **(I)** 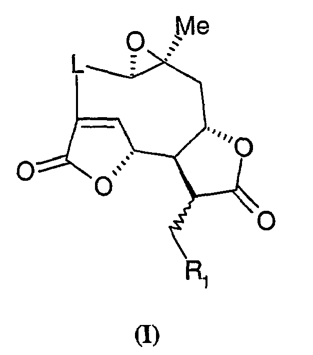
correspondant aux sous-formules générales **(I)**_{**1**} et **(I)**_{**2**} 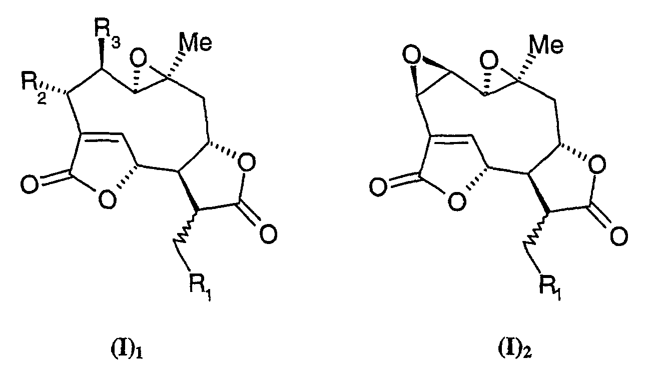
dans lesquelles
R₁ représente un atome d'hydrogène ou un radical SR₄ ou NR₄R₅ ;
R₂ représente SR₆ ou NR₆R₇;
R₃ représente OH, O(CO)R₁₄, OSiR₁₅R₁₆R₁₇, O(CO)OR₁₈ ou O(CO)NHR₁₈ ;
R₄ et R₆ représentent, indépendamment, un radical alkyle, un radical cycloalkyle, cycloalkylalkyle, hydroxyalkyle ou encore l'un des radicaux aryle ou aralkyle éventuellement substitués sur leur groupe aryle par un ou des radicaux choisis parmi les radicaux alkyle, hydroxy ou alkoxy,
R₅ et R₇ représentent, indépendamment, un atome d'hydrogène, un radical alkyle, un radical cycloalkyle, cycloalkylalkyle, hydroxyalkyle ou encore l'un des radicaux aryle ou aralkyle éventuellement substitués sur leur groupe aryle par un ou des radicaux choisis parmi les radicaux alkyle, hydroxy ou alkoxy,
R₄ et R₅ pouvant former ensemble avec l'atome d'azote qui les porte un hétérocycle de 5 à 7 chaînons, les chaînons complémentaires étant choisis parmi les radicaux -CR₈R₉-, -NR₁₀-, -O- et -S-, étant entendu toutefois qu'il ne peut se trouver qu'un seul chaînon choisi parmi -O- ou -S- dans ledit hétérocycle,
et R₆ et R₇ pouvant former ensemble avec l'atome d'azote qui les porte un hétérocycle de 5 à 7 chaînons, les chaînons complémentaires étant choisis parmi les radicaux -CR₁₁R₁₂-, -NR₁₃-, -O- et -S-, étant entendu toutefois qu'il ne peut se trouver qu'un seul chaînon choisi parmi -O- ou -S- dans ledit hétérocycle,
R₈, R₁₀, R₁₁ et R₁₃ représentent, indépendamment à chaque fois qu'ils interviennent, un atome d'hydrogène ou un radical alkyle, alkoxycarbonyle ou aralkyle, R₉ et R₁₂ représentant, indépendamment à chaque fois qu'ils interviennent, un atome d'hydrogène ou chacun de R₉ et R₁₂ pouvant former avec respectivement R₈ et R₁₁ un radical -O-(CH₂)₂-O- attaché de part et d'autre à l'atome de carbone qui le porte, un tel radical n'étant présent toutefois qu'une fois au maximum par radical NR₄R₅ ou NR₆R₇, représentent, indépendamment à chaque fois qu'ils interviennent, un atome d'hydrogène ou un radical alkyle ;
R₁₄ représente un radical alkyle, cycloalkyle ou adamantyle ou l'un des radicaux aryle, hétéroaryle, aralkyle ou hétéroaralkyle éventuellement substitués sur leur groupe aryle ou hétéroaryle par un ou des radicaux choisis parmi un atome halogène et les radicaux alkyle, haloalkyle, nitro, hydroxy, alkoxy, alkylthio ou phényle,
ou encore R₁₄ est tel que R₁₄-COOH représente un acide aminé naturel ou l'énantiomère optique d'un tel acide aminé ;
R₁₅, R₁₆ et R₁₇ représentent, indépendamment, un radical alkyle ou un radical phényle ;
R₁₈ représente un radical alkyle, cycloalkyle ou adamantyle ou l'un des radicaux aryle, hétéroaryle, aralkyle ou hétéroaralkyle éventuellement substitués sur leur groupe aryle ou hétéroaryle par un ou des radicaux choisis parmi un atome halogène et les radicaux alkyle, haloalkyle, nitro, hydroxy, alkoxy, alkylthio ou phényle ;
étant entendu toutefois que lorsque les composés répondent à la sous-formule générale **(I)**_{**2**}**,** alors R₁ ne représente pas un atome d'hydrogène ;
ou les sels de ces derniers.

Par alkyle, lorsqu'il n'est pas donné plus de précision, on entend un radical alkyle linéaire ou ramifié comptant de 1 à 12 atomes de carbone, et de préférence de 1 à 6 atomes de carbone. Par cycloalkyle, lorsqu'il n'est pas donné plus de précision, on entend un système monocyclique carboné comptant de 3 à 7 atomes de carbone. Par haloalkyle, on entend un radical alkyle dont au moins l'un des atomes d'hydrogène (et éventuellement tous) est remplacé par un atome halogène. Par aryle carbocyclique ou hétérocyclique, lorsqu'il n'est pas donné plus de précision, on entend un système carbocyclique ou hétérocyclique comprenant de un à trois cycles condensés dont l'un au moins est un cycle aromatique, un système étant dit hétérocyclique lorsque l'un au moins des cycles qui le composent comporte un ou des hétéroatomes (O, N ou S). Par aryle, lorsqu'il n'est pas donné plus de précision, on entend un radical aryle carbocyclique. Par hétéroaryle, on entend un radical aryle hétérocyclique. Par haloalkyle, on entend un radical alkyle dont au moins l'un des atomes d'hydrogène (et éventuellement tous) est remplacé par un atome halogène. Enfin, par atome halogène, on entend les atomes de fluor, de chlore, de brome ou d'iode.

Par radicaux alkoxy, hydroxyalkyle, cycloalkylalkyle, aralkyle et hétéroaralkyle, on entend respectivement les radicaux alkoxy, hydroxyalkyle, cycloalkylalkyle et aralkyle dont les radicaux alkyle, cycloalkyle, aryle et hétéroaralkyle ont les significations indiquées précédemment.

Par acide aminé naturel, on entend la valine (Val), la leucine (Leu), l'isoleucine (Ile), la méthionine (Met), la phénylalanine (Phe), l'asparagine (Asn), l'acide glutamique (Glu), la glutamine (Gln), l'histidine (His), la lysine (Lys), l'arginine (Arg), l'acide aspartique (Asp), la glycine (Gly), l'alanine (Ala), la sérine (Ser), la thréonine (Thr), la tyrosine (Tyr), le tryptophane (Trp), la cystéine (Cys) ou la proline (Pro).

Par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle. Par alkoxy, on entend notamment les radicaux méthoxy, éthoxy et isopropoxy, et en particulier les radicaux méthoxy et éthoxy. Par cycloalkyle, on entend notamment les radicaux cyclopropyle, cyclobutyle, cyclopentyle et cyclohexyle. Par haloalkyle, on entend notamment le radical trifluorométhyle. Par aryle carbocyclique, on entend notamment les radicaux phényle, naphtyle et phénantryle, de préférence les radicaux phényle et naphtyle et plus préférentiellement le radical phényle. Par aryle hétérocyclique, on entend notamment les radicaux pyrrolyle, furannyle, benzofurannyle, thiényle, benzothiényle, pyridyle, pyrimidinyle, triazinyle, imidazolyle, oxazolyle, thiazolyle, indolyle et quinolyle, et de préférence les radicaux furannyle, benzofurannyle, thiényle et benzothiényle. Par aralkyle, on entend notamment un radical phénalkyle, et de préférence le radical benzyle. Par hétéroaralkyle, on entend notamment un radical thiénylalkyle, furannylalkyle, pyrrolylalkyle et thiazolylalkyle (le radical alkyle desdits radicaux étant de préférence un radical méthyle), et de préférence un radical thiénylalkyle (de préférence thiénylméthyle).

Un composé de formule générale **(I)** présentant au moins l'une des caractéristiques suivantes sera préféré :
- le composé répond à la sous-formule générale **(I)**₁ **;**
- R₁ représente un atome d'hydrogène ou un radical NR₄R₅ ;
- R₂ représente un radical NR₆R₇ ;
- R₃ représente OH ou un radical O(CO)R₁₄, OSiR₁₅R₁₆R₁₇ ou O(CO)NHR₁₈.

Plus préférentiellement, un composé de formule générale **(I)** sera tel qu'il présente au moins l'une des caractéristiques suivantes :
- le composé répond à la sous-formule générale **(I)**_{**1**} **;**
- R₁ représente un atome d'hydrogène ;
- R₂ représente un radical NR₆R₇ ;
- R₃ représente un radical O(CO)R₁₄, OSiR₁₅R₁₆R₁₇ ou O(CO)NHR₁₈.

Tout particulièrement, un composé de formule générale **(I)** sera tel qu'il présente au moins l'une des caractéristiques suivantes :
- le composé répond à la sous-formule générale **(I)**₁ ;
- R₁ représente un atome d'hydrogène ;
- R₂ représente un radical NR₆R₇ et de préférence un radical NR₆R₇ dans lequel R₆ et R₇ sont choisis indépendamment parmi un atome d'hydrogène et un radical alkyle ;
- R₃ représente un radical O(CO)R₁₄, OSiR₁₅R₁₆R₁₇ ou O(CO)NHR₁₈.

Par ailleurs, R₂ représentera tout préférentiellement un radical NR₆R₇ dans lequel R₆ et R₇ sont des radicaux alkyle, et en particulier un radical NR₆R₇ dans lequel R₆ et R₇ sont des radicaux méthyle. R₃ représentera tout préférentiellement un radical O(CO)NHR₁₈.

De préférence également, R₄ représentera un radical alkyle ou aralkyle, et R₅ représentera un atome d'hydrogène ou un radical alkyle, ou encore R₄ et R₅ formeront ensemble avec l'atome d'azote qui les porte un hétérocycle de 5 à 7 chaînons, les chaînons complémentaires étant choisis parmi les radicaux -CR₈R₉-, -NR₁₀-, -O- et -S-. De préférence, R₈ représentera, indépendamment à chaque fois qu'il intervient, un atome d'hydrogène ou un radical alkyle (et de préférence un atome d'hydrogène) et R₉ représentera à chaque fois qu'il intervient un atome d'hydrogène. De préférence, R₁₀ représentera, indépendamment à chaque fois qu'il intervient, un atome d'hydrogène ou un radical alkyle.

De préférence encore, R₆ représentera un radical alkyle ou aralkyle, et R₇ représentera un atome d'hydrogène ou un radical alkyle, ou encore R₆ et R₇ formeront ensemble avec l'atome d'azote qui les porte un hétérocycle de 5 à 7 chaînons, les chaînons complémentaires étant choisis parmi les radicaux -CR₁₁R₁₂-, -NR₁₃-, -O- et -S-. De préférence, R₁₁ représentera, indépendamment à chaque fois qu'il intervient, un atome d'hydrogène ou un radical alkyle ou alkoxycarbonyle (et de préférence un atome d'hydrogène) ou encore R₁₁ et R₁₂ représenteront une fois ensemble un radical -O-(CH₂)₂-O- attaché de part et d'autre à l'atome de carbone qui le porte. De préférence, R₁₃ représentera, indépendamment à chaque fois qu'il intervient, un atome d'hydrogène ou un radical alkyle.

En outre, R₁₄ représentera de préférence un radical alkyle ou cycloalkyle, ou l'un des radicaux aryle ou hétéroaryle éventuellement substitués par un atome halogène ou un radical haloalkyle ou phényle. Plus préférentiellement, R₁₄ représentera un radical cycloalkyle ou l'un des radicaux aryle ou hétéroaryle éventuellement substitués par un atome halogène ou un radical haloalkyle. De façon encore plus préférée, R₁₄ représentera un radical cyclohexyle ou l'un des radicaux phényle, thiényle ou benzothiényle éventuellement substitués par un atome halogène.

Par ailleurs, R₁₅, R₁₆ et R₁₇ représenteront de préférence des radicaux alkyle. De façon particulièrement préférée, l'un des radicaux R₁₅, R₁₆ et R₁₇ représentera un radical *tert*-butyle et les deux autres représenteront des radicaux méthyle.

Enfin, R₁₈ représentera de préférence un radical alkyle, cycloalkyle ou adamantyle, ou l'un des radicaux aryle ou hétéroaryle éventuellement substitués par un atome halogène ou un radical alkyle, haloalkyle, alkoxy, alkylthio ou phényle. Plus préférentiellement, R₁₈ représentera un radical cycloalkyle ou l'un des radicaux aryle ou hétéroaryle éventuellement substitués par un radical alkyle, alkoxy ou alkylthio. De façon encore plus préférée, R₁₈ représentera l'un des radicaux phényle, thiényle ou benzothiényle éventuellement substitués par un radical alkyle, alkoxy ou alkylthio.

Par ailleurs, lorsque R₄ et R₅ forment ensemble avec l'atome d'azote qui les porte un hétérocycle de 5 à 7 chaînons, le radical NR₄R₅ représente de préférence l'un des radicaux pyrrolyle, pipéridyle, pipérazinyle, morpholinyle ou thiomorpholinyle éventuellement substitué par un radical alkyle (celui-ci étant de préférence un radical méthyle ou éthyle, et plus préférentiellement un radical méthyle) sur l'un de ses atomes de carbone ou d'azote, ou par un radical -O-(CH₂)₂-O- attaché de part et d'autre à un atome de carbone. Plus préférentiellement, lorsque R₄ et R₅ forment ensemble avec l'atome d'azote qui les porte un hétérocycle de 5 à 7 chaînons, le radical NR₄R₅ représentera l'un des radicaux pyrrolyle, pipéridyle, pipérazinyle, morpholinyle ou thiomorpholinyle éventuellement substitué par un radical alkyle (celui-ci étant de préférence un radical méthyle) sur l'un de ses atomes de carbone ou d'azote.

De même, lorsque R₆ et R₇ forment ensemble avec l'atome d'azote qui les porte un hétérocycle de 5 à 7 chaînons, le radical NR₆R₇ représente de préférence l'un des radicaux pyrrolyle, pipéridyle, pipérazinyle, morpholinyle ou thiomorpholinyle éventuellement substitué par un radical alkyle (celui-ci étant de préférence un radical méthyle ou éthyle, et plus préférentiellement un radical méthyle) sur l'un de ses atomes de carbone ou d'azote, ou par un radical -O-(CH₂)₂-O- attaché de part et d'autre à un atome de carbone. Plus préférentiellement, lorsque R₆ et R₇ forment ensemble avec l'atome d'azote qui les porte un hétérocycle de 5 à 7 chaînons, le radical NR₆R₇ représentera l'un des radicaux pyrrolyle, pipéridyle, pipérazinyle, morpholinyle ou thiomorpholinyle éventuellement substitué par un radical alkyle (celui-ci étant de préférence un radical méthyle) sur l'un de ses atomes de carbone ou d'azote.

L'invention concerne en particulier les composés suivants décrits dans les exemples :
- 12-diisopropylaminométhyl-7-méthyl-3,6,10,15-tétraoxapentacyclo[12.2.1.0^{2,4}.0^{5,7}.0^{9,13}]heptadéc-1(17)-ène-11,16-dione ;
- 12-diméthylamino-3-diméthylaminométhyl-11-hydroxy-8-méthyl-5,9,15-trioxatétracyclo[11.2.1.0^{2,6}.0^{8,10}]hexadéc-13(16)-ène-4,14-dione ;
- 12-benzyl(méthyl)amino-3-benzyl(méthyl)aminométhyl-11-hydroxy-8-méthyl-5,9,15-trioxatétracyclo[11.2.1.0^{2,6}.0^{8,10}]hexadéc-13(16)-ène-4,14-dione ;
- 11-hydroxy-8-méthyl-12-morpholino-3-morpholinométhyl-5,9,15-trioxatétracyclo[11.2.1.0^{2,6}.0^{8,10}]hexadéc-13(16)-ène-4,14-dione ;
- 12-diméthylamino-11-hydroxy-3,8-diméthyl-5,9,15-trioxatétracyclo[11.2.1.0^{2,6}.0^{8,10}]hexadéc-13(16)-ène-4,14-dione ;
- 11-hydroxy-3,8-diméthyl-12-(4-méthylpipéridino)-5,9,15-trioxatétracyclo[11.2.1.0^{2,6}.0^{8,10}]hexadéc-13(16)-ène-4,14-dione ;
- 11-hydroxy-3,8-diméthyl-12-pyrrolidino-5,9,15-trioxatétracyclo[11.2.1.0^{2,6}.0^{8,10}]hexadéc-13(16)-ène-4,14-dione ;
- 1-[11-hydroxy-3,8-diméthyl-4,14-dioxo-5,9,15-trioxatétracyclo[11.2.1.0^{2,6}.0^{8,10}]hexadéc-13(16)-èn-12-yl]-4-pipéridinecarboxylate d'éthyle ;
- 12-(4-benzylpipéridino)-11-hydroxy-3,8-diméthyl-5,9,15-trioxatétracyclo[11.2.1.0^{2,6}.0^{8,10}]hexadéc-13(16)-ène-4,14-dione ;
- 11-hydroxy-3,8-diméthyl-12-pipéridino-5,9,15-trioxatétracyclo[11.2.1.0^{2,6}.0^{8,10}]hexadéc-13(16)-ène-4,14-dione ;
- 12-(1,4-dioxa-8-azaspiro[4.5]déc-8-yl)-11-hydroxy-3,8-diméthyl-5,9,15-trioxatétracyclo[11.2.1.0^{2,6}.0^{8,10}]hexadéc-13(16)-ène-4,14-dione ;
- 11-hydroxy-3,8-diméthyl-12-morpholino-5,9,15-trioxatétracyclo[11.2.1.0^{2,6}.0^{8,10}]hexadéc-13(16)-ène-4,14-dione ;
- 11-(*tert*-butyldiméthylsiloxy)-12-diméthylamino-3,8-diméthyl-5,9,15-trioxatétracyclo[11.2.1.0^{2,6}.0^{8,10}]hexadéc-13(16)-ène-4,14-dione ;
- acétate de 3,8-diméthyl-12-(4-méthylpipéridino)-4,14-dioxo-5,9,15-trioxatétracyclo[11.2.1.0^{2,6}.0^{8,10}]hexadéc-13(16)-èn-11-yle ;
- 3,8-diméthyl-12-(4-méthylpipéridino)-4,14-dioxo-11-phénylcarbonyloxy-5,9,15-trioxatétracyclo[11.2.1.0^{2,6}.0^{8,10}]hexadéc-13(16)-ène ;
- 3,8-diméthyl-12-(4-méthylpipéridino)-4,14-dioxo-5,9,15-trioxatétracyclo[11.2.1.0^{2,6}.0^{8,10}]hexadéc-13(16)-èn-11-ylcarbonate d'éthyle ;
- 11-hydroxy-12-isobutylsulfanyl-3-isobutylsulfanylméthyl-8-méthyl-5,9,15-trioxatétracyclo[11.2.1.0^{2,6}.0^{8,10}]hexadéc-13(16)-ène-4,14-dione ;
- 11-hydroxy-12-isobutylsulfanyl-3,8-diméthyl-5,9,15-trioxatétracyclo[11.2.1.0^{2,6}.0^{8,10}]hexadéc-13(16)-ène-4,14-dione ;
- benzoate de 12-(diméthylamino)-3,8-diméthyl-4,14-dioxo-5,9,15-trioxatétracyclo[11.2.1.02,6.08,10]hexadéc-13(16)-èn-11-yle ;
- acétate de 12-(diméthylamino)-3,8-diméthyl-4,14-dioxo-5,9,15-trioxatétracyclo[11.2.1.02,6.08,10]hexadéc-13(16)-èn-11-yle ;
- cyclohexanecarboxylate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 4-fluorobenzoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- heptanoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 4-(trifluorométhyl)benzoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 2-thiophènecarboxylate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 3,3-diméthylbutanoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 1-benzothiophène-2-carboxylate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 2-furoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 5-nitro-2-furoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 2-thiénylacétate de 12-(diméthylamino)-3,8-diméthyl-4,14-dioxo-5,9,15-trioxatétracyclo[11.2.1.02,6.08,10]hexadéc-13(16)-èn-11-yle ;
- phénoxyacétate de 12-(diméthylamino)-3,8-diméthyl-4,14-dioxo-5,9,15-trioxatétracyclo[11.2.1.02,6.08,10]hexadéc-13(16)-èn-11-yle ;
- 4-*tert*-butylphénylcarbamate de 8-(diméthylamino)-3,10a-diméthyl-2,6-dioxodécahydro-4,7-méthènofuro[3,2-*c*]oxiréno[*f*]oxacycloundécin-9-yle ;
- thién-2-ylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 2-méthoxyphénylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 2(méthylthio)phénylcarbamate de 8-(diméthylamino)-3,10a-diméthyl-2,6-dioxodécahydro-4,7-méthèno-furo[3,2-*c*]oxiréno[*f*]oxacycloundécin-9-yle ;
- 2-éthoxyphénylcarbamate de 8-(diméthylamino)-3,10a-diméthyl-2,6-dioxodécahydro-4,7-méthènofuro[3,2-*c*]oxiréno[*f*]oxacycloundécin-9-yle ;
- thién-3-ylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 1-benzothién-3-ylcarbamate de 8-(diméthylamino)-3,10a-diméthyl-2,6-dioxodécahydro-4,7-méthènofuro[3,2-*c*]oxiréno[*f*]oxacycloundécin-9-yle ;
- *N*-(*ter*-butoxycarbonyl)glycinate de 8-(diméthylamino)-3,10a-diméthyl-2,6-dioxodécahydro-4,7-méthèno-furo[3,2-*c*]oxiréno[*f*]oxacycloundécin-9-yle ;
- thién-3-ylacétate de 8-(diméthylamino)-3,10a-diméthyl-2,6-dioxodécahydro-4,7-méthènofuro[3,2-*c*]oxiréno[*f*]oxacycloundécin-9-yle ;
- 1-benzothién-3-ylacétate de 8-(diméthylamino)-3,10a-diméthyl-2,6-dioxodécahydro-4,7-méthènofuro[3,2-*c*]oxiréno[*f*]oxacycloundécin-9-yle ;
- thiophène-3-carboxylate de 8-(diméthylamino)-3,10a-diméthyl-2,6-dioxodécahydro-4,7-méthènofuro[3,2-*c*]oxiréno[*f*]oxacycloundécin-9-yle ;
- 5-phénylthién-2-ylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 1-adaniantylcarbalnate de 8-(diméthylamino)-3,10a-diméthyl-2,6-dioxodécahydro-4,7-méthènofuro[3,2-*c*]oxiréno[*f*]oxacycloundécin-9-yle ;
- 2-naphtylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 2-*tert*-butyl-6-méthylphénylcarbamate de 8-(diméthylamino)-3,10a-diméthyl-2,6-dioxodécahydro-4,7-méthèno-furo[3,2-*c*]oxiréno[*f*]oxacycloundécin-9-yle ;
- 2,5-diméthoxyphénylcarbamate de 8-(diméthylamino)-3,10a-diméthyl-2,6-dioxodécahydro-4,7-méthèno-furo[3,2-*c*]oxiréno[*f*]oxacycloundécin-9-yle ;

ainsi que leurs sels.

L'invention concerne tout particulièrement les composés suivants :
- 12-diméthylamino-11-hydroxy-3,8-diméthyl-5,9,15-trioxatétracyclo[11.2.1.0^{2,6}.0^{8,10}]hexadéc-13(16)-ène-4,14-dione ;
- 11-(*tert*-butyldiméthylsiloxy)-12-diméthylamino-3,8-diméthyl-5,9,15-trioxatétracyclo[11.2.1.0^{2,6}.0^{8,10}]hexadéc-13(16)-ène-4,14-dione ;
- benzoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- cyclohexanecarboxylate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 4-fluorobenzoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 2-thiophènecarboxylate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 3,3-diméthylbutanoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 1-benzothiophène-2-carboxylate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 4-*tert*-butylphénylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 2-éthoxyphénylcarbamate de 8-(diméthylamino)-3,10a-diméthyl-2,6-dioxodécahydro-4,7-méthènofuro[3,2-*c*]oxiréno[*f*]oxacycloundécin-9-yle ;
   ainsi que leurs sels.

En ce qui concerne les sels de composés de formule générale **(I)**, les maléates, fumarates, méthanesulfonates et chlorhydrates de composés de formule générale **(I)** seront préférés, et notamment ceux décrits dans les exemples, à savoir :
- le maléate de 11-hydroxy-3,8-diméthyl-4,14-dioxo-5,9,15-trioxatétracyclo[11.2.1.0^{2,6}.0^{8,10}]hexadéc-13(16)-ène-12-yl(diméthyl)ammonium ;
- le fumarate de 11-hydroxy-3,8-diméthyl-4,14-dioxo-5,9,15-trioxatétracyclo[11.2.1.0^{2,6}.0^{8,10}]hexadéc-13(16)-ène-12-yl(diméthyl)ammonium ;
- le méthanesulfonate de 11-hydroxy-3,8-diméthyl-4,14-dioxo-5,9,15-trioxatétracyclo[11.2.1.0^{2,6}.0^{8,10}]hexadéc-13(16)-ène-12-yl(diméthyl)ammonium ;
- le chlorhydrate de 11-{[*tert*-butyl(diméthyl)silyl]oxy}-12-(diméthylamino)-3,8-diméthyl-5,9,15-trioxatétracyclo[11.2.1.0^{2,6}.0^{8,10}]hexadéc-13(16)-ène-4,14-dione ; et
- le chlorhydrate de 2-thiophènecarboxylate de 12-(diméthylamino)-3,8-diméthyl-4,14-dioxo-5,9,15-trioxatétracyclo[11.2.1.0^{2,6}.0^{8,10}]hexadéc-13(16)-èn-11-yle.

L'invention a encore pour objet les composés de formule générale **(I)'** 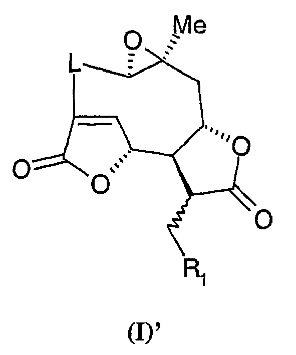
correspondant aux sous-formules générales **(I)'**_{**1**} et **(I)'**_{**2**} 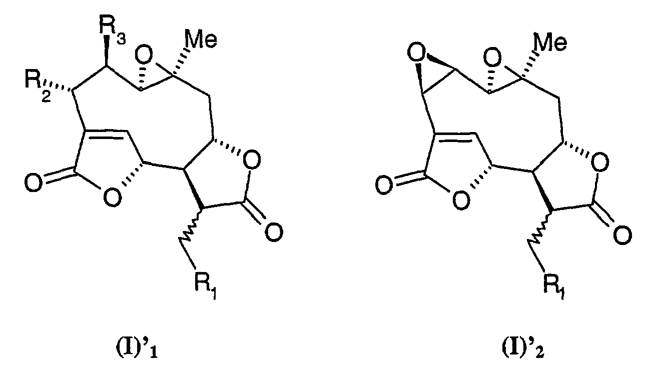
dans lesquelles
R₁ représente un atome d'hydrogène ou un radical SR₄ ou NR₄R₅ ;
R₂ représente SR₆ ou NR₆R₇;
R₃ représente OH, O(CO)R₁₄, O(CO)OR₁₄ ou OSiR₁₅R₁₆R₁₇ ;
R₄ et R₆ représentent, indépendamment, un radical alkyle, un radical cycloalkyle, cycloalkylalkyle, hydroxyalkyle ou encore l'un des radicaux aryle ou aralkyle éventuellement substitués sur leur groupe aryle par un ou des radicaux choisis parmi les radicaux alkyle, hydroxy ou alkoxy,
R₅ et R₇ représentent, indépendamment, un atome d'hydrogène, un radical alkyle, un radical cycloalkyle, cycloalkylalkyle, hydroxyalkyle ou encore l'un des radicaux aryle
ou aralkyle éventuellement substitués sur leur groupe aryle par un ou des radicaux choisis parmi les radicaux alkyle, hydroxy ou alkoxy,
R₄ et R₅ pouvant former ensemble avec l'atome d'azote qui les porte un hétérocycle de 5 à 7 chaînons, les chaînons complémentaires étant choisis parmi les radicaux -CR₈R₉-, -NR₁₀-, -O- et -S-, étant entendu toutefois qu'il ne peut se trouver qu'un seul chaînon choisi parmi -O- ou -S- dans ledit hétérocycle,
et R₆ et R₇ pouvant former ensemble avec l'atome d'azote qui les porte un hétérocycle de 5 à 7 chaînons, les chaînons complémentaires étant choisis parmi les radicaux -CR₁₁R₁₂-, -NR₁₃-, -O- et -S-, étant entendu toutefois qu'il ne peut se trouver qu'un seul chaînon choisi parmi -O- ou -S- dans ledit hétérocycle,
R₈, R₁₀, R₁₁ et R₁₃ représentent, indépendamment à chaque fois qu'ils interviennent, un atome d'hydrogène ou un radical alkyle, alkoxycarbonyle ou aralkyle,
R₉ et R₁₂ représentant, indépendamment à chaque fois qu'ils interviennent, un atome d'hydrogène ou chacun de R₉ et R₁₂ pouvant former avec respectivement R₈ et R₁₁ un radical -O-(CH₂)₂-O- attaché de part et d'autre à l'atome de carbone qui le porte, un tel radical n'étant présent toutefois qu'une fois au maximum par radical NR₄R₅ ou NR₆R₇, représentent, indépendamment à chaque fois qu'ils interviennent, un atome d'hydrogène ou un radical alkyle ;
R₁₄ représente un radical alkyle ou cycloalkyle ou l'un des radicaux aryle, hétéroaryle, aralkyle ou hétéroaralkyle éventuellement substitués sur leur groupe aryle ou hétéroaryle par un ou des radicaux choisis parmi un atome halogène et les radicaux alkyle, haloalkyle, nitro, hydroxy ou alkoxy ;
R₁₅, R₁₆ et R₁₇ représentent, indépendamment, un radical alkyle ou un radical phényle ;
étant entendu toutefois que lorsque les composés répondent à la sous-formule générale **(I)'**_{**2**}, alors R₁ ne représente pas un atome d'hydrogène ;
ou les sels de ces derniers.

L'invention a encore pour objet les composés de formule générale **(I)**" 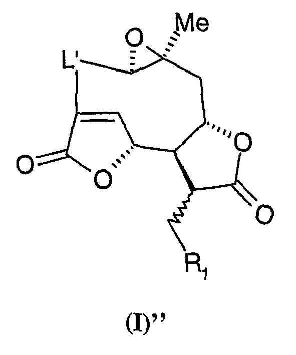
correspondant aux sous-formules générales **(I)"**_{**1**} et **(I)"**_{**2**} 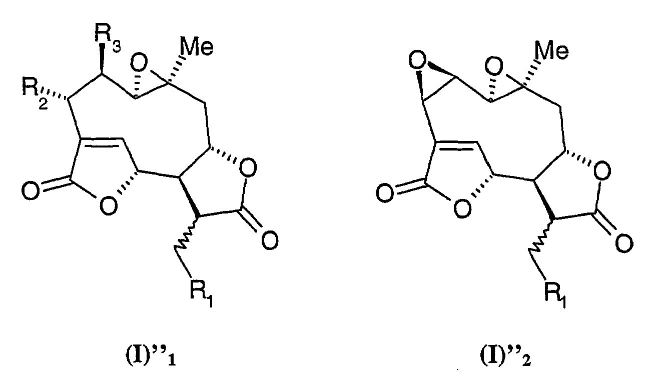
dans lesquelles
R₁ représente un atome d'hydrogène ou un radical SR₄ ou NR₄R₅ ;
R₂ représente SR₆ ou NR₆R₇ ;
R₃ représente OH, O(CO)R₁₄, O(CO)OR₁₄, ou OSiR₁₅R₁₆R₁₇ ;
R₄, R₅, R₆ et R₇ représentent, indépendamment, un atome d'hydrogène, un radical alkyle, un radical cycloalkyle, cycloalkylalkyle, hydroxyalkyle ou encore l'un des radicaux aryle ou aralkyle éventuellement substitués sur leur groupe aryle par un ou des radicaux choisis parmi les radicaux alkyle, hydroxy ou alkoxy,
R₄ et R₅ pouvant former ensemble avec l'atome d'azote qui les porte un hétérocycle de 5 à 7 chaînons, les chaînons complémentaires étant choisis parmi les radicaux -CR₈R₉-, -NR₁₀-, -O- et -S-, étant entendu toutefois qu'il ne peut se trouver qu'un seul chaînon choisi parmi -O- ou -S- dans ledit hétérocycle,
et R₆ et R₇ pouvant former ensemble avec l'atome d'azote qui les porte un hétérocycle de 5 à 7 chaînons, les chaînons complémentaires étant choisis parmi les radicaux -CR₁₁R₁₂-, -NR₁₃-, -O- et -S-, étant entendu toutefois qu'il ne peut se trouver qu'un seul chaînon choisi parmi -O- ou -S- dans ledit hétérocycle,
R₈, R₁₀, R₁₁ et R₁₃ représentent, indépendamment à chaque fois qu'ils interviennent, un atome d'hydrogène ou un radical alkyle, alkoxycarbonyle ou aralkyle,
R₉ et R₁₂ représentant, indépendamment à chaque fois qu'ils interviennent, un atome d'hydrogène ou chacun de R₉ et R₁₂ pouvant former avec respectivement R₈ et R₁₁ un radical -O-(CH₂)₂-O- attaché de part et d'autre à l'atome de carbone qui le porte, un tel radical n'étant présent toutefois qu'une fois au maximum par radical NR₄R₅ ou NR₆R₇, représentent, indépendamment à chaque fois qu'ils interviennent, un atome d'hydrogène ou un radical alkyle ;
R₁₄, R₁₅, R₁₆ et R₁₇ représentent, indépendamment, un atome d'hydrogène, un radical alkyle ou l'un des radicaux aryle ou aralkyle éventuellement substitués sur leur groupe aryle par un ou des radicaux choisis parmi les radicaux alkyle, hydroxy ou alkoxy ;
étant entendu toutefois que lorsque les composés répondent à la sous-formule générale **(I)"**_{**2**}**,** alors R₁ ne représente pas un atome d'hydrogène ;
ou les sels de ces derniers.

Les composés de la présente invention peuvent inhiber les proliférations cellulaires anormales chez un patient, par exemple un mammifère tel que l'homme, par administration à ce patient d'une quantité thérapeutiquement efficace d'un composé de formule générale **(I), (I)'** ou **(I)"** ou d'un sel pharmaceutiquement acceptable d'un tel composé.

Les composés formule générale **(I), (I)'** ou **(I)"** possèdent une activité anti-tumorale. Ils peuvent être utilisés pour le traitement des tumeurs chez un patient par administration audit patient d'une quantité thérapeutiquement efficace d'un composé de formule générale **(I), (I)'** ou **(I)"** ou d'un sel pharmaceutiquement acceptable d'un tel composé. Des exemples de tumeurs ou de cancers comprennent les cancers de l'oesophage, de l'estomac, des intestins, du rectum, de la cavité orale, du pharynx, du larynx, du poumon, du colon, du sein, du cervix uteri, du *corpus endometrium*, des ovaires, de la prostate, des testicules, de la vessie, des reins, du foie, du pancréas, des os, des tissus conjonctifs, de la peau, par exemple les mélanomes, des yeux, du cerveau et du système nerveux central, ainsi que le cancer de la thyroïde, la leucémie, la maladie de Hodgkin, les lymphomes autres que ceux de Hodgkin, les myélomes multiples et autres.

Ils peuvent également être utilisés pour le traitement des infections parasitaires par l'inhibition des hémoflagellates (par exemple dans la trypanosomie ou les infections leishmania) ou par inhibition de la plasmodie (comme par exemple dans la malaria), mais aussi le traitement des infections ou maladies virales.

Ces propriétés rendent les composés de l'invention aptes à une utilisation pharmaceutique. L'invention a donc également pour objet, à titre de médicaments, les composés de formule générale **(I), (I)'** ou **(I)"** décrits précédemment ou leurs sels pharmaceutiquement acceptables. Elle concerne aussi des compositions pharmaceutiques contenant ces composés ou leurs sels pharmaceutiquement acceptables. Elle a en outre pour objet l'utilisation de ces composés ou de leurs sels pharmaceutiquement acceptables pour fabriquer des médicaments destinés à inhiber les ADN polymérases, et notamment à traiter les maladies dues à des proliférations cellulaires anormales (en particulier le cancer ou l'athérosclérose, l'hyperplasie bénigne de la prostate et les fibroses), ainsi que les maladies virales et les maladies parasitaires, en particulier celles causées par des protozoaires (par exemple la malaria) ou des protistes (par exemple les maladies causées par les amibes). Enfin, l'invention concerne l'utilisation des composés de formule générale **(I), (I)'** ou **(I)"** décrits précédemment ou de leurs sels pharmaceutiquement acceptables pour préparer un médicament destiné à traiter des maladies d'origine bactérienne.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", *Int. J. Pharm*. (1986), **33**, 201-217.

Les compositions pharmaceutiques contenant un composé de l'invention peuvent être sous forme solide comme, par exemple, les poudres, pilules, granules, comprimés, liposomes, gélules ou suppositoires. Les pilules, les comprimés ou les gélules peuvent être revêtus d'une substance capable de protéger la composition de l'action de l'acide gastrique ou des enzymes dans l'estomac du sujet pendant une période de temps suffisante pour permettre à cette composition de passer non digérée dans l'intestin grêle de ce dernier. Le composé peut aussi être administré localement, par exemple à l'emplacement même d'une tumeur. Le composé peut aussi être administré selon un processus de libération prolongée (par exemple en utilisant une composition à libération prolongée ou une pompe de perfusion). Les supports solides appropriés peuvent être, par exemple, le phosphate de calcium, le stéarate de magnésium, le carbonate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent également se présenter sous forme liquide comme, par exemple, des solutions, des émulsions, des suspensions ou une formulation à libération prolongée. Les supports liquides appropriés peuvent être, par exemple, l'eau, les solvants organiques tels que le glycérol ou les glycols tel que le polyéthylène glycol, de même que leurs mélanges, dans des proportions variées, dans l'eau.

L'administration d'un médicament selon l'invention pourra se faire par voie topique, orale, parentérale, par injection intramusculaire, etc.

La dose d'un composé selon la présente invention, à prévoir pour le traitement des maladies ou troubles mentionnés ci-dessus, varie suivant le mode d'administration, l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement efficace".

Les mêmes préférences que celles indiquées précédemment pour les composés de formule générale **(I)** sont applicables *mutatis mutandis* aux composés de formule générale **(I)'** ou **(I)"**, aux médicaments de formule générale **(I)**, **(I)'** ou **(I)"**, aux compositions pharmaceutiques contenant un composé de formule générale **(I), (I)'** ou **(I)"** et aux utilisations d'un composé de formule générale **(I), (I)'** ou **(I)"** pour préparer des médicaments.

Conformément à l'invention, on peut préparer les composés de formule générale **(I), (I)'** ou **(I)"** par les procédés décrits ci-après.

### Préparation des composés de formule générale (I), (I)' ou (I)" et de leurs sels

### Préparation des composés de sous-formule générale (I)₁ :

### CAS 1: R₁ = H :

La préparation de ce type de composés est résumée dans le schéma 1 ci-après. Le dihydromikanolide par addition d'un nucléophile tel qu'une amine primaire ou secondaire HNR₆R₇, ou encore d'un thiol R₆SH en présence d'une base, dans un solvant inerte tel que le tétrahydrofuranne ou l'acétone, à une température de préférence comprise entre 0 °C et 50 °C, et plus préférentiellement à température ambiante.

Dans le cas où R₃ n'est pas OH, on traite l'intermédiaire obtenu avec l'un des réactifs de formule générale R₁₄(CO)-Hal (ou un réactif équivalent comme par exemple l'anhydride (R₁₄(CO))₂O), R₁₈O(CO)-Hal, Hal-Si R₁₅R₁₆R₁₇ (Hal représentant un atome halogène) ou R₁₈-NCO pour obtenir le composé final souhaité. D'une façon générale, cette réaction s'effectue dans un solvant aprotique tel que le dichlorométhane, le trichloroéthane, l'acétonitrile, le tétrahydrofuranne ou le toluène, à une température de préférence comprise entre 0 °C et 110 °C et éventuellement en présence d'une base telle que la triéthylamine ou la 4-diméthylaminopyridine. Ces types de réaction sont bien connus de l'homme du métier (qui pourra notamment consulter utilement l'ouvrage de référence suivant : Greene et coll., *"Protective groups in Organic Synthesis"*, 2nd edition, Wiley, New-York, 1991) en raison de leur usage fréquent pour protéger une fonction alcool ou amine. Par exemple, en ce qui concerne la réaction de silylation, celle-ci est généralement effectuée par traitement du composé alcoolique avec un chlorure de silyle en présence d'une base, dans un solvant aprotique à une température comprise entre 0 °C et 50 °C.

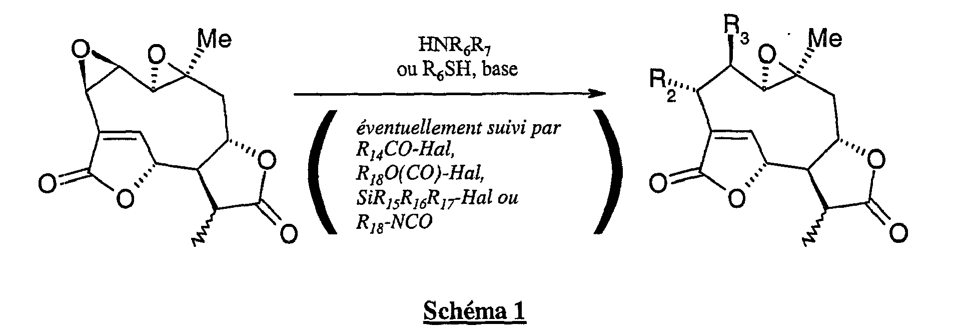

Une méthode supplémentaire pour l'obtention de composés avec R₃ = OCOR₁₄ consiste à traiter l'alcool intermédiaire ave l'acide R₁₄-COOH en présence d'une base, comme par exemple la diméthylaminopyridine, et d'un agent de couplage, comme par exemple le chlorhydrate de 1-(3-diméthylaminopropyl)-3-éthylcarbodiimide (EDC.HCl).

### CAS 2 : R₁ = R₂≠H :

Les composés de formule **(I)**_{**1**} dans laquelle R₁ = R₂ ≠ H et R₃ représente un groupe hydroxyle peuvent être préparés à partir du mikanolide par addition d'un nucléophile tel qu'une amine primaire ou secondaire HNR₄R₅, ou encore d'un thiol R₄SH en présence d'une base, dans un solvant inerte tel que le tétrahydrofuranne ou l'acétone, à une température de préférence comprise entre 0 °C et 50 °C, et plus préférentiellement à température ambiante.

Dans le cas où R₃ n'est pas OH, une deuxième réaction est effectuée à l'aide d'un composé de formule générale R₁₄(CO)-Hal (ou un réactif équivalent comme par exemple l'anhydride (R₁₄(CO))₂O), R₁₈O(CO)-Hal, Hal-SiR₁₅R₁₆R₁₇ (Hal représentant un atome halogène) ou R₁₈-NCO pour obtenir le composé final souhaité. Cette réaction peut être effectuée de façon analogue à celle exposée dans le CAS 1.

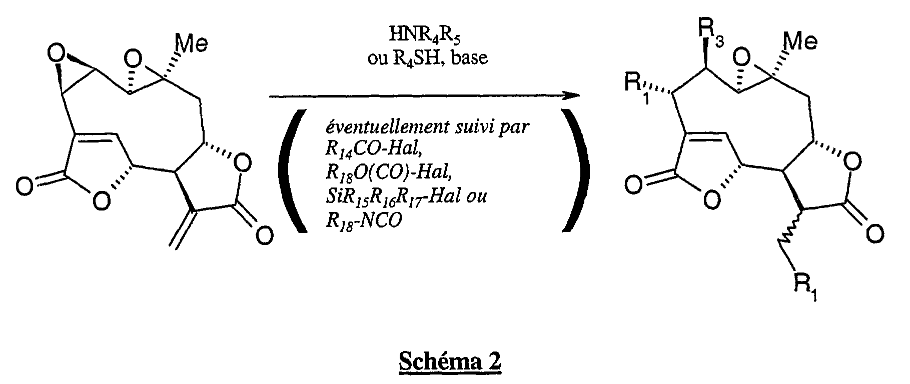

Une méthode supplémentaire pour l'obtention de composés avec R₃ = OCOR₁₄ consiste à traiter l'alcool intermédiaire ave l'acide R₁₄-COOH en présence d'une base, comme par exemple la diméthylaminopyridine, et d'un agent de couplage, comme par exemple le chlorhydrate de 1-(3-diméthylaminopropyl)-3-éthylcarbodiimide (EDC.HCl).

### CAS 3 : R₁ ≠ H et R₁ ≠ R₂ :

Dans ce cas, le composé de formule générale **(I)**_{**2**} sera soumis aux mêmes réactions que dans le CAS 1 pour donner le composé final souhaité dans lequel R₁ ≠ R₂.

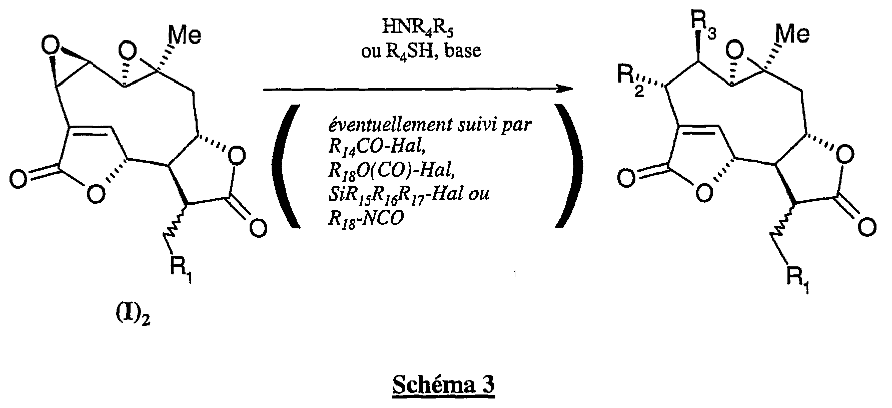

### Préparation des composés de sous-formule générale (I)₂ :

Les composés de sous-formule **(I)**_{**2**} peuvent être préparés, schéma 4, à partir du mikanolide par addition d'un nucléophile tel qu'une amine primaire ou secondaire HNR₆R₇, ou encore d'un thiol R₆SH en présence d'une base, dans un solvant inerte tel que le tétrahydrofuranne ou l'acétone, à une température comprise de préférence entre 0 °C et 50 °C, et plus préférentiellement à température ambiante.

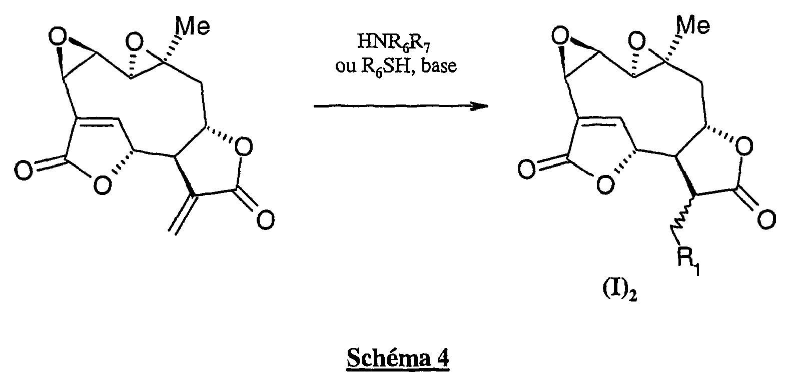

### Sels de composés de formule générale (I) :

Certains composés de l'invention peuvent être préparés sous la forme de sels pharmaceutiquement acceptables selon les méthodes usuelles. En ce qui concerne ces sels, l'homme du métier pourra utilement consulter l'article de Gould et coll., "Salt selection for basic drugs", *Int. J. Pharm*. (1986), **33,** 201-217.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

### EXEMPLES

*La nomenclature utilisée pour les exemples est en principe en conformité avec les normes IUPAC. Elle a été détenninée à l'aide du logiciel ACD*/*Name® (version 4.53) pour les exemples 1 à 36 et à l'aide du logiciel ACD*/*Name® (version 5.0) pour les exemples 37 à 52.*

*La numérotation indiquée sur la figure ci-dessous est employée pour les exemples 1 à 36 en ce qui concerne les positions des substituants -CH*_{*2*}*-R*_{*1*}*, R*_{*2*} *et R*₃ *sur les polycycles de sous-formules générales (**I**)*_{*1*} *et (**I**)*_{*2*} *:*

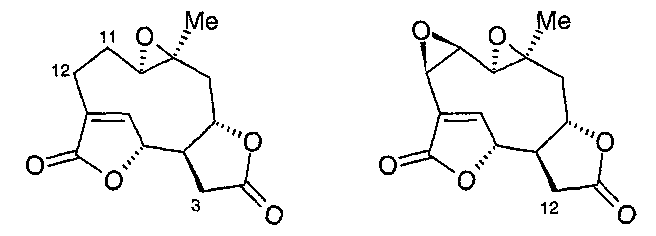

### Exemple 1 : 12-diisopropylaminométhyl-7-méthyl-3,6,10,15-tétraoxapentacyclo [12.2.1.0^{2,4}.0^{5,7}.0^{9,13}]heptadéc-1(17)-ène-11,16-dione :

A une solution de mikanolide (100 µmol ; 29 mg) dans de l'acétone (1 ml), on ajoute de la diisopropylamine (500 µmol ; 70 µl). La masse réactionnelle est agitée 30 min à température ambiante puis le solvant est éliminé par évaporation sous pression réduite. Le résidu est repris dans de l'éther, filtré et séché sous vide. On obtient 10 mg de produit sous la forme d'une poudre blanche.
RMN-¹H (DMSO): 0,90-1,30 (m, 15H); 1,85 (m, 2H); 2,15 (t, 2H) ; 3,15-3,50 (m, 4H); 3,95 (s, 1H); 4,75 (m, 1H) ; 5,50 (s, 1H) ; 6,00 (s, 1H) ; 6,25 (s, 1H) ; 7,60 (s, 1H).

### Exemple 2: 12-diméthylamino-3-diméthylaminométhyl-11-hydroxy-8-méthyl-5,9,15-trioxatétracyclo[11.2.1.0^{2,6}.0^{8,10}]hexadéc-13(16)-ène-4,14-dione :

A une solution de mikanolide (30 µmol ; 9 mg) dans de l'acétone (0,3 ml), on ajoute une solution de diméthylamine (160 µmol; 80 µl; 2M dans du THF). La masse réactionnelle est agitée 30 min à température ambiante puis concentrée sous pression réduite. Le résidu est repris dans de l'éther, filtré et séché sous vide. On obtient 6 mg du composé attendu sous la forme d'une poudre blanche.
RMN-¹H (DMSO): 1,11 (s, 3H); 1,94-1,97 (m, 2H); 2,20 (s, 6H); 2,47 (s, 6H); 2,67 (m, 2H) ; 2,85 (t, 1H); 3,07 (d, 1H) ; 3,15 (m, 1H) ; 3,52 (d, 1H) ; 3,63 (m, 1H); 4,62 (m, 1H) ; 5,36 (s, 1H) ; 5,47 (s, 1H) ; 8,00 (s, 1H).
RMN-¹³C (DMSO) : 20,68 ; 42,85 ; 43,37 ; 44, 71 ; 45,92 ; 49,95 ; 57,84 ; 58,24 ; 61,61 ; 62,97 ; 67,94 ; 77,09 ; 80,67 ; 131,46 ; 151,01 ; 172,03 ; 174,98.

### Exemple 3 : 12-benzyl(méthyl)amino-3-benzyl(méthyl)aminométhyl-11-hydroxy-8-méthyl-5,9,15-trioxatétracyclo[11.2.1.0^{2,6}.0^{8,10}]hexadéc-13(16)-ène-4,14-dione :

Ce composé est obtenu par une procédure similaire à celle décrite pour la synthèse du composé de l'exemple 2. Le produit attendu est obtenu sous la forme d'une poudre blanche.
RMN-¹H (DMSO): 1,12 (s, 3H) ; 1,96 (m, 2H) ; 2,10 (m, 1H) ; 2,15 (s, 3H); 2,47 (m, 2H) ; 2,83 (d, 2H) ; 2,89 (d, 1H) ; 3,22 (d, 1H), 3,26 (m, 1H) ; 3,58 (dd, 2H) ; 3,69 (m, 1H) ; 3,89 (d, 1H) ; 3,93 (s, 2H) ; 4,73 (m, 1H) ; 5,47 (d, 1H) ; 5,52 (s, 1H) ; 7,23-7,40 (m, 10H) ; 8,10 (s, 1H).
RMN-¹³C (DMSO) : 20,65 ; 39,08 ; 40,54 ; 42,09 ; 43,09 ; 43,52 ; 50,18 ; 56,57 ; 57,85 ; 60,17 ; 61,14 ; 62,21 ; 62,33 ; 68,37 ; 77,22 ; 81,01 ; 126,07 ; 128,30 ; 131,48 ; 138,88 ; 139,67 ; 150,35 ; 172,16 ; 175,18.

### Exemple 4 : 11-hydroxy-8-méthyl-12-morpholino-3-morpholinométhyl-5,9,15-trioxatétracyclo[11.2.1.0^{2,6}.0^{8,10}]hexadéc-13(16)-ène-4,14-dione :

Ce composé est obtenu par une procédure similaire à celle décrite pour la synthèse du composé de l'exemple 2. Le produit attendu est obtenu sous la forme d'une poudre blanche.
RMN-¹H (DMSO) : 1,13 (s, 3H) ; 1,85-2,10 (m, 2H); 2,36 (m, 2H) ; 2,40 (m, 2H); 2,74 (m, 4H); 2,88 (t, 1H); 2,95 (m, 2H); 3,10 (d, 1H); 3.24 (m, 1H); 3,50-3,70 (m, 10H); 4,64 (m, 1H); 5,49 (s, 1H); 5,50 (d, 1H); 8,01 (s, 1H).

### Exemple 5: 12-diméthylamino-11-hydroxy-3,8-diméthyl-5,9,15-trioxatétracyclo[11.2.1.0^{2,6}.0^{8,10}]hexadéc-13(16)-ène-4,14-dione :

A une solution de dihydromikanolide (100 µmol, 29 mg) dans de l'acétone (1 ml), on ajoute une solution de diméthylamine (500 µmol, 250 µl, 2*M* dans du THF). La masse réactionnelle est agitée 2 heures à température ambiante puis le solvant est éliminé par évaporation sous pression réduite. Le résidu est repris dans de l'éther, filtré et séché sous vide. On obtient 25 mg de produit sous la forme d'une poudre blanche.
RMN-¹H (DMSO): 1,10 (s, 3H); 1,25 (d, 3H), 1,90 (dd, 1H); 1,99 (t, 1H); 2,49 (s, 6H) ; 2,58 (t, 1H) ; 2,94 (m, 1H) ; 3,06 (d, 1H) ; 3,51 (m, 1H) ; 3,63 (m, 1H) ; 4,62 (m, 1H) ; 5,34 (s, 1H); 5,37 (d, 1H) ; 8,00 (s, 1H).

### Exemple 6 : maléate de 11-hydroxy-3,8-diméthyl-4,14-dioxo-5,9,15-trioxatétracyclo[11.2.1.0^{2,6}.0^{8,10}]hexadéc-13(16)-ène-12-yl(diméthyl)ammonium :

On ajoute une solution d'acide maléique (0,1 mmol ; 11,6 mg) dans de l'acétone (0,5 ml) à une solution du composé de l'exemple 5 (0,1 mmol ; 34 mg) dans de l'acétone (0,5 ml). Le précipité est filtré, lavé avec de l'acétone et séché sous pression réduite. On obtient 24 mg du produit attendu sous la forme d'une poudre blanche. Point de fusion : 178,5 °C.
RMN-¹H (DMSO): 1,09 (s, 3H); 1,28 (d, 3H); 1,94 (dd, 1H); 2,05 (m, 1H); 2,63 (t, 1H); 2,70-3,70 (m, 9H); 3,79 (t, 1H); 4,38 (s, 1H); 4,68 (m, 1H); 5,45 (s, 1H) ; 6,07 (s, 2H) ; 8,31 (s, 1H).

### Exemple 7 : fumarate de 11-hydroxy-3,8-diméthyl-4,14-dioxo-5,9,15-trioxatétracyclo[11.2.1.0^{2,6}.0^{8,10}]hexadéc-13(16)-ène-12-yl(diméthyl)ammonium :

On ajoute une solution d'acide fumarique (0,1 mmol ; 11,6 mg) dans de l'acétone (3 ml) à une solution du composé de l'exemple 5 (0,1 mmol ; 34 mg) dans de l'acétone (0,5 ml). Le précipité est filtré, lavé avec de l'acétone et séché sous pression réduite. On obtient 15 mg du produit attendu sous la forme d'une poudre blanche. Point de fusion : 159 °C.
RMN-¹H (DMSO): 1,11 (s, 3H); 1,25 (d, 3H); 1,92 (dd, 1H); 2,02 (m, 1H); 2,58 (t, 1H); 2,80-4,00 (m, 11H); 4,64 (m, 1H); 5,34 (s, 1H); 6,61 (s, 2H) ; 8,01 (s, 1H).

### Exemple 8 : méthanesulfonate de 11-hydroxy-3,8-diméthyl-4,14-dioxo-5,9,15-trioxatétracyclo[11.2.1.0^{2,6}.0^{8,10}]hexadéc-13(16)-ène-12-yl(diméthyl)ammonium :

On ajoute une solution d'acide méthanesulfonique (0,1 mmol ; 1 ml ; 0,1*N* dans l'acétone) à une solution du composé de l'exemple 5 (0,1 mmol ; 34 mg) dans de l'acétone (2 ml). Le précipité est filtré, lavé avec de l'acétone et séché sous pression réduite. On obtient 24 mg du produit attendu sous la forme d'une poudre blanche. Point de fusion : 220 °C.
RMN-¹H (DMSO) : 1,09 (s, 3H) ; 1,29 (d, 3H) ; 1,97 (dd, 1H) ; 2,07 (m, 1H) ; 2,30 (s, 3H); 2,65 (t, 1H); 2,80-3,15 (m, 7H); 3,28 (d, 1H); 3,85 (t, 1H); 4,66-4,72 (m, 2H) ; 5,49 (s, 1H) ; 6,94 (s, 1H) ; 8,44 (s, 1H) ; 10,04 (s, 1H).

### Exemple 9 : 11-hydroxy-3,8-diméthyl-12-(4-méthylpipéridino)-5,9,15-trioxatétracyclo[11.2.1.0^{2,6}.0^{8,10}]hexadéc-13(16)-ène-4,14-dione :

Ce composé est obtenu par une procédure similaire à celle décrite pour la synthèse du composé de l'exemple 5. Le produit attendu est obtenu sous la forme d'une poudre blanche. Point de fusion : 210 °C.
RMN-¹H (DMSO): 0,80-3,50 (m, 23H) ; 3,60-3,75 (m, 2H); 4,62 (m, 1H) ; 5,32 (s, 2H) ; 8,01 (s, 1H).

### Exemple 10 : 11-hydroxy-3,8-diméthyl-12-pyrrolidino-5,9,15-trioxatétracyclo[11.2.1.0^{2,6}.0^{8,10}]hexadéc-13(16)-ène-4,14-dione :

Ce composé est obtenu par une procédure similaire à celle décrite pour la synthèse du composé de l'exemple 5. Le produit attendu est obtenu sous la forme d'une poudre blanche.
RMN-¹H (DMSO): 1,12 (s, 3H); 1,25 (d, 3H); 1,69 (m, 4H); 1,91 (dd, 1H); 2,00 (m, 1H) ; 2,60 (t, 1H) ; 2,80 (m, 4H) ; 2,95 (m, 1H) ; 3,02 (d, 1H) ; 3,45 (s, 1H) ; 3,63 (m, 1H) ; 4,61 (m, 1H) ; 5,34 (s, 1H) ; 5,42 (d, 1H) ; 7,97 (s, 1H).

### Exemple 11 : 1-[11-hydroxy-3,8-diméthyl-4,14-dioxo-5,9,15-trioxatétracyclo[11.2.1.0^{2,6}.0^{8,10}]hexadéc-13(16)-ène-12-yl]-4-pipéridinecarboxylate d'éthyle :

Ce composé est obtenu par une procédure similaire à celle décrite pour la synthèse du composé de l'exemple 5. Le produit attendu est obtenu sous la forme d'une poudre blanche.
RMN-¹H (DMSO) : 1,00-4,00 (m, 25H); 4,04 (q, 2H) ; 4,64 (m, 1H) ; 5,35 (s, 1H) ; 5,48 (d, 1H) ; 8,07 (s,1H).

### Exemple 12: 12-(4-benzylpipéridino)-11-hydroxy-3,8-diméthyl-5,9,15-trioxatétracyclo[11.2.1.0^{2,6}.0^{8,10}]hexadéc-13(16)-ène-4,14-dione :

Ce composé est obtenu par une procédure similaire à celle décrite pour la synthèse du composé de l'exemple 5. Le produit attendu est obtenu sous la forme d'une poudre blanche.
RMN-¹H (DMSO): 1,00-1,80 (m, 12H); 1,85-2,10 (m, 2H); 2,35-4,00 (m, 6H); 4,63 (m, 1H); 5,33 (m, 2H); 7,00-7,20 (m, 5H); 8,03 (s, 1H).

### Exemple 13 : 11-hydroxy-3,8-diméthyl-12-pipéridino-5,9,15-trioxatétracyclo[11.2.1.0^{2,6}.0^{8,10}]hexadéc-13(16)-ène-4,14-dione

Ce composé est obtenu par une procédure similaire à celle décrite pour la synthèse du composé de l'exemple 5. Le produit attendu est obtenu sous la forme d'une poudre blanche.
RMN-¹H (DMSO) : 1,11 (s, 3H) ; 1,26 (d, 3H) ; 1,35-1,70 (m, 6H) ; 1,85-2,14 (m, 2H) ; 2,57-3,18 (m, 7H) ; 3,50-3,75 (m, 2H) ; 4,64 (m, 1H) ; 5,34 (m, 2H) ; 8,04 (s, 1H).

### Exemple 14 : 12-(1,4-dioxa-8-azaspiro[4.5]déc-8-yl)-11-hydroxy-3,8-diméthyl-5,9,15-trioxatétracyclo[11.2.1.0^{2,6}.0^{8,10}]hexadéc-13(16)-ène-4,14-dione :

Ce composé est obtenu par une procédure similaire à celle décrite pour la synthèse du composé de l'exemple 5. Le produit attendu est obtenu sous la forme d'une poudre blanche.
RMN-¹H (DMSO) : 1,11 (s, 3H) ; 1,26 (d, 3H) ; 1,40-1,80 (m, 6H) ; 1,85-2,05 (m, 2H) ; 2,58-4,00 (m, 17H) ; 4,67 (m, 1H) ; 5,37 (s, 1H) ; 5,44 (d, 1H) ; 8.08 (s, 1H).

### Exemple 15 : 11-hydroxy-3,8-diméthyl-12-morpholino-5,9,15-trioxatétracyclo[11.2.1.0^{2,6}.0^{8,10}]hexadéc-13(16)-ène-4,14-dione :

Ce composé est obtenu par une procédure similaire à celle décrite pour la synthèse du composé de l'exemple 5. Le produit attendu est obtenu sous la forme d'une poudre blanche.
RMN-¹H (DMSO) : 1,10 (s, 3H); 1,25 (d, 3H); 1,89 (dd, 1H); 2,01 (m, 1H); 2,61 (t, 1H); 2,75 (m, 2H); 3,95 (m, 3H); 3,08 (d, 1H); 3,55-3,75 (m, 5H); 4,63 (1H) ; 5,33 (s, 1H) ; 5,54 (d, 1H) ; 8,04 (s, 1H).

### Exemple 16 : 11-(tert-butyldiméthylsiloxy)-12-diméthylamino-3,8-diméthyl-5,9,15-trioxatétracyclo[11.2.1.0^{2,6}.0^{8,10}]hexadéc-13(16)-ène-4,14-dione :

A une solution du composé de l'exemple 5 (80 µmol; 27 mg) et d'imidazole (160 µmol; 11 mg) dans du DMF (0,5 ml), on ajoute du chlorure de terbutyldiméthylsilyle (80 µmol, 12 mg). On agite la solution obtenue pendant 20 heures puis on verse la masse réactionnelle dans l'eau. On extrait deux fois la phase aqueuse avec de l'acétate d'éthyle, on lave la phase organique à l'eau puis avec une solution de chlorure de sodium. La phase organique est séchée sur sulfate de magnésium filtrée puis évaporée. Le résidu est élué sur silice avec un mélange d'acétate d'isopropyle et de dichlorométhane (20/80). On obtient 20 mg de produit sous la forme d'une poudre blanche.
RMN-¹H (DMSO): 0,04 (s, 3H); 0,07 (s, 3H); 0,89 (s, 9H); 1,14 (s, 3H); 1,25 (d, 3H); 1,90 (dd, 1H); 1,99 (dd, 1H); 2,48 (s, 6H); 2,63 (t, 1H); 2,93-2,98 (m, 1H) ; 3,12 (d, 1H) ; 3,43 (m, 1H); 3,80 (m, 1H) ; 4,61 (m, 1H) ; 5,36 (s, 1H) ; 8,03 (s, 1H).

### Exemple 17: acétate de 3,8-diméthyl-12-(4-méthylpipéridino)-4,14-dioxo-5,9,15-trioxatétracyclo[11.2.1.0^{2,6}.0^{8,10}]hexadéc-13(16)-èn-11-yle :

A une solution du composé de l'exemple 9 (100 µmol ; 40 mg) dans de la pyridine (0,5 ml), on ajoute de l'anhydride acétique (150 µmol ; 15 µl). On agite la solution obtenue pendant 20 heures puis on verse la masse réactionnelle dans l'eau. On extrait deux fois la phase aqueuse avec de l'acétate d'éthyle et on lave la phase organique obtenue à l'eau puis par une solution de chlorure de sodium. La phase organique est séchée sur sulfate de magnésium, filtrée puis évaporée. Le résidu est élué sur silice avec un mélange d'acétate d'isopropyle et de dichlorométhane (20/80). On obtient 16 mg de produit sous la forme d'une poudre blanche.
RMN-¹H (DMSO): 0,90 (d, 3H); 1,11 (s, 3H); 1,26 (d, 3H); 1,35 (m, 1H) ; 1,60 (m, 2H), 1,94 (dd, 1H) ; 2,03 (d, 1H); 2,09 (s, 3H) ; 2,43 (t, 1H) ; 2,60 (t, 1H) ; 2,98 (d, 1H) ; 2,94-3,05 (m, 2H) ; 3,36-3,45 (m, 4H) ; 4,07 (d, 1H) ; 4,64 (dd, 1H) ; 4,70 (m, 1H) ; 5,38 (s, 1H) ; 8,12 (s, 1H).

### Exemple 18 : 3,8-diméthyl-12-(4-méthylpipéridino)-4,14-dioxo-11-phénylcarbonyloxy-5,9,15-trioxatétracyclo[11.2.1.0^{2,6}.0^{8,10}]hexadéc-13(16)-ène :

A une solution du composé de l'exemple 9 (100 µmol ; 40 mg) dans de la pyridine (0,5 ml), on ajoute du chlorure de benzoyle (400 µmol ; 46 µl). La masse réactionnelle est agitée pendant 2 heures puis traitée de la même manière que pour la préparation du composé de l'exemple 17. On obtient 25 mg de produit sous la forme d'une poudre blanche. Point de fusion : 234 °C.
RMN-¹H (DMSO): 0,73 (d, 3H); 1,18 (s, 3H); 1,25 (m, 1H); 1,27 (d, 3H); 1,45-1,60 (m, 2H); 2,00 (dd, 1H) ; 2,10 (m, 1H); 2,65 (t, 1H); 2,92-3,15 (m, 3H) ; 3,45 (m, 2H) ; 3,54 (d, 1H) ; 4,18 (d, 1H) ; 4,36 (t, 1H); 4,74 (m, 1H) ; 4,95 (t, 1H) ; 5,41 (s, 1H) ; 7,58 (t, 2H) ; 7,70 (t, 1H) ; 8,01 (d, 2H) ; 8,19 (s, 1H).

### Exemple 19 : 3,8-diméthyl-12-(4-méthylpipéridino)-4,14-dioxo-5,9,15-trioxatétracyclo[11.2.1.0^{2,6}.0^{8,10}]hexadéc-13(16)-èn-11-ylcarbonate d'éthyle:

A une solution du composé de l'exemple 9 (100 µmol; 40 mg) dans de la pyridine (0,5 ml), on ajoute du chloroformiate d'éthyle (300 µmol ; 28 µl). La masse réactionnelle est agitée pendant 2 heures puis traitée de la même manière que pour la préparation du composé de l'exemple 17. On obtient 20 mg de produit sous la forme d'une poudre blanche.
RMN-¹H (DMSO) : 0,88 (d, 3H) ; 1,10-1,40 (m, 12H) ; 1,59 (m, 2H) ; 2,90-2,10 (m, 2H) ; 2,35-2,50 (m, 2H) ; 2,58 (t, 1H) ; 2,80 (d, 1H) ; 2,95-3,07 (m, 2H) ; 3,40 (d, 1H) ; 4,11-4,25 (m, 3H) ; 4,43 (dd, 1H) ; 4,70 (m, 1H) ; 5,39 (s, 1H) ; 8,13 (s, 1H).

### Exemple 20 : 11-hydroxy-12-isobutylsulfanyl-3-isobutylsulfanylméthyl-8-méthyl-5,9,15-trioxatétracyclo[11.2.1.0^{2,6}.0^{8,10}]hexadéc-13(16)-ène-4,14-dione :

A une solution de mikanolide (100 µmol ; 30 mg) et de diméthylaminopyridine (10 µmol ; 1,2 mg) dans de l'acétone (1 ml), on ajoute du 2-méthyl-1-propanethiol (500 µmol ; 54 µl). La masse réactionnelle est agitée pendant deux heures à température ambiante puis le solvant est évaporé sous pression réduite. Le résidu est repris dans de l'éther, le précipité est filtré, lavé à l'éther et séché sous vide. On obtient 35 mg de produit sous la forme d'une poudre blanche.
RMN-¹H (DMSO) : 0,96 (m, 12H) ; 1,15 (s, 3H); 1,77 (m, 2H) ; 1,93 (d, 2H) ; 2,50 (m, 4H) ; 2,80-2,98 (m, 4H) ; 3,39 (m, 1H); 3,76 (m, 1H); 4,07 (d, 1H); 4,62 (q, 1H) ; 5,52 (s, 1H); 5,62 (s, 1H) ; 8,06 (s, 1H).

### Exemple 21 : 11-hydroxy-12-isobutylsulfanyl-3,8-diméthyl-5,9,15-trioxatétracyclo[11.2.1.0^{2,6}.0^{8,10}]hexadéc-13(16)-ène-4,14-dione :

A une solution de dihydromikanolide (100 µmol ; 30 mg) et de diméthylaminopyridine (10 µmol ; 1,2 mg) dans de l'acétone (1 ml), on ajoute du 2-méthyl-1-propanethiol (500 µmol ; 54 µl). La masse réactionnelle est agitée pendant deux heures à température ambiante puis le solvant est évaporé sous pression réduite. Le résidu est repris dans de l'éther puis le précipité formé est filtré, lavé à l'éther et séché sous vide. On obtient 25 mg de produit sous la forme d'une poudre blanche.
RMN-¹H (DMSO): 0,96 (t, 6H); 1,13 (s, 3H); 1,25 (d, 3H) ; 1,78 (m, 1H) ; 1,89 (dd, 1H) ; 2,00 (t, 1H) ; 2,48 (m, 2H) ; 2,62 (t, 1H) ; 2,82 (d, 1H), 2,98 (m, 1H) ; 3,78 (m, 1H) ; 4,07 (d, 1H) ; 4,57 (m, 1H) ; 5,40 (s, 1H) ; 5,61 (d, 1H) ; 8,06 (s, 1H).

### Exemple 22: benzoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle :

Ce composé est obtenu par une procédure similaire à celle décrite pour la synthèse du composé de l'exemple 18. Le produit attendu est obtenu sous la forme d'une poudre blanche.
RMN-¹H (DMSO) :1,21 (s, 3H) ; 1,28 (d, 3H); 1,98 (dd, 1H); 2,08 (t, 1H); 2,48 (s, 6H) ; 2,66 (t, 1H) ; 3,01 (m, 1H) ; 3,51 (d, 1H) ; 4,04 (d, 1H) ; 4,71 (m, 1H) ; 5,06 (dd, 1H) ; 4,43 (s, 1H) ; 7,58 (m, 2H) ; 7,70 (t, 1H) ; 8,01 (d, 2H) ; 8,20 (s, 1H).

### Exemple 23 : acétate de 12-(diméthylamino)-3,8-diméthyl-4,14-dioxo-5,9,15-trioxatétracyclo[11.2.1.0^{2,6}.0^{8,10}]hexadéc-13(16)-èn-11-yle :

Ce composé est obtenu par une procédure similaire à celle décrite pour la synthèse du composé de l'exemple 17. Le produit attendu est obtenu sous la forme d'une poudre blanche.
RMN-¹H (DMSO) :1,14 (s, 3H) ; 1,26 (d, 3H); 1,94 (dd, 1H) ; 2,05 (t, 1H) ; 2,08 (s, 3H) ; 2,45 (s, 6H) ; 2,62 (t, 1H) ; 3,97 (m, 1H) ; 3,32 (m, 1H) ; 3,90 (d, 1H) ; 4,68 (m, 1H) ; 4,78 (m, 1H) ; 5,39 (s, 1H) ; 8,12 (s, 1H).

### Exemple 24 : cyclohexanecarboxylate de 12-(diméthylamino)-3,8-diméthyl-4,14-dioxo-5,9,15-trioxatétracyclo[11.2.1.0^{2,6}.0^{8,10}]hexadéc-13(16)-èn-11-yle :

Ce composé est obtenu par une procédure similaire à celle décrite pour la synthèse du composé de l'exemple 18. Le produit attendu est obtenu sous la forme d'une poudre blanche.
RMN-¹H (DMSO) : 1,14 (s, 3H) ; 1,26 (d, 3H) ; 1,08-1,48 (m, 5H) ; 1,58 (m, 1H) ; 1,68 (m, 2H) ; 1,84 (t, 2H) ; 1,93 (dd, 1H) ; 2,03 (t, 1H) ; 2,37 (m, 1H) ; 2,44 (s, 6H) ; 2,61 (t, 1H) ; 2,98 (m, 1H) ; 3,32 (t, 1H) ; 3,87 (d, 1H) ; 4,66 (m, 1H) ; 4,77 (dd, 1H) ; 5,40 (s, 1H) ; 8,12 (s, 1H).

### Exemple 25 : 4-fluorobenzoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle :

Ce composé est obtenu par une procédure similaire à celle décrite pour la synthèse du composé de l'exemple 18. Le produit attendu est obtenu sous la forme d'une poudre blanche.
RMN-¹H (DMSO): 1,20 (s, 3H) ; 1,28 (d, 3H); 1,97 (dd, 1H) ; 2,08 (t, 1H); 2,46 (s, 6H) ; 2,65 (t, 1H) ; 3,00 (m, 1H) ; 3,50 (d, 1H) ; 4,04 (d, 1H) ; 4,71 (m, 1H) ; 5,04 (dd, 1H) ; 5,43 (s, 1H) ; 7,41 (t, 2H) ; 8,06 (dd, 2H) ; 8,20 (s, 1H).

### Exemple 26 : chlorhydrate de 11-{[tert-butyl(diméthyl)silyl]oxy}-12-(diméthylamino)-3,8-diméthyl-5,9,15-trioxatétracyclo[11.2.1.0^{2,6}.0^{8,10}]hexadéc-13(16)-ène-4,14-dione :

On ajoute une solution d'acide chlorhydrique (0,3 mmol ; 0,3 ml ; 1*N* dans l'ether) à une solution du composé de l'exemple 16 (0,22 mmol ; 100 mg) dans de l'acétone (2 ml). Le précipité est filtré, lavé avec un peu d'acétone, à l'éther et séché sous pression réduite. On obtient 70 mg du produit attendu sous la forme d'une poudre blanche.
RMN-¹H (DMSO) : 0,14 (d, 6H); 0,90 (s, 9H); 1,15 (s, 3H); 1,27 (d, 3H); 1,85 (dd, 1H); 2,05 (t, 1H); 2,72 (t, 1H); 2,90-3,25 (m, 7H); 3,72 (m, 1H); 3,93 (m, 1H); 4,76 (m, 2H) ; 5,46 (s, 1H) ; 8,70 (d, 1H) ; 11,64 (s, 1H).

### Exemple 27 : heptanoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle :

Ce composé est obtenu par une procédure similaire à celle décrite pour la synthèse du composé de l'exemple 18. Le produit attendu est obtenu sous la forme d'une poudre blanche.
RMN-¹H (DMSO) : 0,86 (t, 3H) ; 1,14 (s, 3H) ; 1,20-1,35 (m, 9H) ; 1,55 (m, 2H) ; 1,95 (dd, 1H) ; 2,02 (t, 1H) ; 2,35 (t, 2H) ; 2,44 (s, 6H) ; 2,61 (t, 1H) ; 2,96 (m, 1H) ; 3,33 (t, 1H) ; 3,89 (d, 1H) ; 4,68 (m, 1H) ; 4,77 (dd, 1H) ; 5,40 (s, 1H) ; 8,12 (s, 1H).

### Exemple 28: 4-(trifluorométhyl)benzoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle :

Ce composé est obtenu par une procédure similaire à celle décrite pour la synthèse du composé de l'exemple 18. Le produit attendu est obtenu sous la forme d'une poudre blanche.
RMN-¹H (DMSO) : 1,21 (s, 3H); 1,28 (d, 3H); 2,01 (dd, 1H); 2,06 (t, 1H); 2,48 (s, 6H) ; 2,66 (t, 1H) ; 3,00 (m, 1H) ; 3,55 (d, 1H) ; 4,09 (d, 1H) ; 4,73 (m, 1H) ; 5,04 (dd, 1H) ; 5,44 (s, 1H) ; 7,96 (d, 2H) ; 8,19 (d, 2H) ; 8,21 (s, 1H).

### Exemple 29 : 2-thiophènecarboxylate de 12-(diméthylamino)-3,8-diméthyl-4,14-dioxo-5,9,15-trioxatétracyclo[11.2.1.0^{2,6}.0^{8,10}]hexadéc-13(16)-èn-11-yle :

Ce composé est obtenu par une procédure similaire à celle décrite pour la synthèse du composé de l'exemple 18. Le produit attendu est obtenu sous la forme d'une poudre blanche.
RMN-¹H (DMSO) : 1,20 (s, 3H) ; 1,27 (d, 3H) ; 1,99 (m, 1H) ; 2,07 (t, 1H) ; 2,49 (s, 6H) ; 2,65 (t, 1H) ; 3,00 (m, 1H) ; 3,47 (d, 1H) ; 4,00 (d, 1H) ; 4,70 (m, 1H) ; 5,01 (dd, 1H) ; 5,43 (s, 1H) ; 7,26 (t, 1H) ; 7,87 (d, 1H) ; 8,01 (dd, 1H) ; 8,18 (s, 1H).

### Exemple 30 : 3,3-diméthylbutanoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle :

Ce composé est obtenu par une procédure similaire à celle décrite pour la synthèse du composé de l'exemple 18. Le produit attendu est obtenu sous la forme d'une poudre blanche.
RMN-¹H (DMSO) : 1,00 (s, 9H) ; 1,15 (s, 3H) ; 1,26 (d, 3H); 1,94 (dd, 1H) ; 2,03 (t, 1H) ; 2,24 (dd, 2H) ; 2,45 (s, 6H) ; 2,62 (t, 1H) ; 2,98 (m, 1H) ; 3,32 (d, 1H) ; 3,86 (d, 1H) ; 4,65 (m, 1H) ; 4,81 (dd, 1H) ; 5,40 (s, 1H) ; 8,12 (s, 1H).

### Exemple 31 : 1-benzothiophène-2-carboxylate de 12-(diméthylamino)-3,8-diméthyl-4,14-dioxo-5,9,15-trioxatétracyclo[11.2.1.0^{2,6}.0^{8,10}]hexadéc-13(16)-èn-11-yle :

Ce composé est obtenu par une procédure similaire à celle décrite pour la synthèse du composé de l'exemple 18. Le produit attendu est obtenu sous la forme d'une poudre blanche.
RMN-¹H (DMSO) : 1,22 (s, 3H) ; 1,28 (d, 3H) ; 2,01 (dd, 1H) ; 2,08 (m, 1H) ; 2,50 (s, 6H) ; 2,66 (t, 1H) ; 3,00 (m, 1H) ; 3,52 (d, 1H) ; 4,05 (d, 1H) ; 4,71 (m, 1H) ; 5,06 (dd, 1H) ; 5,44 (s, 1H) ; 7,50 (t, 1H) ; 7,56 (t, 1H) ; 8,09 (t, 2H) ; 8,21 (s, 1H) ; 8,27 (s, 1H).

### Exemple 32 : 2-furoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle :

Ce composé est obtenu par une procédure similaire à celle décrite pour la synthèse du composé de l'exemple 18. Le produit attendu est obtenu sous la forme d'une poudre blanche.
RMN-¹H (DMSO) : 1,19 (s, 3H) ; 1,27 (d, 3H) ; 1,97 (dd, 1H) ; 2,07 (t, 1H) ; 2,47 (s, 6H) ; 2,64 (t, 1H) ; 3,00 (m, 1H) ; 3,46 (d, 1H) ; 4,00 (d, 1H) ; 4,70 (m, 1H) ; 4,98 (dd, 1H) ; 5,43 (s, 1H) ; 6,72 (d, 1H) ; 7,36 (d, 1H) ; 8,03 (s, 1H) ; 8,18 (s, 1H).

### Exemple 33 : 5-nitro-2-furoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle :

Ce composé est obtenu par une procédure similaire à celle décrite pour la synthèse du composé de l'exemple 18. Le produit attendu est obtenu sous la forme d'une poudre blanche.
RMN-¹H (DMSO) : 1,19 (s, 3H) ; 1,28 (d, 3H) ; 1,98 (dd, 1H) ; 2,08 (t, 1H) ; 2,45 (s, 6H) ; 2,64 (t, 1H) ; 3,00 (m, 1H) ; 3,53 (d, 1H) ; 4,08 (d, 1H) ; 4,72 (m, 1H) ; 4,97 (dd, 1H) ; 5,44 (s, 1H) ; 7,65 (d, 1H) ; 7,80 (d, 1H) ; 8,21 (s, 1H).

### Exemple 34: chlorhydrate de 2-thiophènecarboxylate de 12-(diméthylamino)-3,8-diméthyl-4,14-dioxo-5,9,15-trioxatétracyclo[11.2.1.0^{2,6}.0^{8,10}]hexadéc-13(16)-èn-11-yle :

On ajoute une solution d'acide chlorhydrique (0,8 mmol ; 0,8 ml ; 1*N* dans l'éther) à une solution du composé de l'exemple 29 (0,44 mmol ; 196 mg) dans de l'acétone (4 ml). Le précipité est filtré, lavé avec un peu d'acétone, à l'éther et séché sous pression réduite. On obtient 180 mg du produit attendu sous la forme d'une poudre blanche.
RMN-¹H (DMSO) : 1,23 (s, 3H) ; 1,29 (d, 3H) ; 1,90 (dd, 1H) ; 2,13 (t, 1H) ; 2,76 (t, 1H) ; 2,85-3,25 (m, 7H); 3,95 (m, 1H) ; 4,78 (m, 1H); 5,02 (m, 1H) ; 5,38 (m, 1H) ; 5,51 (s, 1H) ; 7,29 (t, 1H) ; 7,97 (s, 1H) ; 8,08 (d, 1H) ; 8,86 (s, 1H) ; 12,12 (s, 1H).

### Exemple 35: 2-thiénylacétate de 12-(diméthylamino)-3,8-diméthyl-4,14-dioxo-5,9,15-trioxatétracyclo[11.2.1.0^{2,6}.0^{8,10}]hexadéc-13(16)-èn-11-yle :

Ce composé est obtenu par une procédure similaire à celle décrite pour la synthèse du composé de l'exemple 18. Le produit attendu est obtenu sous la forme d'une poudre blanche.
RMN-¹H (DMSO) : 1,14 (s, 3H) ; 1,26 (d, 3H); 1,94 (dd, 1H); 2,04 (m, 1H); 2,38 (s, 6H); 2,61 (t, 1H) ; 2,97 (m, 1H); 3,37 (d, 1H) ; 3,88 (d, 1H); 4,00 (d, 2H); 4,68 (m, 1H) ; 4,78 (dd, 1H) ; 5,39 (s, 1H) ; 6,98 (m, 2H) ; 7,43 (d, 1H) ; 8,12 (s, 1H).

### Exemple 36: phénoxyacétate de 12-(diméthylamino)-3,8-diméthyl-4,14-dioxo-5,9,15-trioxatétracyclo[11.2.1.0^{2,6}.0^{8,10}]hexadéc-13(16)-èn-11-yle :

Ce composé est obtenu par une procédure similaire à celle décrite pour la synthèse du composé de l'exemple 18. Le produit attendu est obtenu sous la forme d'une poudre blanche.
RMN-¹H (DMSO) : 1,23 (s, 3H); 1,35 (d, 3H); 2,04 (dd, 1H) ; 2,13 (t, 1H) ; 2,58 (s, 6H) ; 2,67 (t, 1H) ; 3,06 (m, 1H) ; 3,48 (d, 1H) ; 4,08 (d, 1H) ; 4,77 (m, 1H) ; 4,86 (m, 1H) ; 4,96 (dd, 2H) ; 5,50 (s, 1H) ; 7,05 (m, 3H) ; 7,38 (m, 2H) ; 8,23 (s, 1H).

### Exemple 37 : 4-tert-butylphénylcarbamate de 8-(diméthylamino)-3,10a-diméthyl-2,6-dioxodécahydro-4,7-méthènofuro[3,2-c]oxiréno[f]oxacycloundécin-9-yle :

A une solution du composé de l'exemple 5 (200 µmol ; 67 mg) dans du 1,2-dichloroéthane (10 ml), on ajoute du 4-terbutylphénylisocyanate (250 µmol ; 44 mg). On agite la solution obtenue pendant 20 heures à 60 °C avant d'évaporer le solvant sous pression réduite. Le résidu est élué sur silice en utilisant un mélange d'acétone et de dichlorométhane (20/80). Le résidu est repris dans de l'éther, filtré et séché sous vide. On obtient 36 mg de produit sous la forme d'une poudre blanche.
RMN-¹H (DMSO) : 1,18 (s, 3H) ; 1,25 (s, 9H) ; 1,27 (d, 3H) ; 1,95 (dd, 1H) ; 2,08 (t, 1H) ; 2,48 (s, 6H) ; 2,64 (t, 1H) ; 2,98 (m, 1H) ; 3,37 (m, 1H) ; 3,91 (d, 1H) ; 4,69 (m, 1H) ; 4,80 (dd, 11-1) ; 5,40 (s, 1H) ; 7,29 (d, 2H) ; 7,38 (d, 2H) ; 8,12 (s, 1H) ; 9,67 (s, 1H).

### Exemple 38 : thién-2-ylcarbamate de 8-(diméthylamino)-3,10a-diméthyl-2,6-dioxodécahydro-4,7-méthènofuro[3,2-c]oxiréno[f]oxacycloundécin-9-yle :

Ce composé est obtenu par une procédure similaire à celle décrite pour la synthèse du composé de l'exemple 18. Le produit attendu est obtenu sous la forme d'une poudre blanche.
RMN-¹H (DMSO): 1,18 (s, 3H); 1,26 (d, 3H); 1,95 (m, 1H) ; 2,06 (m, 1H) ; 2,48 (s, 6H) ; 2,64 (t, 1H) ; 2,97 (m, 1H) ; 3,36 (m, 1H) ; 3,90 (m, 1H) ; 4,68 (m, 1H) ; 4,79 (m, 1H) ; 5,40 (s, 1H) ; 6,61 (s, 1H) ; 6,82 (s, 1H) ; 6,94 (s, 1H) ; 8,13 (s, 1H) ; 10,78 (s, 1H).

### Exemple 39 : 2-méthoxyphénylcarbamate de 8-(diméthylamino)-3,10a-diméthyl-2,6-dioxodécahydro-4,7-méthènofuro[3,2-c]oxiréno[f]oxacycloundécin-9-yle :

Ce composé est obtenu par une procédure similaire à celle décrite pour la synthèse du composé de l'exemple 18. Le produit attendu est obtenu sous la forme d'une poudre blanche.
RMN-¹H (DMSO): 1,17 (s, 3H); 1,27 (d, 3H); 1,94 (dd, 1H) ; 2,05 (t, 1H); 2,48 (s, 6H) ; 2,63 (t, 1H) ; 2,98 (m, 1H) ; 3,37 (m, 1H) ; 3,80 (s, 3H) ; 3,87 (d, 1H) ; 4,68 (m, 1H) ; 4,80 (dd, 1H) ; 5,40 (s, 1H) ; 6,90 (t, 1H) ; 7,02 (d, 1H) ; 7,09 (t, 1H) ; 7,59 (d, 1H) ; 8,12 (s, 1H) ; 8,59 (s, 1H).

### Exemple 40 : 2(méthylthio)phénylcarbamate de 8-(diméthylamino)-3,10a-diméthyl-2,6-dioxodécahydro-4,7-méthènofuro[3,2-c]oxiréno[f]oxacycloundécin-9-yle :

Ce composé est obtenu par une procédure similaire à celle décrite pour la synthèse du composé de l'exemple 18. Le produit attendu est obtenu sous la forme d'une poudre blanche.
RMN-¹H (DMSO) : 1,17 (s, 3H) ; 1,27 (d, 3H) ; 1,95 (dd, 1H) ; 2,08 (t, 1H) ; 2,40 (s, 3H) ; 2,48 (s, 6H) ; 2,63 (t, 1H) ; 2,98 (m, 1H) ; 3,37 (s, 1H) ; 3,84 (d, 1H) ; 4,67 (m, 1H) ; 4,80 (dd, 1H); 5,39 (s, 1H) ; 7,15-7,25 (m, 3H); 7,32 (t, 1H) ; 8,10 (s, 1H) ; 8,90 (s, 1H).

### Exemple 41 : 2-éthoxyphénylcarbamate de 8-(diméthylamino)-3,10a-diméthyl-2,6-dioxodécahydro-4,7-méthènofuro[3,2-c]oxiréno[f]oxacycloundécin-9-yle :

Ce composé est obtenu par une procédure similaire à celle décrite pour la synthèse du composé de l'exemple 18. Le produit attendu est obtenu sous la forme d'une poudre blanche.
RMN-¹H (DMSO) : 1,17 (s, 3H); 1,27 (d, 3H); 1,35 (t, 3H); 1,95 (dd, 1H) ; 2,05 (t, 1H) ; 2,48 (s, 6H) ; 2,63 (t, 1H) ; 2,98 (m, 1H) ; 3,34 (m, 1H) ; 3,90 (d, 1H) ; 4,07 (q, 2H) ; 4,67 (m, 1H) ; 4,79 (dd, 1H) ; 5,40 (s, 1H) ; 6,90 (t, 1H) ; 7,01 (d, 1H) ; 7,07 (t, 1H) ; 7,58 (d, 1H) ; 8,12 (s, 1H) ; 8,46 (s, 1H).

### Exemple 42 : thién-3-ylcarbamate de 8-(diméthylamino)-3,10a-diméthyl-2,6-dioxodécahydro-4,7-méthènofuro[3,2-c]oxiréno[f]oxacycloundécin-9-yle :

Ce composé est obtenu par une procédure similaire à celle décrite pour la synthèse du composé de l'exemple 18. Le produit attendu est obtenu sous la forme d'une poudre blanche.
RMN-¹H (DMSO) : 1,18 (s, 3H) ; 1,27 (d, 3H) ; 1,95 (dd, 1H) ; 2,05 (m, 1H) ; 2,48 (s, 6H) ; 2,64 (t, 1H) ; 2,98 (m, 1H) ; 3,36 (m, 1H) ; 3,90 (d, 1H) ; 4,68 (m, 1H) ; 4,80 (dd, 1H) ; 5,40 (s, 1H) ; 7,04 (d, 1H) ; 7,22 (s, 1H) ; 7,43 (t, 1H) ; 8,12 (s, 1H) ; 10,08 (s, 1H).

### Exemple 43 : 1-benzothién-3-ylcarbamate de 8-(diméthylamino)-3,10a-diméthyl-2,6-dioxodécahydro-4,7-méthènofuro[3,2-c]oxiréno[f]oxacycloundécin-9-yle :

Ce composé est obtenu par une procédure similaire à celle décrite pour la synthèse du composé de l'exemple 18. Le produit attendu est obtenu sous la forme d'une poudre blanche.
RMN-¹H (DMSO) : 1,20 (s, 3H) ; 1,28 (d, 3H) ; 1,98 (dd, 1H) ; 2,07 (m, 1H) ; 2,48 (s, 6H) ; 2,65 (t, 1H) ; 2,98 (m, 1H) ; 3,40 (m, 1H) ; 3,97 (d, 1H) ; 4,70 (m, 1H) ; 4,82 (dd, 1H) ; 5,40 (s, 1H) ; 7,40 (m, 2H); 7,65 (s, 1H); 7,95 (d, 1H) ; 8,14 (m, 2H) ; 10,00 (s, 1H).

### Exemple 44 : N-(ter-butoxycarbonyl)glycinate de 8-(diméthylamino)-3,10a-diméthyl-2,6-dioxodécahydro-4,7-méthènofuro[3,2-c]oxiréno[f]oxacycloundécin-9-yle :

A une solution du composé de l'exemple 5 (200 µmol; 60 mg) dans du dichlorométhane (5 ml), on ajoute du chlorhydrate de 1-(3-diméthylaminopropyl)-3-éthylcarbodiimide (200 µmol ; 38 mg), la N-terbutyloxycarbonylglycine (200µmol ; 35mg), la triéthylamine (200µmol ; 28µl) et la diméthylaminopyridine (10µmol ; 3mg). La solution est agitée trois heures à température ambiante, versée sur une solution de NaHCO₃ puis extraite avec de l'acétate d'éthyle. La phase organique est lavée avec de l'eau puis avec solution saturée en chlorure de sodium avant d'être séchée sur MgSO₄ et filtrée. Le solvant est éliminé par distillation sous pression réduite. Le résidu est élué sur silice en utilisant un mélange d'acétone et de dichlorométhane (40/60). Le résidu est repris dans de l'éther, filtré et séché sous vide. On obtient 25 mg de produit sous la forme d'une poudre blanche.
RMN-¹H (DMSO): 1,15 (s, 3H) ; 1,26 (d, 3H) ; 1,39 (s, 9H) ; 1,92 (dd, 1H) ; 2,05 (t, 1H) ; 2,43 (s, 6H) ; 2,62 (t, 1H) ; 2,98 (m, 1H) ; 3,35 (t, 1H) ; 3,75 (t, 2H) ; 3,84 (d, 1H) ; 4,67 (m, 1H) ; 4,81 (dd, 1H) ; 5,40 (s, 1H) ; 7,25 (t, 1H) ; 8,13 (s, 1H).

### Exemple 45 : thién-3-ylacétate de 8-(diméthylamino)-3,10a-diméthyl-2,6-dioxodécahydro-4,7-méthènofuro[3,2-c]oxiréno[f]oxacycloundécin-9-yle :

Ce composé est obtenu par une procédure similaire à celle décrite pour la synthèse du composé de l'exemple 44. Le produit attendu est obtenu sous la forme d'une poudre blanche.
RMN-¹H (DMSO) : 1,14 (s, 3H); 1,26 (d, 3H); 1,94 (dd, 1H); 2,05 (m, 1H); 2,37 (s, 6H); 2,61 (t, 1H); 2,97 (m, 1H); 3,33 (t, 1H); 3,77 (s, 2H); 3,88 (d, 1H); 4,67 (m, 1H); 4,78 (dd, 1H); 5,39 (s, 1H); 7,04 (d, 1H); 7,36 (s, 1H); 7,50 (t, 1H); 8,11 (s, 1H).

### Exemple 46 : 1-benzothién-3-ylacétate de 8-(diméthylamino)-3,10a-diméthyl-2,6-dioxodécahydro-4,7-méthènofuro[3,2-c]oxiréno[f]oxacycloundécin-9-yle :

Ce composé est obtenu par une procédure similaire à celle décrite pour la synthèse du composé de l'exemple 44. Le produit attendu est obtenu sous la forme d'une poudre blanche.
RMN-¹H (DMSO): 1,13 (s, 3H); 1,25 (d, 3H) ; 1,94 (dd, 1H) ; 2,04 (t, 1H); 2,26 (s, 6H) ; 2,60 (t, 1H) ; 2,96 (m, 1H) ; 3,32 (m, 1H) ; 3,88 (d, 1H) ; 4,04 (s, 2H) ; 4,67 (m,1H) ; 4,74 (dd, 1H); 5,38 (s, 1H); 7,40 (m, 2H) ; 7,64 (s, 1H) ; 7,79 (d, 1H); 7,99 (d, 1H) ; 8,09 (s, 1H).

### Exemple 47 : thiophène-3-carboxylate de 8-(diméthylamino)-3,10a-diméthyl-2,6-dioxodécahydro-4,7-méthènofuro[3,2-c]oxiréno[f]oxacycloundécin-9-yle :

Ce composé est obtenu par une procédure similaire à celle décrite pour la synthèse du composé de l'exemple 44. Le produit attendu est obtenu sous la forme d'une poudre blanche.
RMN-¹H (DMSO): 1,19 (s, 3H) ; 1,27 (d, 3H); 1,97 (dd, 1H) ; 2,07 (t, 1H) ; 2,48 (s, 6H) ; 2,65 (t, 1H) ; 2,99 (m, 1H) ; 3,46 (d, 1H) ; 3,98 (s, 1H) ; 4,70 (m, 1H) ; 5,02 (m, 1H) ; 5,43 (s, 1H) ; 7,49 (s, 1H) ; 7,69 (s, 1H) ; 8,18 (s, 1H) ; 8,40 (s, 1H).

### Exemple 48: 5-phénylthién-2-ylcarbamate de 8-(diméthylamino)-3,10a-diméthyl-2,6-dioxodécahydro-4,7-méthènofuro[3,2-c]oxiréno[f]oxacycloundécin-9-yle :

Ce composé est obtenu par une procédure similaire à celle décrite pour la synthèse du composé de l'exemple 37. Le produit attendu est obtenu sous la forme d'une poudre blanche.
RMN-¹H (DMSO) : 1,20 (s, 3H); 1,27 (d, 3H) ; 1,97 (dd, 1H) ; 2,03 (t, 1H); 2,38 (s, 6H) ; 2,67 (m, 1H) ; 2,98 (m, 1H) ; 3,37 (d, 1H) ; 3,94 (m, 1H) ; 4,69 (m, 1H) ; 4,81 (m, 1H) ; 5,41 (s, 1H); 6,60 (s, 1H) ; 7,22 (m, 2H) ; 7,36 (t, 2H) ; 7,55 (d, 2H) ; 8,14 (s, 1H) ; 10,93 (s, 1H).

### Exemple 49 : 1-adamantylcarbamate de 8-(diméthylamino)-3,10a-diméthyl-2,6-dioxodécahydro-4,7-méthènofuro[3,2-c]oxiréno[f]oxacycloundécin-9-yle :

Ce composé est obtenu par une procédure similaire à celle décrite pour la synthèse du composé de l'exemple 37. Le produit attendu est obtenu sous la forme d'une poudre blanche.
RMN-¹H (DMSO): 1,14 (s, 3H); 1,26 (d, 3H); 1,60 (s, 6H); 1,80-1,94 (m, 6H); 1,94-2,09 (m, 4H); 2,47 (s, 6H); 2,62 (t, 1H); 2,96 (m, 1H); 3,21 (d, 1H); 3,38 (s, 1H) ; 3,76 (s, 1H) ; 4,64 (m, 2H); 5,37 (s, 1H); 6,96 (s, 1H) ; 8,05 (s, 1H).

### Exemple 50 : 2-naphtylcarbamate de 8-(diméthylamino)-3,10a-diméthyl-2,6-dioxodécahydro-4,7-méthènofuro[3,2-c]oxiréno[f]oxacycloundécin-9-yle :

Ce composé est obtenu par une procédure similaire à celle décrite pour la synthèse du composé de l'exemple 37. Le produit attendu est obtenu sous la forme d'une poudre blanche.
RMN-¹H (DMSO): 1,20 (s, 3H) ; 1,28 (d, 3H); 1,96 (dd, 1H); 2,07 (t, 1H) ; 2,48 (s, 6H) ; 2,66 (t, 1H) ; 3,01 (m, 1H) ; 3,37 (m, 1H) ; 3,95 (d, 1H) ; 4,70 (m, 1H) ; 4,87 (dd, 1H) ; 5,42 (s, 1H) ; 7,38 (t, 1H) ; 7,46 (t, 1H) ; 7,55 (d, 1H) ; 7,82 (m, 3H) ; 8,10 (s, 1H) ; 8,14 (s, 1H) ; 10,01 (s, 1H).

### Exemple 51: 2-tert-butyl-6-méthylphénylcarbamate de 8-(diméthylamino)-3,10a-diméthyl-2,6-dioxodécahydro-4,7-méthènofuro[3,2-c]oxiréno[f]oxacycloundécin-9-yle :

Ce composé est obtenu par une procédure similaire à celle décrite pour la synthèse du composé de l'exemple 37. Le produit attendu est obtenu sous la forme d'une poudre blanche.
RMN-¹H (DMSO) : 1,14 (s, 3H) ; 1,20-1,42 (m, 12H) ; 1,92 (dd, 1H) ; 2,05 (m, 1H) ; 2,25 (s, 3H) ; 2,52 (s, 6H) ; 2,62 (m, 1H) ; 2,95 (m, 1H) ; 3,36 (m, 1H) ; 3,88 (m, 1H) ; 4,80-4,95 (m, 2H) ; 5,40 (s, 1H); 7,13 (m, 2H) ; 7,22 (s, 1H) ; 8,13 (s, 1H); 8,69 (s, 1H).

### Exemple 52 : 2,5-diméthoxyphénylcarbamate de 8-(diméthylamino)-3,10a-diméthyl-2,6-dioxodécahydro-4,7-méthèno-furo[3,2-c]oxiréno[f]oxacycloundécin-9-yle :

Ce composé est obtenu par une procédure similaire à celle décrite pour la synthèse du composé de l'exemple 37. Le produit attendu est obtenu sous la forme d'une poudre blanche.
RMN-¹H (DMSO) : 1,17 (s, 3H) ; 1,27 (d, 3H) ; 1,94 (dd, 1H) ; 2,06 (m, 1H) ; 2,48 (s, 6H) ; 2,64 (t, 1H) ; 2,98 (m, 1H) ; 3,33 (m, 1H) ; 3,69 (s, 3H) ; 3,76 (s, 3H) ; 3,89 (d, 1H) ; 4,68 (m, 1H) ; 4,80 (dd, 1H) ; 5,40 (s, 1H) ; 6,63 (d, 1H) ; 6,94 (d, 1H) ; 7,32 (s, 1H) ; 8,12 (s, 1H) ; 8,58 (s, 1H).

### ÉTUDE PHARMACOLOGIQUE DES COMPOSES DE L'INVENTION

L'utilité des composés de l'invention peut être démontrée notamment par l'effet d'un traitement par ces mêmes composés sur :
- l'incorporation de cytosine marquée au ³²P dans un fragment d'ADN en présence d'ADN polymérase de *E. Coli* (système acellulaire) ;
- l'incorporation de thymidine tritiée dans l'ADN des cellules tumorales HT29 en division sur une période de 3 heures (système cellulaire) ; et
- la prolifération de deux lignées de cellules humaines Mia-Paca2 et DU145.

### 1) Procédures

### Lignées cellulaires

Les lignées cellulaires DU-145 (cellules humaines de cancer de la prostate), HT29 (cancer du colon) et Mia-PaCa2 (cellules humaines de cancer du pancréas) ont été acquises auprès de American Tissue Culture Collection (Rockville, Maryland, USA).

### Incorporation de déoxycytidine 5'-[alpha³²P]-triphosphate dans un ADN en présence d'ADN polymérase dans un système acellulaire

Le marquage de l'ADN se fait sur un fragment d'ADN qui incorpore des nucléotides grâce à l'activité de l'enzyme ADN polymérase. Parmi ces nucléotides, le dCTP est marqué par du phosphore radioactif (³²P).
L'ADN plasmidique (pc DNA 3, invitrogen, Netherlands) est dilué à une concentration de 2 ng dans 45 µl de solution TE (10 mM Tris-HCl, pH 8, 1mM EDTA) et dénaturé par chauffage à 100 °C pendant 10 min avant d'être replacé directement dans la glace. L'ADN dénaturé est placé dans le tube qui contient la solution tampon de dATP, dGTP, dTTP, l'enzyme ADN polymérase Klenow et les amorces aléatoires (Rediprime II random prime labelling system, RPN 1633, RPN 1634, Amersham pharmacia biotech). 2 µl de Redivue deoxycytidine 5'-[alpha³²P]-triphosphate (250 *µ*Ci d'activité spécifique Amersham, Orsay, France) sont ajoutés pour démarrer la réaction. La réaction est faite en présence ou non du composé à tester durant 10 min à 37°. La réaction est arrêtée par ajout de 5 µl de EDTA (0.2M). L'ADN marqué est récupéré, dans 200 µl d'isopropanol après 10 min de centrifugation à 12 000 rpm puis lavé dans l'éthanol à 70%. Après séchage complet, l'ADN est solubilisé dans 50 µl de TE. Dix µL sont comptés dans 5 ml de scintillant (Instagel plus et compteur à scintillation Packard, Rungis, France).

Les résultats sont exprimés en pourcentage d'inhibition de l'incorporation du nucléotide marqué dans l'échantillon par rapport au témoin : 100 - [(DPM de l'échantillon traité/DPM témoin) x 100].

### Incorporation de thymidine marquée au tritium dans l'ADN de cellules en phase exponentielle de croissance

Des cellules HT29 sont ensemencées dans des plaques 96 puits (culturPlate-96, Packard, Rungis, France) (4000 cellules par puits) avec le milieu (DME, Gibco BRL, Cergy-Pontoise, France) complémenté par 10% de sérum de veau foetal, Gibco BRL, Cergy-Pontoise, France). Au jour 3, le milieu est enlevé et remplacé par du milieu contenant la thymidine tritiée (5'-thymidine TRK.328, 1 mCi, 0,6 µCi / puits, Amersham, Orsay, France) avec ou sans le composé. Les traitements s'effectuent pendant 3 heures. Le milieu est alors enlevé et les cellules sont lavées 2 fois avec 200µl de PBS (Gibco BRL, Cergy-Pontoise, France). La solution de lyse (SDS 1,25%, EDTA 5 mM) est ajoutée pendant 10 min à température ambiante, puis 75 µl de liquide scintillant (microscint 40, Packard, Rungis, France) sont ajoutés. La lecture des plaques se fait sur Topcount (Packard, Rungis, France). Ces tests sont effectués en double et les résultats sont exprimés par le rapport de l'incorporation du nucléotide marqué dans l'échantillon traité par le composé sur l'incorporation du nucléotide marqué dans l'échantillon témoin (DPM de l'échantillon/ DPM témoin).

### Mesure de prolifération cellulaire

Les cellules placées dans 80 µl de milieu Eagle modifié de Dulbecco (Gibco-BRL, Cergy-Pontoise, France) complété avec 10% de sérum foetal de veau inactivé par chauffage (Gibco-BRL, Cergy-Pontoise, France), 50000 unités/l de pénicilline et 50 mg/l streptomycine (Gibco-BRL, Cergy-Pontoise, France), et 2 mM de glutamine (Gibco-BRL, Cergy-Pontoise, France) ont été ensemencées sur une plaque de 96 puits au jour 0. Les cellules ont été traitées au jour 1 pendant 96 heures avec des concentrations croissantes de chacun des composés à tester. A la fin de cette période, la quantification de la prolifération cellulaire est évaluée par test colorimétrique en se basant sur le clivage du sel de tétrazolium WST1 par les déhydrogénases mitochondriales dans les cellules viables conduisant à la formation de formazan (Boehringer Mannheim, Meylan, France). Ces tests sont effectués en double avec 8 déterminations par concentration testée. Les produits sont solubilisés dans le diméthylsulfoxide (DMSO) à 10⁻² M et utilisés en culture avec 0,5% DMSO en final.

### 2) Résultats

A une concentration de 100 µg/ml, le composé de l'exemple 16 inhibe, tout comme le dihydromikanolide, l'activité de l'ADN polymérase dans un système acellulaire (tableau I).

**Tableau I**

| % d'inhibition de l'incorporation | |
|---|---|
| Dihydromikanolide | 77 ± 11 |
| Exemple 16 | 70 ± 8 |

Par ailleurs, le composé de l'exemple 16, comme le dihydromikanolide, inhibe l'incorporation de la thymidine tritiée dans l'ADN des cellules humaines HT29 (tableau II).

**Tableau II**

| (dpm échantillon /dpm témoin) | | | |
|---|---|---|---|
| Concentration µg/ml | 100 | 50 | 25 |
| Dihydromikanolide | 0,14 | 0,22 | 0,98 |
| Exemple 16 | 0,52 | 0,58 | 0,71 |

Enfin, les composés des exemples 1 à 3, 9, 16, 18, 22, 24 à 32, 37 et 39 à 43 inhibent la prolifération des lignées cellulaires DU-145 avec une concentration inhibitrice CI₅₀ inférieure ou égale à 30 µM, tandis que les composés des exemples 1 à 10, 12, 16 à 20, 22, 24 à 37 et 39 à 44 inhibent la prolifération des lignées cellulaires Mia-Paca2 avec une concentration inhibitrice CI₅₀ inférieure ou égale à 30 µM.

## Revendications

1. Composé de formule générale **(I)** 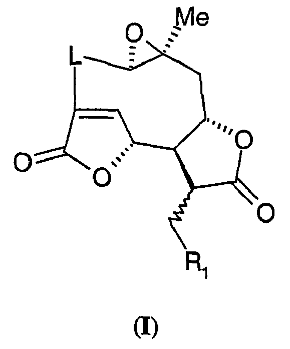
correspondant aux sous-formules générales **(I)**_{**1**} et **(I)**_{**2**} 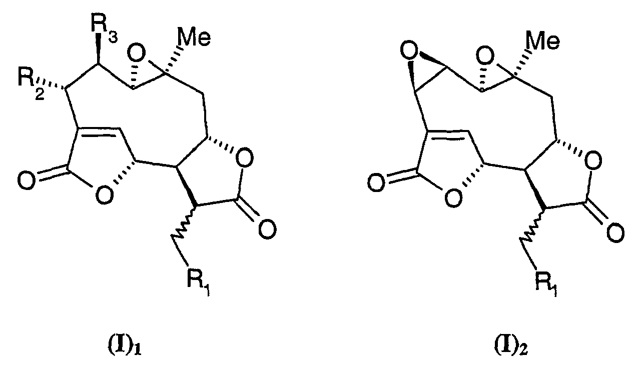
dans lesquelles
R₁ représente un atome d'hydrogène ou un radical SR₄ ou NR₄R₅ ;
R₂ représente SR₆ ou NR₆R₇ ;
R₃ représente OH, O(CO)R₁₄, OSiR₁₅R₁₆R₁₇, O(CO)OR₁₈ ou O(CO)NHR₁₈ ;
R₄ et R₆ représentent, indépendamment, un radical alkyle, un radical cycloalkyle, cycloalkylalkyle, hydroxyalkyle ou encore l'un des radicaux aryle ou aralkyle éventuellement substitués sur leur groupe aryle par un ou des radicaux choisis parmi les radicaux alkyle, hydroxy ou alkoxy,
R₅ et R₇ représentent, indépendamment, un atome d'hydrogène, un radical alkyle, un radical cycloalkyle, cycloalkylalkyle, hydroxyalkyle ou encore l'un des radicaux aryle ou aralkyle éventuellement substitués sur leur groupe aryle par un ou des radicaux choisis parmi les radicaux alkyle, hydroxy ou alkoxy,
R₄ et R₅ pouvant former ensemble avec l'atome d'azote qui les porte un hétérocycle de 5 à 7 chaînons, les chaînons complémentaires étant choisis parmi les radicaux -CR₈R₉-, -NR₁₀-, -O- et -S-, étant entendu toutefois qu'il ne peut se trouver qu'un seul chaînon choisi parmi -O- ou -S- dans ledit hétérocycle,
et R₆ et R₇ pouvant former ensemble avec l'atome d'azote qui les porte un hétérocycle de 5 à 7 chaînons, les chaînons complémentaires étant choisis parmi les radicaux -CR₁₁R₁₂-, -NR₁₃-, -O- et -S-, étant entendu toutefois qu'il ne peut se trouver qu'un seul chaînon choisi parmi -O- ou -S- dans ledit hétérocycle,
R₈, R₁₀, R₁₁ et R₁₃ représentent, indépendamment à chaque fois qu'ils interviennent, un atome d'hydrogène ou un radical alkyle, alkoxycarbonyle ou aralkyle,
R₉ et R₁₂ représentant, indépendamment à chaque fois qu'ils interviennent, un atome d'hydrogène ou un radical alkyl, ou chacun de R₉ et R₁₂ pouvant former avec respectivement R₈ et R₁₁ un radical -O-(CH₂)₂-O- attaché de part et d'autre à l'atome de carbone qui le porte, un tel radical n'étant présent toutefois qu'une fois au maximum par radical NR₄R₅ ou NR₆R₇,
R₁₄ représente un radical alkyle, cycloalkyle ou adamantyle ou l'un des radicaux aryle, hétéroaryle, aralkyle ou hétéroaralkyle éventuellement substitués sur leur groupe aryle ou hétéroaryle par un ou des radicaux choisis parmi un atome halogène et les radicaux alkyle, haloalkyle, nitro, hydroxy, alkoxy, alkylthio ou phényle,
ou encore R₁₄ est tel que R₁₄-COOH représente un acide aminé naturel ou l'énantiomère optique d'un tel acide aminé ;
R₁₅, R₁₆ et R₁₇ représentent, indépendamment, un radical alkyle ou un radical phényle ;
R₁₈ représente un radical alkyle, cycloalkyle ou adamantyle ou l'un des radicaux aryle, hétéroaryle, aralkyle ou hétéroaralkyle éventuellement substitués sur leur groupe aryle ou hétéroaryle par un ou des radicaux choisis parmi un atome halogène et les radicaux alkyle, haloalkyle, nitro, hydroxy, alkoxy, alkylthio ou phényle ;
étant entendu toutefois que lorsque les composés répondent à la sous-formule générale **(I)**_{**2**}, alors R₁ ne représente pas un atome d'hydrogène ;
ou sel d'un composé de formule générale **(I)**.

2. Composé selon la revendication 1, **caractérisé en ce qu'**il répond à la sous-formule générale **(**I**)**_{**1**} ou est un sel d'un tel composé.

3. Composé selon la revendication 2, **caractérisé en ce que** :
• R₁ représente un atome d'hydrogène ou un radical NR₄R₅ ;
• R₂ représentant un radical NR₆R₇ ;
• R₃ représente OH ou un radical O(CO)R₁₄, OSiR₁₅R₁₆R₁₇ ou O(CO)NHR₁₈.

4. Composé selon la revendication 1, **caractérisé en ce qu'**il répond à la sous-formule générale **(**I**)**_{**2**}.

5. Composé selon la revendication 1, **caractérisé en ce qu'**il s'agit d'un des composés suivants :
- 12-diisopropylaminométhyl-7-méthyl-3,6,10,15-tétraoxapentacyclo [12.2.1.0^{2,4}.0^{5,7}.0^{9,13}]heptadéc-1(17)-ène-11,16-dione ;
- 12-diméthylamino-3-diméthylaminométhyl-11-hydroxy-8-méthyl-5,9,15-trioxatétracyclo[11.2.1.0^{2,6}.0^{8,10}]hexadéc-13(16)-ène-4,14-dione ;
- 12-benzyl(méthyl)amino-3-benzyl(méthyl)aminométhyl-11-hydroxy-8-méthyl-5,9,15-trioxatétracyclo[11.2.1.0^{2,6}.0^{8,10}]hexadéc-13(16)-ène-4,14-dione ;
- 11-hydroxy-8-méthyl-12-morpholino-3-morpholinométhyl-5,9,15-trioxatétracyclo[11.2.1.0^{2,6}.0^{8,10}]hexadéc-13(16)-ène-4,14-dione ;
- 12-diméthylamino-11-hydroxy-3,8-diméthyl-5,9,15-trioxatétracyclo[11.2.1.0^{2,6}.0^{8,10}]hexadéc-13(16)-ène-4,14-dione ;
- 11-hydroxy-3,8-diméthyl-12-(4-méthylpipéridino)-5,9,15-trioxatétracyclo[11.2.1.0^{2,6}.0^{8,10}]hexadéc-13(16)-ène-4,14-dione ;
- 11-hydroxy-3,8-diméthyl-12-pyrrolidino-5,9,15-trioxatétracyclo[11.2.1.0^{2,6}.0^{8,10}]hexadéc-13(16)-ène-4,14-dione ;
- 1-[11-hydroxy-3,8-diméthyl-4,14-dioxo-5,9,15-trioxatétracyclo[11.2.1.0^{2,6}.0^{8,10}]hexadéc-13(16)-èn-12-yl]-4-pipéridinecarboxylate d'éthyle ;
- 12-(4-benzylpipéridino)-11-hydroxy-3,8-diméthyl-5,9,15-trioxatétracyclo[11.2.1.0^{2,6}.0^{8,10}]hexadéc-13(16)-ène-4,14-dione ;
- 11-hydroxy-3,8-diméthyl-12-pipéridino-5,9,15-trioxatétracyclo[11.2.1.0^{2,6}.0^{8,10}]hexadéc-13(16)-ène-4,14-dione ;
- 12-(1,4-dioxa-8-azaspiro[4.5]déc-8-yl)-11-hydroxy-3,8-diméthyl-5,9,15-trioxatétracyclo[11.2.1.0^{2,6}.0^{8,10}]hexadéc-13(16)-ène-4,14-dione ;
- 11-hydroxy-3,8-diméthyl-12-morpholino-5,9,15-trioxatétracyclo[11.2.1.0^{2,6}.0^{8,10}]hexadéc-13(16)-ène-4,14-dione ;
- 11-(*tert*-butyldiméthylsiloxy)-12-diméthylamino-3,8-diméthyl-5,9,15-trioxatétracyclo[11.2.1.0^{2,6}.0^{8,10}]hexadéc-13(16)-ène-4,14-dione ;
- acétate de 3,8-diméthyl-12-(4-méthylpipéridino)-4,14-dioxo-5,9,15-trioxatétracyclo[11.2.1.0^{2,6}.0^{8,10}]hexadéc-13(16)-èn-11-yle ;
- 3,8-diméthyl-12-(4-méthylpipéridino)-4,14-dioxo-11-phénylcarbonyloxy-5,9,15-trioxatétracyclo[11.2.1.0^{2,6}.0^{8,10}]hexadéc-13(16)-ène ;
- 3,8-diméthyl-12-(4-méthylpipéridino)-4,14-dioxo-5,9,15-trioxatétracyclo[11.2.1.0^{2,6}.0^{8,10}]hexadéc-13(16)-èn-11-ylcarbonate d'éthyle ;
- 11-hydroxy-12-isobutylsulfanyl-3-isobutylsulfanylméthyl-8-méthyl-5,9,15-trioxatétracyclo[11.2.1.0^{2,6}.0^{8,10}]hexadéc-13(16)-ène-4,14-dione ;
- 11-hydroxy-12-isobutylsulfanyl-3,8-diméthyl-5,9,15-trioxatétracyclo[11.2.1.0^{2,6}.0^{8,10}]hexadéc-13(16)-ène-4,14-dione ;
- benzoate de 12-(diméthylamino)-3,8-diméthyl-4,14-dioxo-5,9,15-trioxatétracyclo[11.2.1.02,6.08,10]hexadéc-13(16)-èn-11-yle ;
- acétate de 12-(diméthylamino)-3,8-diméthyl-4,14-dioxo-5,9,15-trioxatétracyclo[11.2.1.02,6.08,10]hexadéc-13(16)-èn-11-yle ;
- cyclohexanecarboxylate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 4-fluorobenzoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- heptanoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 4-(trifluorométhyl)benzoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 2-thiophènecarboxylate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 3,3-diméthylbutanoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 1-benzothiophène-2-carboxylate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 2-furoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 5-nitro-2-furoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 2-thiénylacétate de 12-(diméthylamino)-3,8-diméthyl-4,14-dioxo-5,9,15-trioxatétracyclo[11.2.1.02,6.08,10]hexadéc-13(16)-èn-11-yle ;
- phénoxyacétate de 12-(diméthylamino)-3,8-diméthyl-4,14-dioxo-5,9,15-trioxatétracyclo[11.2.1.02,6.08,10]hexadéc-13(16)-èn-11-yle ;
- 4-*tert*-butylphénylcarbamate de 8-(diméthylamino)-3,10a-diméthyl-2,6-dioxodécahydro-4,7-méthènofuro[3,2-*c*]oxiréno[*f*]oxacycloundécin-9-yle ;
- thién-2-ylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 2-méthoxyphénylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 2(méthylthio)phénylcarbamate de 8-(diméthylamino)-3,10a-diméthyl-2,6-dioxodécahydro-4,7-méthèno-furo[3,2-*c*]oxiréno[*f*]oxacycloundécin-9-yle ;
- 2-éthoxyphénylcarbamate de 8-(diméthylamino)-3,10a-diméthyl-2,6-dioxodécahydro-4,7-méthènofuro[3,2-*c*]oxiréno[*f*]oxacycloundécin-9-yle ;
- thién-3-ylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 1-benzothién-3-ylcarbamate de 8-(diméthylamino)-3,10a-diméthyl-2,6-dioxodécahydro-4,7-méthènofuro[3,2-*c*]oxiréno[*f*]oxacycloundécin-9-yle ;
- *N*-(*ter*-butoxycarbonyl)glycinate de 8-(diméthylamino)-3,10a-diméthyl-2,6-dioxodécahydro-4,7-méthèno-furo[3,2-*c*]oxiréno[*f*]oxacycloundécin-9-yle
- thién-3-ylacétate de 8-(diméthylamino)-3,10a-diméthyl-2,6-dioxodécahydro-4,7-méthènofuro[3,2-*c*]oxiréno[*f*]oxacycloundécin-9-yle ;
- 1-benzothién-3-ylacétate de 8-(diméthylamino)-3,10a-diméthyl-2,6-dioxodécahydro-4,7-méthènofuro[3,2-*c*]oxiréno[*f*]oxacycloundécin-9-yle ;
- thiophène-3-carboxylate de 8-(diméthylamino)-3,10a-diméthyl-2,6-dioxodécahydro-4,7-méthènofuro[3,2-*c*]oxiréno[*f*]oxacycloundécin-9-yle ;
- 5-phénylthién-2-ylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 1-adamantylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 2-naphtylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 2-*tert*-butyl-6-méthylphénylcarbamate de 8-(diméthylamino)-3,10a-diméthyl-2,6-dioxodécahydro-4,7-méthèno-furo[3,2-*c*]oxiréno[*f*]oxacycloundécin-9-yle ;
- 2,5-diméthoxyphénylcarbamate de 8-(diméthylamino)-3,10a-diméthyl-2,6-dioxodécahydro-4,7-méthèno-furo[3,2-*c*]oxiréno[*f*]oxacycloundécin-9-yle ;
ou d'un sel d'un de ces derniers.

6. A titre de médicament, un composé de formule générale **(I)** telle que définie dans la revendication 1 ou un sel pharmaceutiquement acceptable de ce dernier.

7. Médicament selon la revendication 6, **caractérisé en ce qu'**il s'agit de l'un des composés suivants :
- 12-diisopropylaminométhyl-7-méthyl-3,6,10,15-tétraoxapentacyclo [12.2.1.0^{2,4}.0^{5,7}.0^{9,13}]heptadéc-1(17)-ène-11,16-dione ;
- 12-diméthylamino-3-diméthylaminométhyl-11-hydroxy-8-méthyl-5,9,15-trioxatétracyclo[11.2.1.0^{2,6}.0^{8,10}]hexadéc-13(16)-ène-4,14-dione ;
- 12-benzyl(méthyl)amino-3-benzyl(méthyl)aminométhyl-11-hydroxy-8-méthyl-5,9,15-trioxatétracyclo[11.2.1.0^{2,6}.0^{8,10}]hexadéc-13(16)-ène-4,14-dione ;
- 11-hydroxy-8-méthyl-12-morpholino-3-morpholinométhyl-5,9,15-trioxatétracyclo[11.2.1.0^{2,6}.0^{8,10}]hexadéc-13(16)-ène-4,14-dione ;
- 12-diméthylamino-11-hydroxy-3,8-diméthyl-5,9,15-trioxatétracyclo[11.2.1.0^{2,6}.0^{8,10}]hexadéc-13(16)-ène-4,14-dione ;
- 11-hydroxy-3,8-diméthyl-12-(4-méthylpipéridino)-5,9,15-trioxatétracyclo[11.2.1.0^{2,6}.0^{8,10}]hexadéc-13(16)-ène-4,14-dione ;
- 11-hydroxy-3,8-diméthyl-12-pyrrolidino-5,9,15-trioxatétracyclo[11.2.1.0^{2,6}.0^{8,10}]hexadéc-13(16)-ène-4,14-dione ;
- 1-[11-hydroxy-3,8-diméthyl-4,14-dioxo-5,9,15-trioxatétracyclo[11.2.1.0^{2,6}.0^{8,10}]hexadéc-13(16)-èn-12-yl]-4-pipéridinecarboxylate d'éthyle ;
- 12-(4-benzylpipéridino)-11-hydroxy-3,8-diméthyl-5,9,15-trioxatétracyclo[11.2.1.0^{2,6}.0^{8,10}]hexadéc-13(16)-ène-4,14-dione ;
- 11-hydroxy-3,8-diméthyl-12-pipéridino-5,9,15-trioxatétracyclo[11.2.1.0^{2,6}.0^{8,10}]hexadéc-13(16)-ène-4,14-dione ;
- 12-(1,4-dioxa-8-azaspiro[4.5]déc-8-yl)-11-hydroxy-3,8-diméthyl-5,9,15-trioxatétracyclo[11.2.1.0^{2,6}.0^{8,10}]hexadéc-13(16)-ène-4,14-dione ;
- 11-hydroxy-3,8-diméthyl-12-morpholino-5,9,15-trioxatétracyclo[11.2.1.0^{2,6}.0^{8,10}]hexadéc-13(16)-ène-4,14-dione ;
- 11-(*tert*-butyldiméthylsiloxy)-12-diméthylamino-3,8-diméthyl-5,9,15-trioxatétracyclo[11.2.1.0^{2,6}.0^{8,10}]hexadéc-13(16)-ène-4,14-dione ;
- acétate de 3,8-diméthyl-12-(4-méthylpipéridino)-4,14-dioxo-5,9,15-trioxatétracyclo[11.2.1.0^{2,6}.0^{8,10}]hexadéc-13(16)-èn-11-yle ;
- 3,8-diméthyl-12-(4-méthylpipéridino)-4,14-dioxo-11-phénylcarbonyloxy-5,9,15-trioxatétracyclo[11.2.1.0^{2,6}.0^{8,10}]hexadéc-13(16)-ène ;
- 3,8-diméthyl-12-(4-méthylpipéridino)-4,14-dioxo-5,9,15-trioxatétracyclo[11.2.1.0^{2,6}.0^{8,10}]hexadéc-13(16)-èn-11-ylcarbonate d'éthyle ;
- 11-hydroxy-12-isobutylsulfanyl-3-isobutylsulfanylméthyl-8-méthyl-5,9,15-trioxatétracyclo[11.2.1.0^{2,6}.0^{8,10}]hexadéc-13(16)-ène-4,14-dione ;
- 11-hydroxy-12-isobutylsulfanyl-3,8-diméthyl-5,9,15-trioxatétracyclo[11.2.1.0^{2,6}.0^{8,10}]hexadéc-13(16)-ène-4,14-dione ;
- benzoate de 12-(diméthylamino)-3,8-diméthyl-4,14-dioxo-5,9,15-trioxatétracyclo[11.2.1.02,6.08,10]hexadéc-13(16)-èn-11-yle ;
- acétate de 12-(diméthylamino)-3,8-diméthyl-4,14-dioxo-5,9,15-trioxatétracyclo[11.2.1.02,6.08,10]hexadéc-13(16)-èn-11-yle ;
- cyclohexanecarboxylate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 4-fluorobenzoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- heptanoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 4-(trifluorométhyl)benzoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 2-thiophènecarboxylate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 3,3-diméthylbutanoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 1-benzothiophène-2-carboxylate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 2-furoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 5-nitro-2-furoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 2-thiénylacétate de 12-(diméthylamino)-3,8-diméthyl-4,14-dioxo-5,9,15-trioxatétracyclo[11.2.1.02,6.08,10]hexadéc-13(16)-èn-11-yle ;
- phénoxyacétate de 12-(diméthylamino)-3,8-diméthyl-4,14-dioxo-5,9,15-trioxatétracyclo[11.2.1.02,6.08,10]hexadéc-13(16)-èn-11-yle ;
- 4-*tert*-butylphénylcarbamate de 8-(diméthylamino)-3,10a-diméthyl-2,6-dioxodécahydro-4,7-méthènofuro[3,2-*c*]oxiréno[*f*]oxacycloundécin-9-yle ;
- thién-2-ylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 2-méthoxyphénylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 2(méthylthio)phénylcarbamate de 8-(diméthylamino)-3,10a-diméthyl-2,6-dioxodécahydro-4,7-méthèno-furo[3,2-*c*]oxiréno[*f*]oxacycloundécin-9-yle ;
- 2-éthoxyphénylcarbamate de 8-(diméthylamino)-3,10a-diméthyl-2,6-dioxodécahydro-4,7-méthènofuro[3,2-*c*]oxiréno[*f*]oxacycloundécin-9-yle ;
- thién-3-ylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 1-benzothién-3-ylcarbamate de 8-(diméthylamino)-3,10a-diméthyl-2,6-dioxodécahydro-4,7-méthènofuro[3,2-*c*]oxiréno[*f*]oxacycloundécin-9-yle ;
- *N*-(*ter*-butoxycarbonyl)glycinate de 8-(diméthylamino)-3,10a-diméthyl-2,6-dioxodécahydro-4,7-méthèno-furo[3,2-*c*]oxiréno[*f*]oxacycloundécin-9-yle ;
- thién-3-ylacétate de 8-(diméthylamino)-3,10a-diméthyl-2,6-dioxodécahydro-4,7-méthènofuro[3,2-*c*]oxiréno[*f*]oxacycloundécin-9-yle ;
- 1-benzothién-3-ylacétate de 8-(diméthylamino)-3,10a-diméthyl-2,6-dioxodécahydro-4,7-méthènofuro[3,2-*c*]oxiréno[*f*]oxacycloundécin-9-yle ;
- thiophène-3-carboxylate de 8-(diméthylamino)-3,10a-diméthyl-2,6-dioxodécahydro-4,7-méthènofuro[3,2-*c*]oxiréno[*f*]oxacycloundécin-9-yle ;
- 5-phénylthién-2-ylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 1-adamantylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 2-naphtylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 2-*tert*-butyl-6-méthylphénylcarbamate de 8-(diméthylamino)-3,10a-diméthyl-2,6-dioxodécahydro-4,7-méthèno-furo[3,2-*c*]oxiréno[*f*]oxacycloundécin-9-yle ;
- 2,5-diméthoxyphénylcarbamate de 8-(diméthylamino)-3,10a-diméthyl-2,6-dioxodécahydro-4,7-méthèno-furo[3,2-*c*]oxiréno[*f*]oxacycloundécin-9-yle ;
ou d'un sel pharmaceutiquement acceptable d'un de ces derniers.

8. Composition pharmaceutique contenant, à titre de principe actif, un composé de formule générale **(I)** telle que définie dans la revendication 1 ou un sel pharmaceutiquement acceptable de ce dernier.

9. Composition pharmaceutique selon la revendication 8, **caractérisée en ce qu'**elle contient, à titre de principe actif, l'un des composés suivants :
- 12-diisopropylaminométhyl-7-méthyl-3,6,10,15-tétraoxapentacyclo [12.2.1.0^{2,4}.0^{5,7}.0^{9,13}]heptadéc-1(17)-ène-11,16-dione ;
- 12-diméthylamino-3-diméthylaminométhyl-11-hydroxy-8-méthyl-5,9,15-trioxatétracyclo[11.2.1.0^{2,6}.0^{8,10}]hexadéc-13(16)-ène-4,14-dione ;
- 12-benzyl(méthyl)amino-3-benzyl(méthyl)aminométhyl-11-hydroxy-8-méthyl-5,9,15-trioxatétracyclo[11.2.1.0^{2,6}.0^{8,10}]hexadéc-13(16)-ène-4,14-dione ;
- 11-hydroxy-8-méthyl-12-morpholino-3-morpholinométhyl-5,9,15-trioxatétracyclo[11.2.1.0^{2,6}.0^{8,10}]hexadéc-13(16)-ène-4,14-dione ;
- 12-diméthylamino-11-hydroxy-3,8-diméthyl-5,9,15-trioxatétracyclo[11.2.1.0^{2,6}.0^{8,10}]hexadéc-13(16)-ène-4,14-dione ;
- 11-hydroxy-3,8-diméthyl-12-(4-méthylpipéridino)-5,9,15-trioxatétracyclo[11.2.1.0^{2,6}.0^{8,10}]hexadéc-13(16)-ène-4,14-dione ;
- 11-hydroxy-3,8-diméthyl-12-pyrrolidino-5,9,15-trioxatétracyclo[11.2.1.0^{2,6}.0^{8,10}]hexadéc-13(16)-ène-4,14-dione ;
- 1-[11-hydroxy-3,8-diméthyl-4,14-dioxo-5,9,15-trioxatétracyclo[11.2.1.0^{2,6}.0^{8,10}]hexadéc-13(16)-èn-12-yl]-4-pipéridinecarboxylate d'éthyle ;
- 12-(4-benzylpipéridino)-11-hydroxy-3,8-diméthyl-5,9,15-trioxatétracyclo[11.2.1.0^{2,6}.0^{8,10}]hexadéc-13(16)-ène-4,14-dione ;
- 11-hydroxy-3,8-diméthyl-12-pipéridino-5,9,15-trioxatétracyclo[11.2.1.0^{2,6}.0^{8,10}]hexadéc-13(16)-ène-4,14-dione ;
- 12-(1,4-dioxa-8-azaspiro[4.5]déc-8-yl)-11-hydroxy-3,8-diméthyl-5,9,15-trioxatétracyclo[11.2.1.0^{2,6}.0^{8,10}]hexadéc-13(16)-ène-4,14-dione ;
- 11-hydroxy-3,8-diméthyl-12-morpholino-5,9,15-trioxatétracyclo[11.2.1.0^{2,6}.0^{8,10}]hexadéc-13(16)-ène-4,14-dione ;
- 11-(*tert*-butyldiméthylsiloxy)-12-diméthylamino-3,8-diméthyl-5,9,15-trioxatétracyclo[11.2.1.0^{2,6}.0^{8,10}]hexadéc-13(16)-ène-4,14-dione ;
- acétate de 3,8-diméthyl-12-(4-méthylpipéridino)-4,14-dioxo-5,9,15-trioxatétracyclo[11.2.1.0^{2,6}.0^{8,10}]hexadéc-13(16)-èn-11-yle ;
- 3,8-diméthyl-12-(4-méthylpipéridino)-4,14-dioxo-11-phénylcarbonyloxy-5,9,15-trioxatétracyclo[11.2.1.0^{2,6}.0^{8,10}]hexadéc-13(16)-ène ;
- 3,8-diméthyl-12-(4-méthylpipéridino)-4,14-dioxo-5,9,15-trioxatétracyclo[11.2.1.0^{2,6}.0^{8,10}]hexadéc-13(16)-én-11-ylcarbonate d'éthyle ;
- 11-hydroxy-12-isobutylsulfanyl-3-isobutylsulfanylméthyl-8-méthyl-5,9,15-trioxatétracyclo[11.2.1.0^{2,6}.0^{8,10}]hexadéc-13(16)-ène-4,14-dione ;
- 11-hydroxy-12-isobutylsulfanyl-3,8-diméthyl-5,9,15-trioxatétracyclo[11.2.1.0^{2,6}.0^{8,10}]hexadéc-13(16)-ène-4,14-dione ;
- benzoate de 12-(diméthylamino)-3,8-diméthyl-4,14-dioxo-5,9,15-trioxatétracyclo[11.2.1.02,6.08,10]hexadéc-13(16)-èn-11-yle ;
- acétate de 12-(diméthylamino)-3,8-diméthyl-4,14-dioxo-5,9,15-trioxatétracyclo[11.2.1.02,6.08,10]hexadéc-13(16)-èn-11-yle ;
- cyclohexanecarboxylate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 4-fluorobenzoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- heptanoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 4-(trifluorométhyl)benzoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 2-thiophènecarboxylate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 3,3-diméthylbutanoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 1-benzothiophène-2-carboxylate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 2-furoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 5-nitro-2-furoate de 12-(diméthylamino)-3,8-diméthyl-4,14-dioxo-5,9,15-trioxatétracyclo[11.2.1.0^{2,6}.0^{8,10}]hexadéc-13(16)-èn-11-yle ;
- 2-thiénylacétate de 12-(diméthylamino)-3,8-diméthyl-4,14-dioxo-5,9,15-trioxatétracyclo[11.2.1.02,6.08,10]hexadéc-13(16)-èn-11-yle ;
- phénoxyacétate de 12-(diméthylamino)-3,8-diméthyl-4,14-dioxo-5,9,15-trioxatétracyclo[11.2.1.02,6.08,10]hexadéc-13(16)-èn-11-yle ;
- 4-*tert*-butylphénylcarbamate de 8-(diméthylamino)-3,10a-diméthyl-2,6-dioxodécahydro-4,7-méthènofuro[3,2-*c*]oxiréno[*f*]oxacycloundécin-9-yle ;
- thién-2-ylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 2-méthoxyphénylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 2(méthylthio)phénylcarbamate de 8-(diméthylamino)-3,10a-diméthyl-2,6-dioxodécahydro-4,7-méthèno-furo[3,2-*c*]oxiréno[*f*]oxacycloundécin-9-yle ;
- 2-éthoxyphénylcarbamate de 8-(diméthylamino)-3,10a-diméthyl-2,6-dioxodécahydro-4,7-méthènofuro[3,2-*c*]oxiréno[*f*]oxacycloundécin-9-yle ;
- thién-3-ylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 1-benzothién-3-ylcarbamate de 8-(diméthylamino)-3,10a-diméthyl-2,6-dioxodécahydro-4,7-méthènofuro[3,2-*c*]oxiréno[*f*]oxacycloundécin-9-yle ;
- *N*-(*ter*-butoxycarbonyl)glycinate de 8-(diméthylamino)-3,10a-diméthyl-2,6-dioxodécahydro-4,7-méthèno-furo[3,2-*c*]oxiréno[*f*]oxacycloundécin-9-yle ;
- thién-3-ylacétate de 8-(diméthylamino)-3,10a-diméthyl-2,6-dioxodécahydro-4,7-méthènofuro[3,2-*c*]oxiréno[*f*]oxacycloundécin-9-yle ;
- 1-benzothién-3-ylacétate de 8-(diméthylamino)-3,10a-diméthyl-2,6-dioxodécahydro-4,7-méthènofuro[3,2-*c*]oxiréno[*f*]oxacycloundécin-9-yle ;
- thiophène-3-carboxylate de 8-(diméthylamino)-3,10a-diméthyl-2,6-dioxodécahydro-4,7-méthènofuro[3,2-*c*]oxiréno[*f*]oxacycloundécin-9-yle ;
- 5-phénylthién-2-ylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 1-adamantylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 2-naphtylcarbamate de 8-(diméthylamino)-3,10a-diméthyl-2,6-dioxodécahydro-4,7-méthènofuro[3,2-*c*]oxiréno[*f*]oxacycloundécin-9-yle ;
- 2-*tert*-butyl-6-méthylphénylcarbamate de 8-(diméthylamino)-3,10a-diméthyl-2,6-dioxodécahydro-4,7-méthèno-furo[3,2-*c*]oxiréno[*f*]oxacycloundécin-9-yle ;
- 2,5-diméthoxyphénylcarbamate de 8-(diméthylamino)-3,10a-diméthyl-2,6-dioxodécahydro-4,7-méthèno-furo[3,2-*c*]oxiréno[*f*]oxacycloundécin-9-yle ;
ou un sel pharmaceutiquement acceptable d'un de ces derniers.

10. Utilisation d'un composé de formule générale **(I)** telle que définie dans la revendication 1 ou d'un sel pharmaceutiquement acceptable de ce dernier pour préparer un médicament destiné à inhiber les ADN polymérases.

11. Utilisation d'un composé de formule générale **(I)** telle que définie dans la revendication 1 ou d'un sel pharmaceutiquement acceptable de ce dernier pour préparer un médicament destiné à traiter les maladies dues à des proliférations cellulaires anormales.

12. Utilisation selon la revendication 11, **caractérisée en ce que** la maladie due à des proliférations cellulaires anormales est le cancer.

13. Utilisation selon la revendication 11, **caractérisée en ce que** la maladie due à des proliférations cellulaires anormales est choisie parmi l'athérosclérose, l'hyperplasie bénigne de la prostate et les fibroses.

14. Utilisation d'un composé de formule générale **(I)** telle que définie dans la revendication 1 ou d'un sel pharmaceutiquement acceptable de ce dernier pour préparer un médicament destiné à traiter les maladies virales.

15. Utilisation d'un composé de formule générale **(I)** telle que définie dans la revendication 1 ou d'un sel pharmaceutiquement acceptable de ce dernier pour préparer un médicament destiné à traiter les maladies parasitaires.

16. Utilisation d'un composé de formule générale **(I)** telle que définie dans la revendication 1 ou d'un sel pharmaceutiquement acceptable de ce dernier pour préparer un médicament destiné à traiter les maladies d'origine bactérienne.

## Patentansprüche

1. Verbindung der allgemeinen Formel (I) 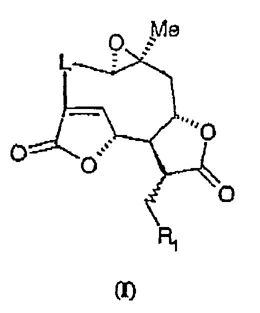
entsprechend den allgemeinen Unterformeln (I)₁ und (I)₂, 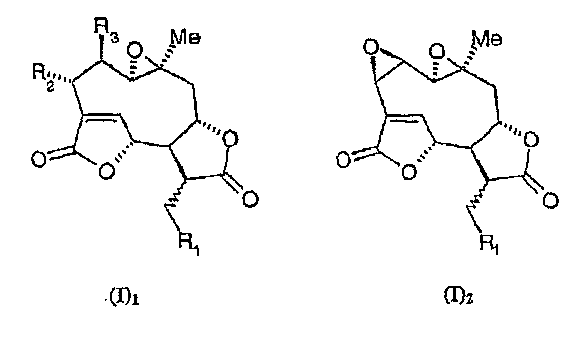
in denen
R₁ ein Wasserstoffatom oder einen Rest SR₄ oder NR₄R₅ darstellt;
R₂ SR₆ oder NR₆R₇ darstellt;
R₃ OH, O(CO)R₁₄, OSiR₁₅R₁₆R₁₇, O(CO)OR₁₈ oder O(CO)NHR₁₈ darstellt;
R₄ und R₆ unabhängig voneinander einen Alkylrest, einen Cycloalkyl-, Cycloalkylalkyl-, Hydroxyalkylrest oder auch einen der Reste Aryl oder Aralkyl darstellen, die gegebenenfalls an ihrer Arylgruppe mit einem oder mehreren Resten substituiert sind, die aus den Resten Alkyl, Hydroxy oder Alkoxy ausgewählt sind,
R₅ und R₇ unabhängig voneinander ein Wasserstoffatom, einen Alkylrest, einen Cycloalkyl-, Cycloalkylalkyl-, Hydroxyalkylrest oder auch einen der Reste Aryl oder Aralkyl darstellen, die gegebenenfalls an ihrer Arylgruppe mit einem oder mehreren Resten substituiert sind, die aus den Resten Alkyl, Hydroxy oder Alkoxy ausgewählt sind,
wobei R₄ und R₅ zusammen mit dem Stickstoffatom, das sie trägt, einen Heterocyclus mit 5 bis 7 Gliedern bilden können, wobei die ergänzenden Glieder aus den Resten -CR₈R₉-, -NR₁₀-, -O- und -S- ausgewählt sind, wobei jedoch gilt, dass sich in diesem Heterocyclus nur ein einziges aus -O- oder -S- ausgewähltes Glied befinden kann,
und R₆ und R₇ zusammen mit dem Stickstoffatom, das sie trägt, einen Heterocyclus mit 5 bis 7 Gliedern bilden können, wobei die ergänzenden Glieder aus den Resten -CR₁₁R₁₂-, -NR₁₃-, -O- und -S- ausgewählt sind, wobei jedoch gilt, dass sich in diesem Heterocyclus nur ein einziges aus -O- oder -S- ausgewähltes Glied befinden kann,
R₈, R₁₀, R₁₁ und R₁₃ jeweils unabhängig voneinander ein Wasserstoffatom oder einen Alkyl-, Alkoxycarbonyl- oder Aralkylrest darstellen,
wobei R₉ und R₁₂ jeweils unabhängig voneinander ein Wasserstoffatom oder einen Alkylrest darstellen oder jeder der Reste R₉ und R₁₂ mit R₈ bzw. R₁₁ einen Rest -O- (CH₂)₂-O-bilden kann, der zu beiden Seiten an das ihn tragende Kohlenstoffatom gebunden ist, wobei ein solcher Rest jedoch pro Rest NR₄R₅ oder NR₆R₇ maximal nur einmal vorhanden ist;
R₁₄ einen Alkyl-, Cycloalkyl- oder Adamantylrest oder einen der Reste Aryl, Heteroaryl, Aralkyl oder Heteroaralkyl darstellt, die gegebenenfalls an ihrer Aryl- oder Heteroarylgruppe mit einem oder mehreren Resten substituiert sind, die aus einem Halogenatom und den Resten Alkyl, Halogenalkyl, Nitro, Hydroxy, Alkoxy, Alkylthio oder Phenyl ausgewählt sind,
oder R₁₄ so beschaffen ist, dass R₁₄-COOH eine natürliche Aminosäure oder das optische Enantiomer einer solchen Aminosäure darstellt;
R₁₅, R₁₆ und R₁₇ unabhängig voneinander einen Alkylrest oder einen Phenylrest darstellen;
R₁₈ einen Alkyl-, Cycloalkyl- oder Adamantylrest oder einen der Reste Aryl, Heteroaryl, Aralkyl oder Heteroaralkyl darstellt, die gegebenenfalls an ihrer Aryl- oder Heteroarylgruppe mit einem oder mehreren Resten substituiert sind, die aus einem Halogenatom und den Resten Alkyl, Halogenalkyl, Nitro, Hydroxy, Alkoxy, Alkylthio oder Phenyl ausgewählt sind;
wobei jedoch gilt, dass, wenn die Verbindungen der allgemeinen Unterformel (I)₂ entsprechen, dann stellt R₁ kein Wasserstoffatom dar;
oder ein Salz einer Verbindung der allgemeinen Formel (I).

2. Verbindung nach Anspruch 1, **dadurch gekennzeichnet, dass** sie der allgemeinen Unterformel (I)₁ entspricht oder ein Salz einer solchen Verbindung ist.

3. Verbindung nach Anspruch 2, **dadurch gekennzeichnet, dass** :
• R₁ ein Wasserstoffatom oder einen Rest NR₄R₅ darstellt;
• R₂ einen Rest NR₆R₇ darstellt;
• R₃ OH oder einen der Reste O(CO)R₁₄, OSiR₁₅R₁₆R₁₇ oder O(CO)NHR18 darstellt.

4. Verbindung nach Anspruch 1, **dadurch gekennzeichnet, dass** sie der allgemeinen Unterformel (I)₂ entspricht.

5. Verbindung nach Anspruch 1, **dadurch gekennzeichnet, dass** es sich um eine der folgenden Verbindungen handelt:
- 12-Diisopropylaminomethyl-7-methyl-3,6,10,15-tetraoxapentacyclo[12.2.1.0^{2,4}.0^{5,7}.0^{9,13}]heptadec-1(17)-en-11,16-dion;
- 12-Dimethylamino-3-dimethylaminomethyl-11-hydroxy-8-methyl-5,9,15-trioxatetracyclo[11.2.1.0^{2,6}.0^{8,10}]hexadec-13(16)-en-4,14-dion;
- 12-Benzyl(methyl)amino-3-benzyl(methyl)aminomethyl-11-hydroxy-8-methyl-5,9,15-trioxatetracyclo[11.2.1.0^{2,6}.0^{8,10}]-hexadec-13(16)-en-4,14-dion;
- 11-Hydroxy-8-methyl-12-morpholino-3-morpholinomethyl-5,9,15-trioxatetracyclo[11.2.1.0^{2,6}.0^{8,10}]hexadec-13(16)-en-4,14-dion;
- 12-Dimethylamino-11-hydroxy-3,8-dimethyl-5,9,15-trioxatetracyclo[11.2.1.0^{2,6}.0^{8,10}]hexadec-13(16)-en-4,14-dion;
- 11-Hydroxy-3,8-dimethyl-12-(4-methylpiperidino)-5,9,15-trioxatetracyclo[11.2.1.0^{2,6}.0^{8,10}]hexadec-13(16)-en-4,14-dion;
- 11-Hydroxy-3,8-dimethyl-12-pyrrolidino-5,9,15-trioxatetracyclo[11.2.1.0^{2,6}.0^{8,10}]hexadec-13(16)-en-4,14-dion;
- 1-[11-Hydroxy-3,8-dimethyl-4,14-dioxo-5,9,15-trioxatetracyclo[11.2.1.0^{2,6}.0^{8,10}]hexadec-13(16)-en-12-yl]-4-piperidinethylcarboxylat;
- 12-(4-Benzylpiperidino)-11-hydroxy-3,8-dimethyl-5,9,15-trioxatetracyclo[11.2.1.0^{2,6}.0^{8,10}]hexadec-13(16)-en-4,14-dion;
- 11-Hydroxy-3,8-dimethyl-12-piperidino-5,9,15-trioxatetracyclo[11.2.1.0^{2,6}.0^{8,10}]hexadec-13(16)-en-4,14-dion;
- 12-(1,4-Dioxa-8-azaspiro[4.5]dec-8-yl)-11-hydroxy-3,8-dimethyl-5,9,15-trioxatetracyclo[11.2.1.0^{2,6}.0^{8,10}]hexadec-13(16)-en-4,14-dion;
- 11-Hydroxy-3,8-dimethyl-12-morpholino-5,9,15-trioxatetracyclo[11.2.1.0^{2,6}.0^{8,10}]hexadec-13-(16)-en-4,14-dion;
- 11-(*tert*-Butyldimethylsiloxy)-12-dimethylamino-3,8-dimethyl-5,9,15-trioxatetracyclo[11.2.1.0^{2,6}.0^{8,10}]hexadec-13-(16)-en-4,14-dion;
- 3,8-Dimethyl-12-(4-methylpiperidino)-4,14-dioxo-5,9,15-trioxatetracyclo[11.2.1.0^{2,6}.0^{8,10}]hexadec-13(16)-en-11-ylacetat;
- 3,8-Dimethyl-12-(4-methylpiperidino)-4,14-dioxo-11-phenylcarbonyloxy-5,9,15-trioxatetracyclo[11.2.1.0^{2,6}.0^{8,10}]hexadec-13(16)-en;
- 3,8-Dimethyl-12-(4-methylpiperidino)-4,14-dioxo-5,9,15-trioxatetracyclo[11.2.1.0^{2,6}.0^{8,10}]hexadec-13(16)-en-11-ylethylcarbonat;
- 11-Hydroxy-12-isobutylsulfanyl-3-isobutylsulfanylmethyl-8-methyl-5,9,15-trioxatetracyclo[11.2.1.0^{2,6}.0^{8,10}]hexadec-13(16)-en-4,14-dion;
- 11-Hydroxy-12-isobutylsulfanyl-3,8-dimethyl-5,9,15-trioxatetracyclo[11.2.1.0^{2,6}.0^{8,10}]hexadec-13(16)-en-4,14-dion;
- 12-(Dimethylamino)-3,8-dimethyl-4,14-dioxo-5,9,15-trioxatetracyclo[11.2.1.0^{2,6}.0^{8,10}]hexadec-13(16)-en-11-ylbenzoat;
- 12-(Dimethylamino)-3,8-dimethyl-4,14-dioxo-5,9,15-trioxatetracyclo[11.2.1.0^{2,6}.0^{8,10}]hexadec-13(16)-en-11-ylacetat;
- 12-(Dimethylamino)-3,8-dimethyl-4,14-dioxo-5,9,15-trioxatetracyclo[11.2.1.0^{2,6}.0^{8,10}]hexadec-13(16)-en-11-ylcyclohexancarboxylat;
- 12-(Dimethylamino)-3,8-dimethyl-4,14-dioxo-5,9,15-trioxatetracyclo[11.2.1.0^{2,6}.0^{8,10}]hexadec-13(16)-en-11-yl-4-fluorbenzoat;
- 12-(Dimethylamino)-3,8-dimethyl-4,14-dioxo-5,9,15-trioxatetracyclo[11.2.1.0^{2,6}.0^{8,10}]hexadec-13(16)-en-11-ylheptanoat;
- 12-(Dimethylamino)-3,8-dimethyl-4,14-dioxo-5,9,15-trioxatetracyclo[11.2.1.0^{2,6}.0^{8,10}]hexadec-13(16)-en-11-yl-4-(trifluormethyl)benzoat;
- 12-(Dimethylamino)-3,8-dimethyl-4,14-dioxo-5,9,15-trioxatetracyclo[11.2.1.0^{2,6}.0^{8,10}]hexadec-13(16)-en-11-yl-2-thiophencarboxylat;
- 12-(Dimethylamino)-3,8-dimethyl-4,14-dioxo-5,9,15-trioxatetracyclo[11.2.1.0^{2,6}.0^{8,10}]hexadec-13(16)-en-11-yl-3,3-dimethylbutanoat;
- 12-(Dimethylamino)-3,8-dimethyl-4,14-dioxo-5,9,15-trioxatetracyclo[11.2.1.0^{2,6}.0^{8,10}]hexadec-13(16)-en-11-yl-1-benzothiophen-2-carboxylat;
- 12-(Dimethylamino)-3,8-dimethyl-4,14-dioxo-5,9,15-trioxatetracyclo[11.2.1.0^{2,6}.0^{8,10}]hexadec-13(16)-en-11-yl-2-furoat;
- 12-(Dimethylamino)-3,8-dimethyl-4,14-dioxo-5,9,15-trioxatetracyclo[11.2.1.0^{2,6}.0^{8,10}]hexadec-13(16)-en-11-yl-5-nitro-2-furoat;
- 12-(Dimethylamino)-3,8-dimethyl-4,14-dioxo-5,9,15-trioxatetracyclo[11.2.1.0^{2,6}.0^{8,10}]hexadec-13(16)-en-11-yl-2-thienylacetat;
- 12-(Dimethylamino)-3,8-dimethyl-4,14-dioxo-5,9,15-trioxatetracyclo[11.2.1.0^{2,6}.0^{8,10}]hexadec-13(16)-en-11-ylphenoxyacetat;
- 8-(Dimethylamino)-3,10a-dimethyl-2,6-dioxodecahydro-4,7-methenofuro[3,2-*c*]oxireno[*f*]oxacycloundecin-9-yl-4-tert-butylphenylcarbamat;
- 8-(Dimethylamino)-3,10a-dimethyl-2,6-dioxodecahydro-4,7-methenofuro[3,2-*c*]oxireno[*f*]oxacycloundecin-9-ylthien-2-ylcarbamat;
- 8-(Dimethylamino)-3,10a-dimethyl-2,6-dioxodecahydro-4,7-methenofuro[3,2-*c*]oxireno[*f*]oxacycloundecin-9-yl-2-methoxyphenylcarbamat;
- 8-(Dimethylamino)-3,10a-dimethyl-2,6-dioxodecahydro-4,7-methenofuro[3,2-*c*]oxireno[*f*]oxacycloundecin-9-yl-2-(methylthio)phenylcarbamat;
- 8-(Dimethylamino)-3,10a-dimethyl-2,6-dioxodecahydro-4,7-methenofuro[3,2-*c*]oxireno[*f*]oxacycloundecin-9-yl-2-ethoxyphenylcarbamat;
- 8-(Dimethylamino)-3,10a-dimethyl-2,6-dioxodecahydro-4,7-methenofuro[3,2-*c*]oxireno[*f*]oxacycloundecin-9-ylthien-3-ylcarbamat;
- 8-(Dimethylamino)-3,10a-dimethyl-2,6-dioxodecahydro-4,7-methenofuro[3,2-*c*]oxireno[*f*]oxacycloundecin-9-yl-1-benzothien-3-ylcarbamat;
- 8-(Dimethylamino)-3,10a-dimethyl-2,6-dioxodecahydro-4,7-methenofuro[3,2-*c*]oxireno[*f*]oxacycloundecin-9-yl-N-(*tert*-butoxycarbonyl)glycinat;
- 8-(Dimethylamino)-3,10a-dimethyl-2,6-dioxodecahydro-4,7-methenofuro[3,2-*c*]oxireno[*f*]oxacycloundecin-9-ylthien-3-ylacetat;
- 8-(Dimethylamino)-3,10a-dimethyl-2,6-dioxodecahydro-4,7-methenofuro[3,2-*c*]oxireno[*f*]oxacycloundecin-9-yl-1-benzothien-3-ylacetat;
- 8-(Dimethylamino)-3,10a-dimethyl-2,6-dioxodecahydro-4,7-methenofuro[3,2-*c*]oxireno[*f*]oxacycloundecin-9-ylthiophen-3-carboxylat;
- 8-(Dimethylamino)-3,10a-dimethyl-2,6-dioxodecahydro-4,7-methenofuro[3,2-*c*]oxireno[*f*]oxacycloundecin-9-yl-5-phenylthien-2-ylcarbamat;
- 8-(Dimethylamino)-3,10a-dimethyl-2,6-dioxodecahydro-4,7-methenofuro[3,2-*c*]oxireno[*f*]oxacycloundecin-9-yl-1-adamantylcarbamat;
- 8-(Dimethylamino)-3,10a-dimethyl-2,6-dioxodecahydro-4,7-methenofuro[3,2-*c*]oxireno[*f*]oxacycloundecin-9-yl-2-naphtylcarbamat;
- 8-(Dimethylamino)-3,10a-dimethyl-2,6-dioxodecahydro-4,7-methenofuro[3,2-*c*]oxireno[*f*]oxacycloundecin-9-yl-2-*tert*-butyl-6-methylphenylcarbamat;
- 8-(Dimethylamino)-3,10a-dimethyl-2,6-dioxodecahydro-4,7-methenofuro[3,2-*c*]oxireno[*f*]oxacycloundecin-9-yl-2,5-dimethoxyphenylcarbamat;
oder ein Salz einer dieser Verbindungen.

6. Eine Verbindung der allgemeinen Formel (I), wie sie in Anspruch 1 definiert ist, oder ein pharmazeutisch annehmbares Salz davon in Form eines Medikaments.

7. Medikament nach Anspruch 6, **dadurch gekennzeichnet, dass** es sich um eine der folgenden Verbindungen handelt:
- 12-Diisopropylaminomethyl-7-methyl-3,6,10,15-tetraoxapentacyclo[12.2.1.0^{2,4}.0^{5,7}.0^{9,13}]heptadec-1(17)-en-11,16-dion;
- 12-Dimethylamino-3-dimethylaminomethyl-11-hydroxy-8-methyl-5,9,15-trioxatetracyclo[11.2.1.0^{2,6}.0^{8,10}]hexadec-13(16)-en-4,14-dion;
- 12-Benzyl(methyl)amino-3-benzyl(methyl)aminomethyl-11-hydroxy-8-methyl-5,9,15-trioxatetracyclo[11.2.1.0^{2,6}.0^{8,10}]-hexadec-13(16)-en-4,14-dion;
- 11-Hydroxy-8-methyl-12-morpholino-3-morpholinomethyl-5,9,15-trioxatetracyclo[11.2.1.0^{2,6}.0^{8,10}]hexadec-13 (16)-en-4,14-dion;
- 12-Dimethylamino-11-hydroxy-3,8-dimethyl-5,9,15-trioxatetracyclo[11.2.1.0^{2,6}.0^{8,10}]hexadec-13(16)-en-4,14-dion;
- 11-Hydroxy-3,8-dimethyl-12-(4-methylpiperidino)-5,9,15-trioxatetracyclo[11.2.1.0^{2,6}.0^{8,10}]hexadec-13(16)-en-4,14-dion;
- 11-Hydroxy-3,8-dimethyl-12-pyrrolidino-5,9,15-trioxatetracyclo[11.2.1.0^{2,6},0^{8,10}]hexadec-13(16)-en-4,14-dion;
- 1-[11-Hydroxy-3,8-dimethyl-4,14-dioxo-5,9,15-trioxatetracyclo[11.2.1.0^{2,6}.0^{8,10}]hexadec-13(16)-en-12-yl]-4-piperidinethylcarboxylat;
- 12-(4-Benzylpiperidino)-11-hydroxy-3,8-dimethyl-5,9,15-trioxatetracyclo[11.2.1.0^{2,6}.0^{8,10}]hexadec-13(16)-en-4,14-dion;
- 11-Hydroxy-3,8-dimethyl-12-piperidino-5,9,15-trioxotetracyclo[11.2.1.0^{2,6}.0^{8,10}]hexadec-13(16)-en-4,14-dion;
- 12-(1,4-Dioxa-8-azaspiro[4.5]dec-8-yl)-11-hydroxy-3,8-dimethyl-5,9,15-trioxatetracyclo[11.2.1.0^{2,6}.8^{8,10}]hexadec-13(16)-en-4,14-dion;
- 11-Hydroxy-3,8-dimethyl-12-morpholino-5,9,15-trioxatetracyclo[11.2.1.0^{2,6}.0^{8,10}]hexadec-13-(16)-en-4,14-dion;
- 11-(*tert*-Butyldimethylsiloxy)-12-dimethylamino-3,8-dimethyl-5,9,15-trioxatetracyclo[11.2.1.0^{2,6}.0^{8,10}]hexadec-13-(16)-en-4,14-dion;
- 3,8-Dimethyl-12-(4-methylpiperidino)-4,14-dioxo-5,9,15-trioxatetracyclo[11.2.1.0^{2,6}.0^{8,10}]hexadec-13(16)-en-11-ylacetat;
- 3,8-Dimethyl-12-(4-methylpiperidino)-4,14-dioxo-11-phenylcarbonyloxy-5,9,15-trioxatetracyclo[11.2.1.0^{2,6}.0^{8,10}]hexadec-13(16)-en;
- 3,8-Dimethyl-12-(4-methylpiperidino)-4,14-dioxo-5,9,15-trioxatetracyclo[11.2.1.0^{2,6}.0^{8,10}]hexadec-13(16)-en-11-ylethylcarbonat;
- 11-Hydroxy-12-isobutylsulfanyl-3-isobutylsulfanylmethyl-8-methyl-5,9,15-trioxatetracyclo[11.2.1.0^{2,6}.0^{8,10}]hexadec-13(16)-en-4,14-dion;
- 11-Hydroxy-12-isobutylsulfanyl-3,8-dimethyl-5,9,15-trioxatetracyclo[11.2.1.0^{2,6}.0^{8,10}]hexadec-13(16)-en-4,14-dion;
- 12-(Dimethylamino)-3,8-dimethyl-4,14-dioxo-5,9,15-trioxatetracyclo[11.2.1.0^{2,6}.0^{8,10}]hexadec-13(16)-en-11-ylbenzoat;
- 12-(Dimethylamino)-3,8-dimethyl-4,14-dioxo-5,9,15-trioxatetracyclo[11.2.1.0^{2,6}.0^{8,10}]hexadec-13(16)-en-11-ylacetat ;
- 12-(Dimethylamino)-3,8-dimethyl-4,14-dioxo-5,9,15-trioxatetracyclo[11.2.1.0^{2,6}.0^{8,10}]hexadec-13(16)-en-11-ylcyclohexancarboxylat;
- 12-(Dimethylamino)-3,8-dimethyl-4,14-dioxo-5,9,15-trioxatetracyclo[11.2.1.0^{2,6}.0^{8,10}]hexadec-13(16)-en-11-yl-4-fluorbenzoat;
- 12-(Dimethylamino)-3,8-dimethyl-4,14-dioxo-5,9,15-trioxatetracyclo[11.2.1.0^{2,6}.0^{8,10}]hexadec-13(16)-en-11-ylheptanoat;
- 12-(Dimethylamino)-3,8-dimethyl-4,14-dioxo-5,9,15-trioxatetracyclo[11.2.1.0^{2,6}.0^{8,10}]hexadec-13(16)-en-11-yl-4-(trifluormethyl)benzoat;
- 12-(Dimethylamino)-3,8-dimethyl-4,14-dioxo-5,9,15-trioxatetracyclo[11.2.1.0^{2,6}.0^{8,10}]hexadec-13(16)-en-11-yl-2-thiophencarboxylat;
- 12-(Dimethylamino)-3,8-dimethyl-4,14-dioxo-5,9,15-trioxatetracyclo[11.2.1.0^{2,6}.0^{8,10}]hexadec-13(16)-en-11-yl-3,3-dimethylbutanoat;
- 12-(Dimethylamino)-3,8-dimethyl-4,14-dioxo-5,9,15-trioxatetracyclo[11.2.1.0^{2,6}.0^{8,10}]hexadec-13(16)-en-11-yl-1-benzothiophen-2-carboxylat;
- 12-(Dimethylamino)-3,8-dimethyl-4,14-dioxo-5,9,15-trioxatetracyclo[11.2.1.0^{2,6}.0^{8,10}]hexadec-13(16)-en-11-yl-2-furoat;
- 12-(Dimethylamino)-3,8-dimethyl-4,14-dioxo-5,9,15-tri.oxatetracyclo[11.2.1.0^{2,6}.0^{8,10}]hexadec-13(16)-en-11-yl-5-nitro-2-furoat;
- 12-(Dimethylamino)-3,8-dimethyl-4,14-dioxo-5,9,15-trioxatetracyclo[11.2.1.0^{2,6}.0^{8,10}]hexadec-13(16)-en-11-yl-2-thienylacetat;
- 12-(Dimethylamino)-3,8-dimethyl-4,14-dioxo-5,9,15-trioxatetracyclo[11.2.1.0^{2,6}.0^{8,10}]hexadec-13(16)-en-11-ylphenoxyacetat;
- 8-(Dimethylamino)-3,10a-dimethyl-2,6-dioxodecahydro-4,7-methenofuro[3,2-*c*]oxireno[*f*]oxacycloundecin-9-yl-4-*tert*-butylphenylcarbamat;
- 8-(Dimethylamino)-3,10a-dimethyl-2,6-dioxodecahydro-4,7-methenofuro[3,2-*c*]oxireno[*f*]oxacycloundecin-9-ylthien-2-ylcarbamat;
- 8-(Dimethylamino)-3,10a-dimethyl-2,6-dioxodecahydro-4,7-methenofuro[3,2-*c*]oxireno[*f*]oxacycloundecin-9-yl-2-methoxyphenylcarbamat;
- 8-(Dimethylamino)-3,10a-dimethyl-2,6-dioxodecahydro-4,7-methenofuro[3,2-*c*]oxireno[*f*]oxacycloundecin-9-yl-2-(methylthio)phenylcarbamat;
- 8-(Dimethylamino)-3,10a-dimethyl-2,6-dioxodecahydro-4,7-methenofuro[3,2-*c*]oxireno[*f*]oxacycloundecin-9-yl-2-ethoxyphenylcarbamat;
- 8-(Dimethylamino)-3,10a-dimethyl-2,6-dioxodecahydro-4,7-methenofuro[3,2-*c*]oxireno[*f*]oxacycloundecin-9-ylthien-3-ylcarbamat;
- 8-(Dimethylamino)-3,10a-dimethyl-2,6-dioxodecahydro-4,7-methenofuro[3,2-*c*]oxireno[*f*]oxacycloundecin-9-yl-1-benzothien-3-ylcarbamat;
- 8-(Dimethylamino)-3,10a-dimethyl-2,6-dioxodecahydro-4,7-methenofuro[3,2-*c*]oxireno[*f*]oxacycloundecin-9-yl-N-(*tert*-butoxycarbonyl)glycinat;
- 8-(Dimethylamino)-3,10a-dimethyl-2,6-dioxodecahydro-4,7-methenofuro[3,2-*c*]oxireno[*f*]oxacycloundecin-9-ylthien-3-ylacetat;
- 8-(Dimethylamino)-3,10a-dimethyl-2,6-dioxodecahydro-4,7-methenofuro[3,2-*c*]oxireno[*f*]oxacycloundecin-9-yl-1-benzothien-3-ylacetat;
- 8-(Dimethylamino)-3,10a-dimethyl-2,6-dioxodecahydro-4,7-methenofuro[3,2-*c*]oxireno[*f*]oxacycloundecin-9-ylthiophen-3-carboxylat;
- 8-(Dimethylamino)-3,10a-dimethyl-2,6-dioxodecahydro-4,7-methenofuro[3,2-*c*]oxireno[*f*]oxacycloundecin-9-yl-5-phenylthien-2-ylcarbamat;
- 8-(Dimethylamino)-3,10a-dimethyl-2,6-dioxodecahydro-4,7-methenofuro[3,2-*c*]oxireno[*f*]oxacycloundecin-9-yl-1-adamantylcarbamat;
- 8-(Dimethylamino)-3,10a-dimethyl-2,6-dioxodecahydro-4,7-methenofuro[3,2-*c*]oxireno[*f*]oxacycloundecin-9-yl-2-naphtylcarbamat;
- 8-(Dimethylamino)-3,10a-dimethyl-2,6-dioxodecahydro-4,7-methenofuro[3,2-*c*]oxireno[*f*]oxacycloundecin-9-yl-2-*tert*-butyl-6-methylphenylcarbamat ;
- 8-(Dimethylamino)-3,10a-dimethyl-2,6-dioxodecahydro-4,7-methenofuro[3,2-*c*]oxireno[*f*]oxacycloundecin-9-yl-2,5-dimethoxyphenylcarbamat;
oder ein pharmazeutisch annehmbares Salz einer dieser Verbindungen.

8. Pharmazeutische Zusammensetzung, die als Wirkstoff eine Verbindung der allgemeinen Formel (I), wie sie in Anspruch 1 definiert ist, oder ein pharmazeutisch annehmbares Salz davon enthält.

9. Pharmazeutische Zusammensetzung nach Anspruch 8, **dadurch gekennzeichnet, dass** sie als Wirkstoff eine der folgenden Verbindungen enthält:
- 12-Diisopropylaminomethyl-7-methyl-3,6,10,15-tetraoxapentacyclo[12.2.1.0^{2,4}.0^{5,7}.0^{9,13}]heptadec-1(17)-en-11,16-dion;
- 12-Dimethylamino-3-dimethylaminomethyl-11-hydroxy-8-methyl-5,9,15-trioxatetracyclo[11.2.1.0^{2,6}.0^{8,10}]hexadec-13(16)-en-4,14-dion;
- 12-Benzyl(methyl)amino-3-benzyl(methyl)aminomethyl-11-hydroxy-8-methyl-5,9,15-trioxatetracyclo[11.2.1.0^{2,6}.0^{8,10}]hexadec-13(16)-en-4,14-dion;
- 11-Hydroxy-8-methyl-12-morpholino-3-morpholinomethyl-5,9,15-trioxatetracyclo[11.2.1.0^{2,6}.0^{8,10}]hexadec-13(16)-en-4,14-dion;
- 12-Dimethylamino-11-hydroxy-3,8-dimethyl-5,9,15-trioxatetracyclo[11.2.1.0^{2,6}.0^{8,10}]hexadec-13(16)-en-4,14-dion;
- 11-Hydroxy-3,8-dimethyl-12-(4-methylpiperidino)-5,9,15-trioxatetracyclo[11.2.1.0^{2,6}.0^{8,10}]hexadec-13(16)-en-4,14-dion;
- 11-Hydroxy-3,8-dimethyl-12-pyrrolidino-5,9,15-trioxatetracyclo[11.2.1.0^{2,6}.0^{8,10}]hexadec-13(16)-en-4,14-dion;
- 1-[11-Hydroxy-3,8-dimethyl-4,14-dioxo-5,9,15-trioxatetracyclo [11.2.1.0^{2,6}.0^{8,10}]hexadec-13(16)-en-12-yl]-4-piperidinethylcarboxylat;
- 12-(4-Benzylpiperidino)-11-hydroxy-3,8-dimethyl-5,9,15-trioxatetracyclo[11.2.1.0^{2,6}.0^{8,10}]hexadec-13(16)-en-4,14-dion;
- 11-Hydroxy-3,8-dimethyl-12-piperidino-5,9,15-trioxotetracyclo[11.2.1.0^{2,6}.0^{8,10}]hexadec-13(16)-en-4,14-dion;
- 12-(1,4-Dioxa-8-azaspiro[4.5]dec-8-yl)-11-hydroxy-3,8-dimethyl-5,9,15-trioxatetracyclo[11.2.1.0^{2,6}.8^{8,10}]hexadec-13(16)-en-4,14-dion;
- 11-Hydroxy-3,8-dimethyl-12-morpholino-5,9,15-trioxatetracyclo[11.2.1.0^{2,6}.0^{8,10}]hexadec-13-(16)-en-4,14-dion;
- 11-(*tert*-Butyldimethylsiloxy)-12-dimethylamino-3,8-dimethyl-5,9, 15-trioxatetracyclo [11.2.1.0^{2,6}.0^{8,10}]hexadec-13-(16)-en-4,14-dion;
- 3,8-Dimethyl-12-(4-methylpiperidino)-4,14-dioxo-5,9,15-trioxatetracyclo[11.2.1.0^{2,6}.0^{8,10}]hexadec-13(16)-en-11-ylacetat;
- 3,8-Dimethyl-12-(4-methylpiperidino)-4,14-dioxo-11-phenylcarbonyloxy-5,9,15-trioxatetracyclo[11.2.1.0^{2,6}.0^{8,10}]hexadec-13(16)-en;
- 3,8-Dimethyl-12-(4-methylpiperidino)-4,14-dioxo-5,9,15-trioxatetracyclo[11.2.1.0^{2,6}.0^{8,10}]hexadec-13(16)-en-11-ylethylcarbonat;
- 11-Hydroxy-12-isobutylsulfanyl-3-isobutylsulfanylmethyl-8-methyl-5,9,15-trioxatetracyclo[11.2.1.0^{2,6}.0^{8,10}]hexadec-13(16)-en-4,14-dion;
- 11-Hydroxy-12-isobutylsulfanyl-3,8-dimethyl-5,9,15-trioxatetracyclo[11.2.1.0^{2,6}.0^{8,10}]hexadec-13(16)-en-4,14-dion;
- 12-(Dimethylamino)-3,8-dimethyl-4,14-dioxo-5,9,15-trioxatetracyclo[11.2.1.0^{2,6}.0^{8,10}]hexadec-13(16)-en-11-ylbenzoat;
- 12-(Dimethylamino)-3,8-dimethyl-4,14-dioxo-5,9,15-trioxatetracyclo[11.2.1.0^{2,6}.0^{8,10}]hexadec-13(16)-en-11-ylacetat;
- 12-(Dimethylamino)-3,8-dimethyl-4,14-dioxo-5,9,15-trioxatetracyclo[11.2.1.0^{2,6}.0^{8,10}]hexadec-13(16)-en-11-ylcyclohexancarboxylat;
- 12-(Dimethylamino)-3,8-dimethyl-4,14-dioxo-5,9,15-trioxatetracyclo[11.2.1.0^{2,6}.0^{8,10}]hexadec-13(16)-en-11-yl-4-fluorbenzoat;
- 12-(Dimethylamino)-3,8-dimethyl-4,14-dioxo-5,9,15-trioxatetracyclo[11.2.1.0^{2,6}.0^{8,10}]hexadec-13(16)-en-11-ylheptanoat;
- 12-(Dimethylamino)-3,8-dimethyl-4,14-dioxo-5,9,15-trioxatetracyclo [11.2.1.0^{2,6}.0^{8,10}]hexadec-13(16)-en-11-yl-4-(trifluormethyl)benzoat;
- 12-(Dimethylamino)-3,8-dimethyl-4,14-dioxo-5,9,15-trioxatetracyclo[11.2.1.0^{2,6}.0^{8,10}]hexadec-13(16)-en-11-yl-2-thiophencarboxylat;
- 12-(Dimethylamino)-3,8-dimethyl-4,14-dioxo-5,9,15-trioxatetracyclo[11.2.1.0^{2,6}.0^{8,10}]hexadec-13(16)-en-11-yl-3,3-dimethylbutanoat;
- 12-(Dimethylamino)-3,8-dimethyl-4,14-dioxo-5,9,15-trioxatetracyclo[11.2.1.0^{2,6}.0^{8,10}]hexadec-13(16)-en-11-yl-1-benzothiophen-2-carboxylat;
- 12-(Dimethylamino)-3,8-dimethyl-4,14-dioxo-5,9,15-trioxatetracyclo[11.2.1.0^{2,6}.0^{8,10}]hexadec-13(16)-en-11-yl-2-furoat;
- 12-(Dimethylamino)-3,8-dimethyl-4,14-dioxo-5,9,15-trioxatetracyclo[11.2.1.0^{2,6}.0^{8,10}] hexadec-13(16)-en-11-yl-5-nitro-2-furoat;
- 12-(Dimethylamino)-3,8-dimethyl-4,14-dioxo-5,9,15-trioxatetracyclo [11.2.1.0^{2,6}.0^{8,10}]hexadec-13(16)-en-11-yl-2-thienylacetat;
- 12-(Dimethylamino)-3,8-dimethyl-4,14-dioxo-5,9,15-trioxatetracyclo[11.2.1.0^{2,6}.0^{8,10}]hexadec-13(16)-en-11-ylphenoxyacetat;
- 8-(Dimethylamino)-3,10a-dimethyl-2,6-dioxodecahydro-4,7-methenofuro[3,2-*c*]oxireno[*f*]oxacycloundecin-9-yl-4-*tert*-butylphenylcarbamat;
- 8-(Dimethylamino)-3,10a-dimethyl-2,6-dioxodecahydro-4,7-methenofuro[3,2-*c*]oxireno[*f*]oxacycloundecin-9-ylthien-2-ylcarbamat;
- 8-(Dimethylamino)-3,10a-dimethyl-2,6-dioxodecahydro-4,7-methenofuro[3,2-*c*]oxireno[*f*]oxacycloundecin-9-yl-2-methoxyphenylcarbamat;
- 8-(Dimethylamino)-3,10a-dimethyl-2,6-dioxodecahydro-4,7-methenofuro[3,2-*c*]oxireno[*f*]oxacycloundecin-9-yl-2-(methylthio)phenylcarbamat;
- 8-(Dimethylamino)-3,10a-dimethyl-2,6-dioxodecahydro-4,7-methenofuro[3,2-*c*]oxireno[*f*]oxacycloundecin-9-yl-2-ethoxyphenylcarbamat;
- 8-(Dimethylamino)-3,10a-dimethyl-2,6-dioxodecahydro-4,7-methenofuro[3,2-*c*]oxireno[*f*]oxacycloundecin-9-ylthien-3-ylcarbamat;
- 8-(Dimethylamino)-3,10a-dimethyl-2,6-dioxodecahydro-4,7-methenofuro[3,2-*c*]oxireno[*f*]oxacycloundecin-9-yl-1-benzothien-3-ylcarbamat;
- 8-(Dimethylamino)-3,10a-dimethyl-2,6-dioxodecahydro-4,7-methenofuro[3,2-*c*]oxireno[*f*]oxacycloundecin-9-yl-N-(*tert*-butoxycarbonyl)glycinat;
- 8-(Dimethylamino)-3,10a-dimethyl-2,6-dioxodecahydro-4,7-methenofuro[3,2-*c*]oxireno[*f*]oxacycloundecin-9-ylthien-3-ylacetat;
- 8-(Dimethylamino)-3,10a-dimethyl-2,6-dioxodecahydro-4,7-methenofuro[3,2-*c*]oxireno[*f*]oxacycloundecin-9-yl-1-benzothien-3-ylacetat;
- 8-(Dimethylamino)-3,10a-dimethyl-2,6-dioxodecahydro-4,7-methenofuro[3,2-*c*]oxireno[*f*]oxacycloundecin-9-ylthiophen-3-carboxylat;
- 8-(Dimethylamino)-3,10a-dimethyl-2,6-dioxodecahydro-4,7-methenofuro[3,2-*c*]oxireno[*f*]oxacycloundecin-9-yl-5-phenylthien-2-ylcarbamat;
- 8-(Dimethylamino)-3,10a-dimethyl-2,6-dioxodecahydro-4,7-methenofuro[3,2-*c*]oxireno[*f*]oxacycloundecin-9-yl-1-adamantylcarbamat;
- 8-(Dimethylamino)-3,10a-dimethyl-2,6-dioxodecahydro-4,7-methenofuro[3,2-*c*]oxireno[*f*]oxacycloundecin-9-yl-2-naphtylcarbamat;
- 8-(Dimethylamino)-3,10a-dimethyl-2,6-dioxodecahydro-4,7-methenofuro[3,2-*c*]oxireno[*f*]oxacycloundecin-9-yl-2-*tert*-butyl-6-methylphenylcarbamat;
- 8-(Dimethylamino)-3,10a-dimethyl-2,6-dioxodecahydro-4,7-methenofuro[3,2-*c*]oxireno[*f*]oxacycloundecin-9-yl-2,5-dimethoxyphenylcarbamat;
oder ein pharmazeutisch annehmbares Salz einer dieser Verbindungen.

10. Verwendung einer Verbindung der allgemeinen Formel (I), wie sie in Anspruch 1 definiert ist, oder eines pharmazeutisch annehmbaren Salzes dieser Verbindung für die Herstellung eines Medikaments, das zur Hemmung der DNA-Polymerasen bestimmt ist.

11. Verwendung einer Verbindung der allgemeinen Formel (I), wie sie in Anspruch 1 definiert ist, oder eines pharmazeutisch annehmbaren Salzes dieser Verbindung für die Herstellung eines Medikaments, das für die Behandlung von Krankheiten bestimmt ist, die durch anormale Zellenproliferationen verursacht werden.

12. Verwendung nach Anspruch 11, **dadurch gekennzeichnet, dass** die durch anormale Zellenproliferationen verursachte Krankheit Krebs ist.

13. Verwendung nach Anspruch 11, **dadurch gekennzeichnet, dass** die durch anormale Zellenproliferationen verursachte Krankheit ausgewählt ist aus: Atherosklerose, gutartige Hyperplasie der Prostata und Fibrosen.

14. Verwendung einer Verbindung der allgemeinen Formel (I), wie sie in Anspruch 1 definiert ist, oder eines pharmazeutisch annehmbaren Salzes dieser Verbindung für die Herstellung eines Medikaments, das für die Behandlung von Viruskrankheiten bestimmt ist.

15. Verwendung einer Verbindung der allgemeinen Formel (I), wie sie in Anspruch 1 definiert ist, oder eines pharmazeutisch annehmbaren Salzes dieser Verbindung für die Herstellung eines Medikaments, das für die Behandlung von parasitären Krankheiten bestimmt ist.

16. Verwendung einer Verbindung der allgemeinen Formel (I), wie sie in Anspruch 1 definiert ist, oder eines pharmazeutisch annehmbaren Salzes dieser Verbindung für die Herstellung eines Medikaments, das für die Behandlung von Krankheiten bakteriellen Ursprungs bestimmt ist.

## Claims

1. Compound of general formula **(I)** 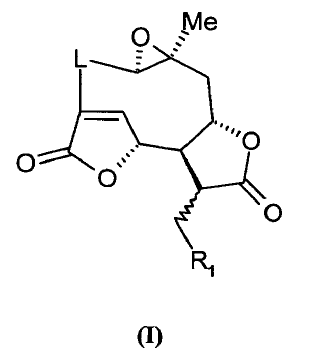
corresponding to general sub-formulae **(I)**_{**1**} and **(I)**_{**2**} 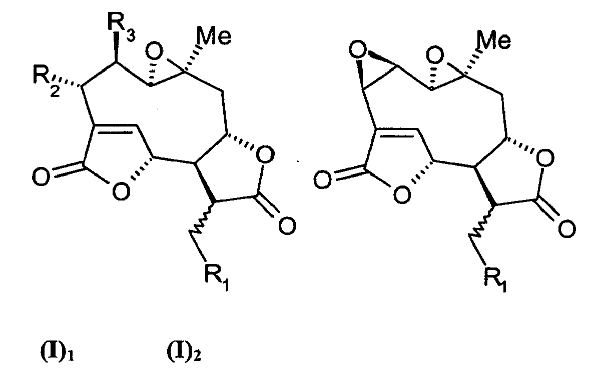
in which
R₁ represents a hydrogen atom or an SR₄ or NR₄R₅ radical;
R₂ represents SR₆ or NR₆R_{7;}
R₃ represents OH, O(CO)R₁₄, OSiR₁₅R₁₆R₁₇, O(CO)OR₁₈ or O(CO)NHR₁₈;
R₄ and R₆ represent, independently, an alkyl radical, a cycloalkyl, cycloalkylalkyl, hydroxyalkyl radical or also one of the aryl or aralkyl radicals optionally substituted on their aryl group by one or more radicals chosen from the alkyl, hydroxy or alkoxy radicals,
R₅ and R₇ represent, independently, a hydrogen atom, an alkyl radical, a cycloalkyl, cycloalkylalkyl, hydroxyalkyl radical or also one of the aryl or aralkyl radicals optionally substituted on their aryl group by one or more radicals chosen from the alkyl, hydroxy or alkoxy radicals,
R₄ and R₅ being able to form together with the nitrogen atom which carries them a heterocycle with 5 to 7 members, the additional members being chosen from the -CR₈R₉-, -NR₁₀-, -O- and -S- radicals, it being understood however that it there can only be one member chosen from -O- or -S- in said heterocycle,
and R₆ and R₇ being able to form together with the nitrogen atom which carries them a heterocycle with 5 to 7 members, the additional members being chosen from the -CR₁₁R₁₂-, -NR₁₃-, -O- and -S- radicals, it being understood however that there can only be one member chosen from -O- or -S- in said heterocycle,
R₈, R₁₀, R₁₁ and R₁₃ represent, independently each time they are involved, a hydrogen atom or an alkyl, alkoxycarbonyl or aralkyl radical,
R₉ and R₁₂ representing, independently each time they are involved, a hydrogen atom or an alkyl radical, or each of R₉ and R₁₂ being able to form respectively together with R₈ and R₁₁ an -O-(CH₂)₂-O- radical attached on either side to the carbon atom which carries it, such a radical only being present however once at most per NR₄R₅ or NR₆R₇ radical,
R₁₄ represents an alkyl, cycloalkyl or adamantyl radical or one of the aryl, heteroaryl, aralkyl or heteroaralkyl radicals optionally substituted on their aryl or heteroaryl group by one or more radicals chosen from a halogen atom and the alkyl, haloalkyl, nitro, hydroxy, alkoxy, alkylthio or phenyl radicals;
or also R₁₄ is such that R₁₄-COOH represents a natural amino acid or the optical enantiomer of such an amino acid;
R₁₅, R₁₆ and R₁₇ represent, independently, an alkyl radical or a phenyl radical;
R₁₈ represents an alkyl, cycloalkyl or adamantyl radical or one of the aryl, heteroaryl, aralkyl or heteroaralkyl radicals optionally substituted on their aryl or heteroaryl group by one or more radicals chosen from a halogen atom and the alkyl, haloalkyl, nitro, hydroxy, alkoxy, alkylthio or phenyl radicals;
it being understood however that when the compounds correspond to general sub-formula **(I)**_{**2**}**,** then R₁ does not represent a hydrogen atom;
or a salt of a compound of general formula **(I).**

2. Compound according to claim 1, **characterized in that** it corresponds to general sub-formula **(I)**_{**1**} or is a salt of such a compound.

3. Compound according to claim 2, **characterized in that**:
• R₁ represents a hydrogen atom or an NR₄R₅ radical;
• R₂ representing an NR₆R₇ radical;
• R₃ represents OH or an O(CO)R₁₄, OSiR₁₅R₁₆R₁₇ or O(CO)NHR₁₈ radical.

4. Compound according to claim 1, **characterized in that** it corresponds to general sub-formula **(I)**_{**2**}.

5. Compound according to claim 1, **characterized in that** it is one of the following compounds:
- 12-diisopropylaminomethyl-7-methyl-3,6,10,15-tetraoxapentacyclo[12.2.1.0^{2,4}.0^{5,7}.0^{9,13}]heptadec-1(17)-ene-11,16-dione;
- 12-dimethylamino-3-dimethylaminomethyl-11-hydroxy-8-methyl-5,9,15-trioxatetracyclo[11.2.1.0^{2,6}.0^{8,10}]hexadec-13(16)-ene-4,14-dione;
- 12-benzyl(methyl)amino-3-benzyl(methyl)aminomethyl-11-hydroxy-8-methyl-5,9,15-trioxatetracyclo[11.2.1.0^{2,6}.0^{8,10}]hexadec-13(16)-ene-4,14-dione;
- 11-hydroxy-8-methyl-12-morpholino-3-morpholinomethyl-5,9,15-trioxatetracyclo[11.2.1.0^{2,6}.0^{8,10}]hexadec-13(16)-ene-4,14-dione;
- 12-dimethylamino-11-hydroxy-3,8-dimethyl-5,9,15-trioxatetracyclo[11.2.1.0^{2,6}.0^{8,10}]hexadec-13(16)-ene-4,14-dione;
- 11-hydroxy-3,8-dimethyl-12-(4-methylpiperidino)-5,9,15-trioxatetracyclo[11.2.1.0^{2,6}.0^{8,10}]hexadec-13(16)-ene-4,14-dione;
- 11-hydroxy-3,8-dimethyl-12-pyrrolidino-5,9,15-trioxatetracyclo[11.2.1.0^{2,6}.0^{8,10}]hexadec-13(16)-ene-4,14-dione;
- ethyl 1-[11-hydroxy-3,8-dimethyl-4,14-dioxo-5,9,15-trioxatetracyclo[11.2.1.0^{2,6}.0^{8,10}]hexadec-13(16)-en-12-yl]-4-piperidinecarboxylate;
- 12-(4-benzylpiperidino)-11-hydroxy-3,8-dimethyl-5,9,15-trioxatetracyclo[11.2.1.0^{2,6}.0^{8,10}]hexadec-13(16)-ene-4,14-dione;
- 1 1-hydroxy-3,8-dimethyl-12-piperidino-5,9,15-trioxatetracyclo[11.2.1.0^{2,6}.0^{8,10}]hexadec-13(16)-ene-4,14-dione;
- 12-(1,4-dioxa-8-azaspiro[4.5]dec-8-yl)-11-hydroxy-3,8-dimethyl-5,9,15-trioxatetracyclo[11.2.1.0^{2,6}.0^{8,10}]hexadec-13(16)-ene-4,14-dione;
- 11-hydroxy-3,8-dimethyl-12-morpholino-5,9,15-trioxatetracyclo[11.2.1.0^{2,6}.0^{8,10}]hexadec-13(16)-ene-4,14-dione;
- 11-(*tert*-butyldimethylsiloxy)-12-dimethylamino-3,8-dimethyl-5,9,15-trioxatetracyclo[11.2.1.0^{2,6}.0^{8,10}]hexadec-13(16)-ene-4,14-dione;
- 3,8-dimethyl-12-(4-methylpiperidino)-4,14-dioxo-5,9,15-trioxatetracyclo[1 11.2.1.0^{2,6}.0^{8,10}]hexadec-13(16)-en-11-yl acetate;
- 3,8-dimethyl-12-(4-methylpiperidino)-4,14-dioxo-11-phenylcarbonyloxy-5 ,9,15-trioxatetracyclo[11.2.1.0^{2,6}.0^{8,10}]hexadec-13(16)-ene;
- ethyl 3,8-dimethyl-12-(4-methylpiperidino)-4,14-dioxo-5,9,15-trioxatetracyclo[11.2.1.0^{2,6}.0^{8,10}]hexadec-13(16)-en-11-yl carbonate;
- 11-hydroxy-12-isobutylsulphanyl-3-isobutylsulphanylmethyl-8-methyl-5,9,15-trioxatetracyclo[11.2.1.0^{2,6}.0^{8,10}]hexadec-13(16)-ene-4,14-dione;
- 11-hydroxy-12-isobutylsulphanyl-3,8-dimethyl-5,9,15-trioxatetracyclo[11.2.1.0^{2,6}.0^{8,10}]hexadec-13(16)-ene-4,14-dione;
- 12-(dimethylamino)-3,8-dimethyl-4,14-dioxo-5,9,15-trioxatetracyclo[11.2.1.02.6.08.10]hexadec-13(16)-en-11-yl benzoate;
- 12-(dimethylamino)-3,8-dimethyl-4,14-dioxo-5,9,15-trioxatetracyclo[11.2.1.02.6.08.10]hexadec-13(16)-en-11-yl acetate;
- 12-(dimethylamino)-3,8-dimethyl-4,14-dioxo-5,9,15-trioxatetracyclo[11.2.1.0^{2,6}.0^{8,10}]hexadec-13(16)-en-11-yl cyclohexanecarboxylate;
- 12-(dimethylamino)-3,8-dimethyl-4,14-dioxo-5,9,15-trioxatetracyclo[11.2.1.0^{2,6}.0^{8,10}]hexadec-13(16)-en-11-yl 4-fluorobenzoate;
- 12-(dimethylamino)-3,8-dimethyl-4,14-dioxo-5,9,15-trioxatetracyclo[11.2.1.0^{2,6}.0^{8,10}]hexadec-13(16)-en-11-yl heptanoate;
- 12-(dimethylamino)-3,8-dimethyl-4,14-dioxo-5,9,15-trioxatetracyclo[11.2.1.0^{2,6}.0^{8,10}]hexadec-13(16)-en-11-yl 4-(trifluoromethyl)benzoate;
- 12-(dimethylamino)-3,8-dimethyl-4,14-dioxo-5,9,15-trioxatetracyclo[11.2.1.0^{2,6}.0^{8,10}]hexadec-13(16)-en-11-yl 2-thiophenecarboxylate;
- 12-(dimethylamino)-3,8-dimethyl-4,14-dioxo-5,9,15-trioxatetracyclo[11.2.1.0^{2,6}.0^{8,10}]hexadec-13(16)-en-11-yl 3,3-dimethylbutanoate;
- 12-(dimethylamino)-3,8-dimethyl-4,14-dioxo-5,9,15-trioxatetracyclo[11.2.1.0^{2,6}.0^{8,10}]hexadec-13(16)-en-11-yl 1-benzothi ophene-2-carboxylate;
- 12-(dimethylamino)-3,8-dimethyl-4,14-dioxo-5,9,15-trioxatetracyclo[11.2.1.0^{2,6}.0^{8,10}]hexadec-13(16)-en-11-yl 2-furoate;
- 12-(dimethylamino)-3,8-dimethyl-4,14-dioxo-5,9,15-trioxatetracyclo[11.2.1.0^{2,6}.0^{8,10}]hexadec-13(16)-en-11-yl 5-nitro-2-furoate;
- 12-(dimethylamino)-3,8-dimethyl-4,14-dioxo-5,9,15-trioxatetracyclo[11.2.1.02,6.08,10]hexadec-13(16)-en-11-yl 2-thienylacetate;
- 12-(dimethylamino)-3,8-dimethyl-4,14-dioxo-5,9,15-trioxatetracyclo[11.2.1.02,6.08,10]hexadec-13(16)-en-11-ylphenoxyacetate;
- 8-(dimethylamino)-3,10a-dimethyl-2,6-dioxodecahydro-4,7-methenofuro[3,2-*c*]oxireno[*f*]oxacycloundecin-9-yl 4-*tert*-butylphenylcarbamate;
- 8-(dimethylamino)-3,10a-dimethyl-2,6-dioxodecahydro-4,7-methenofuro[3,2-*c*]oxireno[*f*]oxacycloundecin-9-yl thien-2-ylcarbamate;
- 8-(dimethylamino)-3,10a-dimethyl-2,6-dioxodecahydro-4,7-methenofuro[3,2-*c*]oxireno[*f*]oxacycloundecin-9-yl 2-methoxyphenylcarbamate;
- 8-(dimethylamino)-3,10a-dimethyl-2,6-dioxodecahydro-4,7-metheno-furo[3,2-*c*]oxireno[*f*]oxacycloundecin-9-yl 2(methylthio)phenylcarbamate;
- 8-(dimethylamino)-3,10a-dimethyl-2,6-dioxodecahydro-4,7-methenofuro[3,2-*c*]oxireno[*f*]oxacycloundecin-9-yl 2-ethoxyphenylcarbamate;
- 8-(dimethylamino)-3,10a-dimethyl-2,6-dioxodecahydro-4,7-methenofuro[3,2-*c*]oxireno[*f*]oxacycloundecin-9-yl thien-3-ylcarbamate;
- 8-(dimethylamino)-3,10a-dimethyl-2,6-dioxodecahydro-4,7-methenofuro[3,2-*c*]oxireno[*f*]oxacycloundecin-9-yl 1-benzothien-3-ylcarbamate;
- 8-(dimethylamino)-3,10a-dimethyl-2,6-dioxodecahydro-4,7-metheno-furo[3,2-*c*]oxireno[*f*]oxacycloundecin-9-yl *N*-(*ter*-butoxycarbonyl)glycinate;
- 8-(dimethylamino)-3,10a-dimethyl-2,6-dioxodecahydro-4,7-methenofuro[3,2-*c*]oxireno[*f*]oxacycloundecin-9-yl thien-3-ylacetate;
- 8-(dimethylamino)-3,10a-dimethyl-2,6-dioxodecahydro-4,7-methenofuro[3,2-*c*]oxireno[*f*]oxacycloundecin-9-yl 1-benzothien-3-ylacetate;
- 8-(dimethylamino)-3,10a-dimethyl-2,6-dioxodecahydro-4,7-methenofuro[3,2-*c*]oxireno[*f*]oxacycloundecin-9-yl thiophene-3-carboxylate;
- 8-(dimethylamino)-3, 10a-dimethyl-2,6-dioxodecahydro-4,7-methenofuro[3,2-*c*]oxireno[*f*]oxacycloundecin-9-yl 5-phenylthien-2-ylcarbamate;
- 8-(dimethylamino)-3,10a-dimethyl-2,6-dioxodecahydro-4,7-methenofuro[3,2-*c*]oxireno[*f*]oxacycloundecin-9-yl 1-adamantylcarbamate;
- 8-(dimethylamino)-3,10a-dimethyl-2,6-dioxodecahydro-4,7-methenofuro[3,2-*c*]oxireno[*f*]oxacycloundecin-9-yl 2-naphthylcarbamate;
- 8-(dimethylamino)-3,10a-dimethyl-2,6-dioxodecahydro-4,7-metheno-furo[3,2-*c*]oxireno[*f*]oxacycloundecin-9-yl 2-*tert*-butyl-6-methylphenylcarbamate;
- 8-(dimethylamino)-3,10a-dimethyl-2,6-dioxodecahydro-4,7-metheno-furo[3,2-*c*]oxireno[*f*]oxacycloundecin-9-yl 2,5-dimethoxyphenylcarbamate;
or a salt of one of the latter.

6. As a medicament, a compound of general formula **(I)** as defined in claim 1 or a pharmaceutically acceptable salt of the latter.

7. Medicament according to claim 6, **characterized in that** it is one of the following compounds:
- 12-diisopropylaminomethyl-7-methyl-3,6,10,15-tetraoxapentacyclo [12.2.1.0^{2,4}.0^{5,7}.0^{9,13}]heptadec-1(17)-ene-11,16-dione;
- 12-dimethylamino-3-dimethylaminomethyl-11-hydroxy-8-methyl-5,9,15-trioxatetracyclo[11.2.1.0^{2,6}.0^{8,10}]hexadec-13(16)-ene-4,14-dione;
- 12-benzyl(methyl)amino-3-benzyl(methyl)aminomethyl-11-hydroxy-8-methyl-5,9,15-trioxatetracyclo[11.2.1.0^{2,6}.0^{8,10}]hexadec-13(16)-ene-4,14-dione;
- 11-hydroxy-8-methyl-12-morpholino-3-morpholinomethyl-5,9,15-trioxatetracyclo[11.2.1.0^{2,6}.0^{8,10}]hexadec-13(16)-ene-4,14-dione;
- 12-dimethylamino-11-hydroxy-3,8-dimethyl-5,9,15-trioxatetracyclo[11.2.1.0^{2,6}.0^{8,10}]hexadec-13(16)-ene-4,14-dione;
- 11-hydroxy-3,8-dimethyl-12-(4-methylpiperidino)-5,9,15-trioxatetracyclo[11.2.1.0^{2,6}.0^{8,10}]hexadec-13(16)-ene-4,14-dione;
- 11-hydroxy-3,8-dimethyl-12-pyrrolidino-5,9,15-trioxatetracyclo[11.2.1.0^{2,6}.0^{8,10}]hexadec-13(16)-ene-4,14-dione;
- ethyl 1-[11-hydroxy-3,8-dimethyl-4,14-dioxo-5,9,15-trioxatetracyclo[11.2.1.0^{2,6}.0^{8,10}]hexadec-13(16)-en-12-yl]-4-piperidinecarboxylate;
- 12-(4-benzylpiperidino)-11-hydroxy-3,8-dimethyl-5,9,15-trioxatetracyclo[11.2.1.0^{2,6}.0^{8,10}]hexadec-13(16)-ene-4,14-dione ;
- 11-hydroxy-3,8-dimethyl-12-piperidino-5,9,15-trioxatetracyclo[11.2.1.0^{2,6}.0^{8,10}]hexadec-13(16)-ene-4,14-dione;
- 12-(1,4-dioxa-8-azaspiro[4.5]dec-8-yl)-11-hydroxy-3,8-dimethyl-5,9,15-trioxatetracyclo[11.2.1.0^{2,6}.0^{8,10}]hexadec-13(16)-ene-4,14-dione;
- 11-hydroxy-3,8-dimethyl-12-morpholino-5,9,15-trioxatetracyclo[11.2.1.0^{2,6}.0^{8,10}]hexadec-13(16)-ene-4,14-dione;
- 11-(*tert*-butyldimethylsiloxy)-12-dimethylamino-3,8-dimethyl-5,9,15-trioxatetracyclo[11.2.1.0^{2,6}.0^{8,10}]hexadec-13(16)-ene-4,14-dione;
- 3,8-dimethyl-12-(4-methylpiperidino)-4,14-dioxo-5,9,15-trioxatetracyclo[11.2.1.0^{2,6}.0^{8,10}]hexadec-13(16)-en-11-yl acetate;
- 3,8-dimethyl-12-(4-methylpiperidino)-4,14-dioxo-11-phenylcarbonyloxy-5,9,15-trioxatetracyclo[11.2.1.0^{2,6}.0^{8,10}]hexadec-13(16)-ene;
- ethyl 3,8-dimethyl-12-(4-methylpiperidino)-4,14-dioxo-5,9,15-trioxatetracyclo[11.2.1.0^{2,6}.0^{8,10}]hexadec-13(16)-en-11-ylcarbonate;
- 11-hydroxy-12-isobutylsulphanyl-3-isobutylsulphanylmethyl-8-methyl-5,9,15-trioxatetracyclo[11.2.1.0^{2,6}.0^{8,10}]hexadec-13(16)-ene-4,14-dione;
- 11-hydroxy-12-isobutylsulphanyl-3,8-dimethyl-5,9,15-trioxatetracyclo[11.2.1.0^{2,6}.0^{8,10}]hexadec-13(16)-ene-4,14-dione;
- 12-(dimethylamino)-3,8-dimethyl-4,14-dioxo-5,9,15-trioxatetracyclo[11.2.1.02,6.08,10]hexadec-13(16)-en-11-yl benzoate;
- 12-(dimethylamino)-3,8-dimethyl-4,14-dioxo-5,9,15-trioxatetracyclo[11.2.1.02,6.08,10]hexadec-13(16)-en-11-yl acetate;
- 12-(dimethylamino)-3,8-dimethyl-4,14-dioxo-5,9,15-trioxatetracyclo[11.2.1.0^{2,6}.0^{8,10}]hexadec-13(16)-en-11-yl cyclohexanecarboxylate;
- 12-(dimethylamino)-3,8-dimethyl-4,14-dioxo-5,9,15-trioxatetracyclo[11.2.1.0^{2,6}.0^{8,10}]hexadec-13(16)-en-11-yl 4-fluorobenzoate;
- 12-(dimethylamino)-3,8-dimethyl-4,14-dioxo-5,9,15-trioxatetracyclo[11.2.1.0^{2,6}.0^{8,10}]hexadec-13(16)-en-11-yl heptanoate;
- 12-(dimethylamino)-3,8-dimethyl-4,14-dioxo-5,9,15-trioxatetracyclo[11.2.1.0^{2,6}.0^{8,10}]hexadec-13(16)-en-11-yl 4-(trifluoromethyl)benzoate;
- 12-(dimethylamino)-3,8-dimethyl-4,14-dioxo-5,9,15-trioxatetracyclo[11.2.1.0^{2,6}.0^{8,10}]hexadec-13(16)-en-11-yl 2-thiophenecarboxylate;
- 12-(dimethylamino)-3,8-dimethyl-4,14-dioxo-5,9,15-trioxatetracyclo[11.2.1.0^{2,6}.0^{8,10}]hexadec-13(16)-en-11-yl 3,3-dimethylbutanoate;
- 12-(dimethylamino)-3,8-dimethyl-4,14-dioxo-5,9,15-trioxatetracyclo[11.2.1.0^{2,6}.0^{8,10}]hexadec-13(16)-en-11-yl 1-benzothiophene-2-carboxylate;
- 12-(dimethylamino)-3,8-dimethyl-4,14-dioxo-5,9,15-trioxatetracyclo[11.2.1.0^{2,6}.0^{8,10}]hexadec-13(16)-en-11-yl 2-furoate;
- 12-(dimethylamino)-3,8-dimethyl-4,14-dioxo-5,9,15-trioxatetracyclo[11.2.1.0^{2,6}.0^{8,10}]hexadec-13(16)-en-11-yl 5-nitro-2-furoate;
- 12-(dimethylamino)-3,8-dimethyl-4,14-dioxo-5,9,15-trioxatetracyclo[11.2.1.02,6.08,10]hexadec-13(16)-en-11-yl 2-thienylacetate;
- 12-(dimethylamino)-3,8-dimethyl-4,14-dioxo-5,9,15-trioxatetracyclo[11.2.1.02,6.08,10]hexadec-13(16)-en-11-yl phenoxyacetate;
- 8-(dimethylamino)-3.10a-dimethyl-2,6-dioxodecahydro-4,7-methenofuro[3,2-*c*]oxireno[*f*]oxacycloundecin-9-yl 4-*tert*-butylphenylcarbamate;
- 8-(dimethylamino)-3.10a-dimethyl-2,6-dioxodecahydro-4,7-methenofuro[3,2-*c*]oxireno[*f*]oxacycloundecin-9-yl thien-2-ylcarbamate;
- 8-(dimethylamino)-3.10a-dimethyl-2,6-dioxodecahydro-4,7-methenofuro[3,2-*c*]oxireno[*f*]oxacycloundecin-9-yl 2-methoxyphenylcarbamate;
- 8-(dimethylamino)-3.10a-dimethyl-2,6-dioxodecahydro-4,7-metheno-furo[3,2-*c*]oxireno[*f*]oxacycloundecin-9-yl 2(methylthio)phenylcarbamate;
- 8-(dimethylamino)-3.10a-dimethyl-2,6-dioxodecahydro-4,7-methenofuro[3,2-*c*]oxireno[*f*]oxacycloundecin-9-yl 2-ethoxyphenylcarbamate;
- 8-(dimethylamino)-3.10a-dimethyl-2,6-dioxodecahydro-4,7-methenofuro[3,2-*c*]oxireno[*f*]oxacycloundecin-9-yl thien-3-ylcarbamate;
- 8-(dimethylamino)-3.10a-dimethyl-2,6-dioxodecahydro-4,7-methenofuro[3,2-*c*]oxireno[*f*]oxacycloundecin-9-yl 1-benzothien-3-ylcarbamate;
- 8-(dimethylamino)-3.10a-dimethyl-2,6-dioxodecahydro-4,7-metheno-furo[3,2-*c*]oxireno[*f*]oxacycloundecin-9-yl *N*-(*ter*-butoxycarbonyl)glycinate;
- 8-(dimethylamino)-3.10a-dimethyl-2,6-dioxodecahydro-4,7-methenofuro[3,2-*c*]oxireno[*f*]oxacycloundecin-9-yl thien-3-ylacetate;
- 8-(dimethylamino)-3.10a-dimethyl-2,6-dioxodecahydro-4,7-methenofuro[3,2-*c*]oxireno[*f*]oxacycloundecin-9-yl 1-benzothien-3-ylacetate;
- 8-(dimethylamino)-3.10a-dimethyl-2,6-dioxodecahydro-4,7-methenofuro[3,2-*c*]oxireno[*f*]oxacycloundecin-9-yl thiophene-3-carboxylate;
- 8-(dimethylamino)-3.10a-dimethyl-2,6-dioxodecahydro-4,7-methenofuro[3,2-*c*]oxireno[*f*]oxacycloundecin-9-yl 5-phenylthien-2-ylcarbamate;
- 8-(dimethylamino)-3.10a-dimethyl-2,6-dioxodecahydro-4,7-methenofuro[3,2-*c*]oxireno[*f*]oxacycloundecin-9-yl 1-adamantylcarbamate;
- 8-(dimethylamino)-3.10a-dimethyl-2,6-dioxodecahydro-4,7-methenofuro[3,2-*c*]oxireno[*f*]oxacycloundecin-9-yl 2-naphthylcarbamate;
- 8-(dimethylamino)-3.10a-dimethyl-2,6-dioxodecahydro-4,7-metheno-furo[3,2-*c*]oxireno[*f*]oxacycloundecin-9-yl 2-*tert*-butyl-6-methylphenylcarbamate;
- 8-(dimethylamino)-3.10a-dimethyl-2,6-dioxodecahydro-4,7-metheno-furo[3,2-*c*]oxireno[*f*]oxacycloundecin-9-yl 2,5-dimethoxyphenylcarbamate;
or a pharmaceutically acceptable salt of one of the latter.

8. Pharmaceutical composition containing, as active ingredient, a compound of general formula **(I)** as defined in claim 1 or a pharmaceutically acceptable salt of the latter.

9. Pharmaceutical composition according to claim 8, **characterized in that** it contains, as active ingredient, one of the following compounds:
- 12-diisopropylaminomethyl-7-methyl-3,6,10,15-tetraoxapentacyclo[12.2.1.0^{2,4}.0^{5,7}.0^{9,13}]heptadec-1(17)-ene-11,16-dione;
- 12-dimethylamino-3-dimethylaminomethyl-11-hydroxy-8-methyl-5,9,15-trioxatetracyclo[11.2.1.0^{2,6}.0^{8,10}]hexadec-13(16)-ene-4,14-dione;
- 12-benzyl(methyl)amino-3-benzyl(methyl)aminomethyl-11-hydroxy-8-methyl-5,9,15-trioxatetracyclo[11.2.1.0^{2,6}.0^{8,10}]hexadec-13(16)-ene-4,14-dione;
- 11-hydroxy-8-methyl-12-morpholino-3-morpholinomethyl-5,9,15-trioxatetracyclo[11.2.1.0^{2,6}.0^{8,10}]hexadec-13(16)-ene-4,14-dione;
- 12-dimethylamino-11-hydroxy-3,8-dimethyl-5,9,15-trioxatetracyclo[11.2.1.0^{2,6}.0^{8,10}]hexadec-13(16)-ene-4,14-dione;
- 11-hydroxy-3,8-dimethyl-12-(4-methylpiperidino)-5,9,15-trioxatetracyclo[11.2.1.0^{2,6}.0^{8,10}]hexadec-13(16)-ene-4,14-dione;
- 11-hydroxy-3,8-dimethyl-12-pyrrolidino-5,9,15-trioxatetracyclo[11.2.1.0^{2,6}.0^{8,10}]hexadec-13(16)-ene-4,14-dione;
- ethyl 1-[11-hydroxy-3,8-dimethyl-4,14-dioxo-5,9,15-trioxatetracyclo[11.2.1.0^{2,6}.0^{8,10}]hexadec-13(16)-en-12-yl]-4-piperidinecarboxylate;
- 12-(4-benzylpiperidino)-11-hydroxy-3,8-dimethyl-5,9,15-trioxatetracyclo[11.2.1.0^{2,6}.0^{8,10}]hexadec-13(16)-ene-4,14-dione;
- 11-hydroxy-3,8-dimethyl-12-piperidino-5,9,15-trioxatetracyclo[11.2.1.0^{2,6}.0^{8,10}]hexadec-13(16)-ene-4,14-dione;
- 12-(1,4-dioxa-8-azaspiro[4.5]dec-8-yl)-11-hydroxy-3,8-dimethyl-5,9,15-trioxatetracyclo[11.2.1.0^{2,6}.0^{8,10}]hexadec-13(16)-ene-4,14-dione;
- 11-hydroxy-3,8-dimethyl-12-morpholino-5,9,15-trioxatetracyclo[11.2.1.0^{2,6}.0^{8,10}]hexadec-13(16)-ene-4,14-dione;
- 11-(*tert*-butyldimethylsiloxy)-12-dimethylamino-3,8-dimethyl-5,9,15-trioxatetracyclo[11.2.1.0^{2,6}.0^{8,10}]hexadec-13(16)-ene-4,14-dione;
- 3,8-dimethyl-12-(4-methylpiperidino)-4,14-dioxo-5,9,15-trioxatetracyclo[11.2.1.0^{2,6}.0^{8,10}]hexadec-13(16)-en-11-yl acetate;
- 3,8-dimethyl-12-(4-methylpiperidino)-4,14-dioxo-11-phenylcarbonyloxy-5,9,15-trioxatetracyclo[11.2.1.0^{2,6}.0^{8,10}]hexadec-13(16)-ene;
- ethyl 3,8-dimethyl-12-(4-methylpiperidino)-4,14-dioxo-5,9,15-trioxatetracyclo[11.2.1.0^{2,6}.0^{8,10}]hexadec-13(16)-en-11-yl carbonate;
- 11-hydroxy-12-isobutylsulphanyl-3-isobutylsulphanylmethyl-8-methyl-5,9,15-trioxatetracyclo[11.2.1.0^{2,6}.0^{8,10}]hexadec-13(16)-ene-4,14-dione;
- 11-hydroxy-12-isobutylsulphanyl-3,8-dimethyl-5,9,15-trioxatetracyclo[11.2.1.0^{2,6}.0^{8,10}]hexadec-13(16)-ene-4,14-dione;
- 12-(dimethylamino)-3,8-dimethyl-4,14-dioxo-5,9,15-trioxatetracyclo[11.2.1.02.6.08.10]hexadec-13(16)-en-11-yl benzoate;
- 12-(dimethylamino)-3,8-dimethyl-4,14-dioxo-5,9,15-trioxatetracyclo[11.2.1.02.6.08.10]hexadec-13(16)-en-11-yl acetate;
- 12-(dimethylamino)-3,8-dimethyl-4,14-dioxo-5,9,15-trioxatetracyclo[11.2.1.0^{2,6}.0^{8,10}]hexadec-13(16)-en-11-yl cyclohexanecarboxylate;
- 12-(dimethylamino)-3,8-dimethyl-4,14-dioxo-5,9,15-trioxatetracyclo[11.2.1.0^{2,6}.0^{8,10}]hexadec-13(16)-en-11-yl 4-fluorobenzoate;
- 12-(dimethylamino)-3,8-dimethyl-4,14-dioxo-5,9,15-trioxatetracyclo[11.2.1.0^{2,6}.0^{8,10}]hexadec-13(16)-en-11-yl heptanoate;
- 12-(dimethylamino)-3,8-dimethyl-4,14-dioxo-5,9,15-trioxatetracyclo[11.2.1.0^{2,6}.0^{8,10}]hexadec-13(16)-en-11-yl 4-(trifluoromethyl)benzoate;
- 12-(dimethylamino)-3,8-dimethyl-4,14-dioxo-5,9,15-trioxatetracyclo[11.2.1.0^{2,6}.0^{8,10}]hexadec-13(16)-en-11-yl 2-thiophenecarboxylate;
- 12-(dimethylamino)-3,8-dimethyl-4,14-dioxo-5,9,15-trioxatetracyclo[11.2.1.0^{2,6}.0^{8,10}]hexadec-13(16)-en-11-yl 3,3-dimethylbutanoate;
- 12-(dimethylamino)-3,8-dimethyl-4,14-dioxo-5,9,15-trioxatetracyclo[11.2.1.0^{2,6}.0^{8,10}]hexadec-13(16)-en-11-yl 1-benzothiophene-2-carboxylate;
- 12-(dimethylamino)-3,8-dimethyl-4,14-dioxo-5,9,15-trioxatetracyclo[11.2.1.0^{2,6}.0^{8,10}]hexadec-13(16)-en-11-yl 2-furoate;
- 12-(dimethylamino)-3,8-dimethyl-4,14-dioxo-5,9,15-trioxatetracyclo[11.2.1.0^{2,6}.0^{8,10}]hexadec-13(16)-en-11-yl 5-nitro-2-furoate;
- 12-(dimethylamino)-3,8-dimethyl-4,14-dioxo-5,9,15-trioxatetracyclo[11.2.1.02.6.08.10]hexadec-13(16)-en-11-yl 2-thienylacetate;
- 12-(dimethylamino)-3,8-dimethyl-4,14-dioxo-5,9,15-trioxatetracyclo[11.2.1.02.6.08.10]hexadec-13(16)-en-11-yl phenoxyacetate;
- 8-(dimethylamino)-3,10a-dimethyl-2,6-dioxodecahydro-4,7-methenofuro[3,2-*c*]oxireno[*f*]oxacycloundecin-9-yl 4-*tert*-butylphenylcarbamate;
- 8-(dimethylamino)-3,10a-dimethyl-2,6-dioxodecahydro-4,7-methenofuro[3,2-*c*]oxireno[*f*]oxacycloundecin-9-yl thien-2-ylcarbamate;
- 8-(dimethylamino)-3,10a-dimethyl-2,6-dioxodecahydro-4,7-methenofuro[3,2-*c*]oxireno[*f*]oxacycloundecin-9-yl 2-methoxyphenylcarbamate;
- 8-(dimethylamino)-3,10a-dimethyl-2,6-dioxodecahydro-4,7-metheno-furo[3,2-*c*]oxireno[*f*]oxacycloundecin-9-yl 2(methylthio)phenylcarbamate;
- 8-(dimethylamino)-3,10a-dimethyl-2,6-dioxodecahydro-4,7-methenofuro[3,2-*c*]oxireno[*f*]oxacycloundecin-9-yl 2-ethoxyphenylcarbamate;
- 8-(dimethylamino)-3,10a-dimethyl-2,6-dioxodecahydro-4,7-methenofuro[3,2-*c*]oxireno[*f*]oxacycloundecin-9-yl thien-3-ylcarbamate;
- 8-(dimethylamino)-3,10a-dimethyl-2,6-dioxodecahydro-4,7-methenofuro[3,2-*c*]oxireno[*f*]oxacycloundecin-9-yl 1-benzothien-3-ylcarbamate;
- 8-(dimethylamino)-3,10a-dimethyl-2,6-dioxodecahydro-4,7-metheno-furo[3,2-*c*]oxireno[*f*]oxacycloundecin-9-yl *N*-(*ter*-butoxycarbonyl)glycinate;
- 8-(dimethylamino)-3,10a-dimethyl-2,6-dioxodecahydro-4,7-methenofuro[3,2-*c*]oxireno[*f*]oxacycloundecin-9-yl thien-3-ylacetate;
- 8-(dimethylamino)-3,10a-dimethyl-2,6-dioxodecahydro-4,7-methenofuro[3,2-*c*]oxireno[*f*]oxacycloundecin-9-yl 1-benzothien-3-ylacetate;
- 8-(dimethylamino)-3,10a-dimethyl-2,6-dioxodecahydro-4,7-methenofuro[3,2-*c*]oxireno[*f*]oxacycloundecin-9-yl thiophene-3-carboxylate;
- 8-(dimethylamino)-3,10a-dimethyl-2,6-dioxodecahydro-4,7-methenofuro[3,2-*c*]oxireno[*f*]oxacycloundecin-9-yl 5-phenylthien-2-ylcarbamate;
- 8-(dimethylamino)-3,10a-dimethyl-2,6-dioxodecahydro-4,7-methenofuro[3,2-*c*]oxireno[*f*]oxacycloundecin-9-yl 1-adamantylcarbamate;
- 8-(dimethylamino)-3,10a-dimethyl-2,6-dioxodecahydro-4,7-methenofuro[3,2-*c*]oxireno[*f*]oxacycloundecin-9-yl 2-naphthylcarbamate;
- 8-(dimethylamino)-3,10a-dimethyl-2,6-dioxodecahydro-4,7-metheno-furo[3,2-*c*]oxireno[*f*]oxacycloundecin-9-yl 2-*tert*-butyl-6-methylphenylcarbamate;
- 8-(dimethylamino)-3,10a-dimethyl-2,6-dioxodecahydro-4,7-metheno-furo[3,2-*c*]oxireno[*f*]oxacycloundecin-9-yl 2,5-dimethoxyphenylcarbamate;
or a pharmaceutically acceptable salt of one of the latter.

10. Use of a compound of general formula **(I)** as defined in claim 1 or a pharmaceutically acceptable salt of the latter for preparing a medicament intended to inhibit DNA polymerases.

11. Use of a compound of general formula (**I**) as defined in claim 1 or a pharmaceutically acceptable salt of the latter for preparing a medicament intended to treat diseases due to abnormal cell proliferation.

12. Use according to claim 11, **characterized in that** the disease due to abnormal cell proliferation is cancer.

13. Use according to claim 11, **characterized in that** the disease due to abnormal cell proliferation is chosen from atherosclerosis, benign hyperplasia of the prostate and fibroses.

14. Use of a compound of general formula **(I)** as defined in claim 1 or a pharmaceutically acceptable salt of the latter for preparing a medicament intended to treat viral diseases.

15. Use of a compound of general formula **(I)** as defined in claim 1 or a pharmaceutically acceptable salt of the latter for preparing a medicament intended to treat parasitic diseases.

16. Use of a compound of general formula **(I)** as defined in claim 1 or a pharmaceutically acceptable salt of the latter for preparing a medicament intended to treat diseases of bacterial origin.
